# EUROPÄISCHE PATENTSCHRIFT

(11) **EP 0 723 534 B1**
(45) Veröffentlichungstag und Bekanntmachung des Hinweises auf die Patenterteilung: **29.07.1998**
(21) Anmeldenummer: 94929539.8
(22) Anmeldetag: 12.10.1994
(51) Int. Cl.: C07D 239/54, A01N 47/36, C07D 251/52, C07D 239/47, C07D 239/42, C07D 251/16, C07D 239/46, C07D 239/52

(54) **PHENYLSULFONYLHARNSTOFFE, VERFAHREN ZU IHRER HERSTELLUNG UND IHRE VERWENDUNG ALS HERBIZIDE UND PFLANZENWACHSTUMSREGULATOREN**
PHENYLSULFONYL UREAS, PROCESS FOR PRODUCING THE SAME AND THEIR USE AS HERBICIDES AND PLANT GROWTH REGULATORS
UREES DE PHENYLSULFONYLE, LEUR PROCEDE DE PRERATION ET LEUR UTILISATION COMME HERBICIDES ET COMME REGULATEURS DE LA CROISSANCE VEGETALE

(30) Priorität: 15.10.1993 DE 4335297
(43) Veröffentlichungstag der Anmeldung: 31.07.1996
(73) Patentinhaber: Hoechst Schering AgrEvo GmbH, 13509 Berlin (DE)
(72) Erfinder: LORENZ, Klaus, D-64331 Weiterstadt (DE); WILLMS, Lothar, D-65719 Hofheim (DE); BAUER, Klaus, D-63456 Hanau (DE); BIERINGER, Hermann, D-65817 Eppstein (DE)
(86) Internationale Anmeldenummer: EP9403369
(87) Internationale Veröffentlichungsnummer: WO9510507

(56) Entgegenhaltungen:
- WO-A-89/10921
- US-A- 4 927 453

## Beschreibung

Es ist bekannt, daß einige Phenylsulfonylharnstoffe herbizide und pflanzenwachstumsregulierende Eigenschaften besitzen; vgl. US-A-4,786,314, US-A-4,927,453 und WO 89/10921. Diese weisen jedoch bei ihrer Anwendung zum Teil Nachteile auf, wie beispielsweise eine hohe Persistenz oder unzureichende Selektivität in wichtigen Nutzpflanzenkulturen.

Es wurden nun Phenylsulfonylharnstoffe mit speziellen Resten am Phenylring gefunden, die mit Vorteilen als Herbizide und Pflanzenwachstumsregulatoren einzusetzen sind.

Gegenstand der vorliegenden Erfindung sind Verbindungen der Formel (I) und deren Salze, 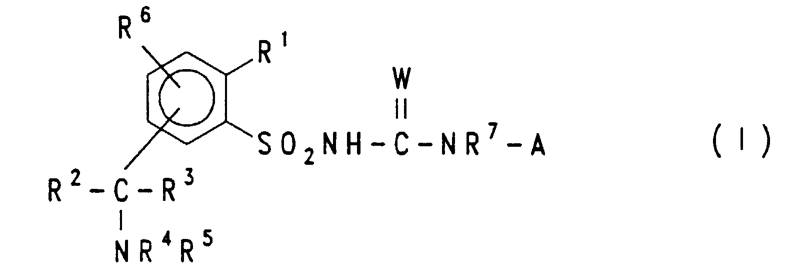 worin
- R¹: CO-Q-R⁸,
- R², R³: unabhängig voneinander H oder (C₁-C₄)Alkyl,
- R⁴: H, (C₁-C₈)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, [(C₁-C₄)Alkoxy]-carbonyl und CN substituiert ist, oder (C₃-C₆)Alkenyl, das unsubstituiert oder durch ein oder mehrere Halogenatome substituiert ist, oder (C₃-C₆)Alkinyl, das unsubstituiert oder durch ein oder mehrere Halogenatome substituiert ist, oder Hydroxy, (C₁-C₄)Alkoxy, [(C₁-C₄)Alkyl]-carbonyl oder (C₁-C4)Alkylsulfonyl, wobei jeder der letztgenannten drei Reste unsubstituiert oder im Alkylteil durch ein oder mehrere Halogenatome oder durch (C₁-C₄)Alkoxy oder (C₁-C₄)Alkylthio substituiert ist, oder Phenylsulfonyl, wobei der Phenylrest unsubstituiert oder substituiert ist, vorzugsweise durch einen oder mehrere Reste aus der Gruppe Halogen, CN, NO₂, (C₁-C₄)Alkyl, (C₁-C₄)Haloalkyl und (C₁-C₄)Alkoxy substituiert ist, und
- R⁵: (C₁-C₄)Alkylsulfonyl oder (C₃-C₆)Alkenylsulfonyl, wobei jeder der beiden letztgenannten Reste unsubstituiert oder durch ein oder mehrere Halogenatome oder durch (C₁-C₄)Alkoxy oder (C₁-C₄)Alkylthio substituiert ist, oder Phenylsulfonyl oder Phenylcarbonyl, wobei der Phenylrest in jedem der beiden letztgenannten Reste unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, Hydroxy, Amino, Nitro, Cyano, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, Mono- und Di-[(C₁-C₄)alkyl]-amino, (C₁-C₄)Alkylsulfinyl und (C₁-C₄)Alkylsulfonyl substituiert ist, vorzugsweise durch einen oder mehrere Reste aus der Gruppe Halogen, CN, NO₂, (C₁-C₄)Alkyl, (C₁-C₄)Haloalkyl und (C₁-C₄)Alkoxy substituiert ist, oder Mono- oder Di-[(C₁-C₄)Alkyl]-aminosulfonyl oder [(C₁-C₆)Alkyl]-carbonyl, wobei jeder der drei letztgenannten Reste unsubstituiert oder durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, [(C₁-C₄)Alkyl]-carbonyl, [(C₁-C₄)Alkoxy]-carbonyl und CN substituiert ist, oder Formyl, eine Gruppe der Formel -CO-CO-R', worin R'= H, OH, (C₁-C₄)-Alkoxy oder (C₁-C₄)Alkyl ist, oder [(C₃-C₆)Cycloalkyl]-carbonyl, [(C₂-C₆)Alkenyl]-carbonyl oder [(C₂-C₆)Alkinyl]-carbonyl, wobei jeder der letztgenannten drei Reste unsubstituiert oder durch ein oder mehrere Halogenatome substituiert ist, oder eine Gruppe der Formel 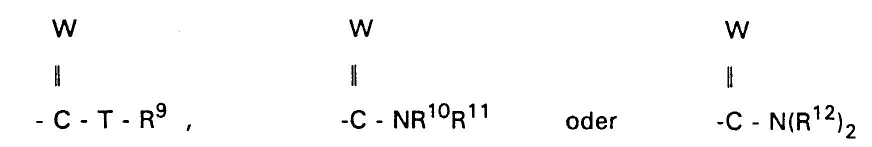 bedeuten,
wobei im Falle, daß der Rest -CR²R³-NR⁴R⁵ in Parastellung zur SO₂-Gruppe des Sulfonylharnstoffmoleküls steht, die Bedeutung R⁵ = unsubstituiertes [(C₁-C₆)Alkyl]-carbonyl ausgenommen ist, oder
- R⁴ und R⁵: gemeinsam eine Kette der Formel (-CH₂)ₘ B- oder -B¹-(CH₂)ₘ₁-B-, wobei die Kette unsubstituiert oder durch einen oder mehrere, vorzugsweise bis zu vier (C₁-C₃)Alkylreste substituiert ist und m = 3 oder 4 bzw. m1 = 2 oder 3 sind, bedeuten sowie
- W: ein Sauerstoff- oder Schwefelatom (d.h. O oder S),
- B, B¹: unabhängig voneinander SO₂ oder CO,
- Q: O, S oder -NR¹³-,
- T: O oder S,
- R⁶: H, (C₁-C₄)Alkyl, (C₁-C₄)Alkoxy, [(C₁-C₄)Alkyl]-carbonyl oder [(C₁-C₄)Alkoxy]-carbonyl, wobei jeder der letztgenannten 4 Reste unsubstituiert oder im Alkylteil durch ein oder mehrere Halogenatome substituiert ist, oder Halogen, NO₂ oder CN,
- R⁷: H oder CH₃,
- R⁸: H, (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, [(C₁-C₄)Alkyl]-carbonyl und [(C₁-C₄)Alkoxy]-carbonyl substituiert ist,
- R⁹: (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, [(C₁-C₄)Alkyl]-carbonyl und [(C₁-C₄)Alkoxy]-carbonyl substituiert ist,
- R¹⁰, R¹¹: unabhängig voneinander H, (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, [(C₁-C₄)Alkyl]-carbonyl und [(C₁-C₄)Alkoxy]-carbonyl substituiert ist,
- die Reste R¹²: gemeinsam mit dem N-Atom einen heterocyclischen Ring mit 5 oder 6 Ringgliedern, der weitere Heteroatome aus der Gruppe N, O und S in den möglichen Oxidationsstufen enthalten kann und unsubstituiert oder durch (C₁-C₄)Alkyl oder die Oxogruppe substituiert ist oder benzokondensiert ist,
- R¹³: H, (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist,
- A: einen Rest der Formel 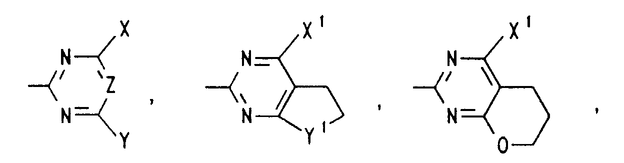 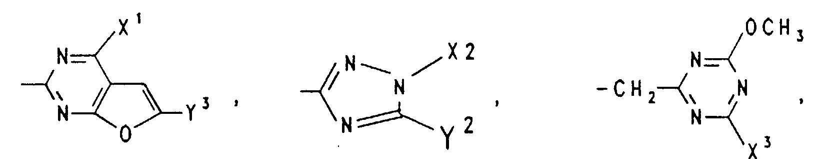 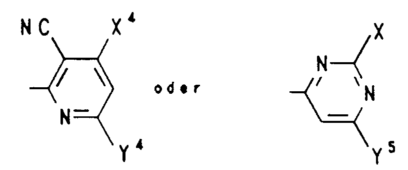
einer der Reste X und Y Wasserstoff, Halogen, (C₁-C₃)Alkyl oder (C₁-C₃)Alkoxy, wobei die beiden letztgenannten Reste unsubstituiert oder ein- oder mehrfach durch Halogen oder einfach durch (C₁-C₃)Alkoxy substituiert sind, und
der andere der Reste X und Y Wasserstoff, (C₁-C₃)Alkyl, (C₁-C₃)Alkoxy oder (C₁-C₃)Alkylthio, wobei die drei letztgenannten alkylhaltigen Reste unsubstituiert oder ein- oder mehrfach durch Halogen oder ein- oder zweifach durch (C₁-C₃)Alkoxy oder (C₁-C₃)Alkylthio substituiert sind, oder einen Rest der Formel NR¹⁴R¹⁵, (C₃-C₆)-Cycloalkyl, (C₂-C₄)Alkenyl, (C₂-C₄)Alkinyl, (C₃-C₄)-Alkenyloxy oder (C₃-C₄)Alkinyloxy,
- Z: CH oder N,
- R¹⁴ und R¹⁵: unabhängig voneinander H, (C₁-C₃)Alkyl oder (C₃-C₄)Alkenyl,
- X¹: CH₃, OCH₃, OC₂H₅ oder OCHF_{2 '}
- Y¹: -O- oder -CH₂- ,
- X²: CH₃, C₂H₅ oder CH₂CF₃,
- Y²: OCH₃, OC₂H₅, SCH₃, SCH₂CH₃, CH₃ oder C₂H₅,
- X³: CH₃ oder OCH₃,
- Y³: H oder CH₃,
- X⁴: CH₃, OCH₃, OC₂H_{5'} CH₂OCH₃ oder Cl,
- Y⁴: CH₃, OCH₃, OC₂H₅ oder Cl und
- Y⁵: CH₃, C₂H₅, OCH₃ oder Cl bedeuten.

In der Formel (I) und im folgenden können Alkyl-, Alkoxy-, Haloalkyl-, Alkylamino- und Alkylthioreste sowie die entsprechenden ungesättigten und/oder substituierten Reste im Kohlenstoffgerüst jeweils geradkettig oder verzweigt sein. Alkylreste, auch in den zusammengesetzten Bedeutungen wie Alkoxy, Haloalkyl usw., bedeuten Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet Fluor, Chlor, Brom oder Jod; Haloalkyl bedeutet Alkyl, das durch ein oder mehrere Atome aus der Gruppe Halogen substituiert ist; Haloalkyl ist beispielsweise CF₃, CHF₂, CH₂CF₃. Substituiertes Phenyl bedeutet vorzugsweise Phenyl, das durch einen oder mehrere, vorzugsweise 1, 2 oder 3 Reste aus der Gruppe Halogen, wie F, Cl, Br, I, vorzugsweise F, Cl und Br, ferner Alkyl, Haloalkyl, Alkoxy, Haloalkoxy, Hydroxy, Amino, Nitro, Cyano, Alkoxycarbonyl, Alkylcarbonyl, Formyl, Carbamoyl, Mono- und Dialkylaminocarbonyl, Mono- und Dialkylamino, Alkylsulfinyl und Alkylsulfonyl substituiert ist, und bei den Resten mit C-Atomen, solche mit 1 bis 4 C-Atomen, insbesondere 1 oder 2, bevorzugt sind. Bevorzugt sind dabei in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano.

Ein 5- oder 6-gliedriger heterocyclischer Rest, der gegebenenfalls benzokondensiert ist und ein N-Atom enthält (z.B. im Falle N(R¹²)₂), kann ein gesättigter, ungesättigter oder heteroaromatischer Rest sein, z.B. ein über das N-Atom gebundener Rest aus der Gruppe Pyrrolidyl, Piperidyl, Pyrazolyl, Morpholinyl, Indolyl, Chinolinyl, Pyrimidinyl, Triazolyl, Oxazolyl, Pyridyl, Pyrimidinyl, Pyridazinyl, Pyrazinyl, Thiazolyl, Oxazolyl, Pyrrolyl, Imidazolyl und Benzoxazolyl.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen.

Von besonderem Interesse sind erfindungsgemäße Verbindungen der Formel (1) oder deren Salze, worin
- R⁴: H, (C₁-C₄)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]-carbonyl und CN substituiert ist, oder (C₃-C₄)Alkenyl, (C₃-C₄)Alkinyl, Hydroxy, (C₁-C₄)Alkoxy, [(C₁-C₄)Alkyl]-carbonyl oder (C₁-C₄)Alkylsulfonyl, wobei jeder der letztgenannten fünf Reste unsubstituiert oder im Alkylteil durch ein oder mehrere Halogenatome substituiert ist, oder Phenylsulfonyl, wobei der Phenylrest unsubstituiert oder durch einen oder mehrere Reste aus der Gruppe Halogen, (C₁-C₄)Alkyl und (C₁-C₄)Alkoxy substituiert ist, und
- R⁵: (C₁-C₄)Alkylsulfonyl, (C₁-C₄)Haloalkylsulfonyl, Phenylsulfonyl oder Phenylcarbonyl, wobei der Phenylrest in den beiden letztgenannten Resten unsubstituiert oder durch einen oder mehrere Reste aus der Gruppe Halogen, (C₁-C₄)Alkyl und (C₁-C₄)Alkoxy substituiert ist, oder Mono- oder Di-[(C₁-C₄)Alkyl]-aminosulfonyl, [(C₁-C₆)Alkyl]-carbonyl, das unsubstituiert oder durch ein oder mehrere Halogenatome oder (C₁-C₄)Alkoxy und (C₁-C₄)Alkylthio substituiert ist, oder Formyl, OHC-CO-, 2-Oxo-(C₃-C₅)alkanoyl, [(C₁-C₄)Alkoxy]-oxalyl, [(C₃-C₆)Cycloalkyl]-carbonyl, [(C₂-C₄)Alkenyl]-carbonyl oder [(C₂-C₄)Alkinyl]-carbonyl oder eine Gruppe der Formel 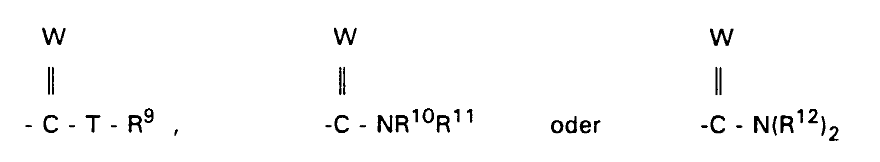
- W: O oder S,
- R⁶: H, (C₁-C₄)Alkyl, (C₁-C₄)Haloalkyl, (C₁-C₄)Alkoxy, (C₁-C₄)-Haloalkoxy oder Halogen,
- R⁸: unabhängig voneinander (C₁-C₄)Alkyl, das unsubstituiert oder durch ein oder mehrere Halogenatome substituiert ist, oder (C₃-C₄)-Alkenyl oder (C₃-C₄)Alkinyl,
- R⁹: H, (C₁-C₄)Alkyl, das unsubstituiert oder durch ein oder mehrere Halogenatome oder durch (C₁-C₄)Alkoxy, (C₁-C₄)Alkylthio, [(C₁-C₄)Alkoxy]-carbonyl und [(C₁-C₄)Alkyl]-carbonyl substituiert ist,
- R¹⁰, R¹¹: unabhängig voneinander H, (C₁-C₄)Alkyl, das unsubstituiert oder durch ein oder mehrere Halogenatome substituiert ist, oder (C₃-C₄)-Alkenyl oder (C₃-C₄)Alkinyl, wobei mindestens einer der Reste R¹⁰ und R¹¹ von Wasserstoff verschieden ist,
- die Reste R¹²: gemeinsam mit dem N-Atom einen heterocyclischen Ring mit 5 oder 6 Ringgliedern, der ein weiteres Heteroatom aus der Gruppe N, O und S in den verschiedenen Oxidationsstufen enthalten kann und unsubstituiert oder durch (C₁-C₄)Alkyl oder die Oxogruppe substituiert ist, und
- R¹³: unabhängig voneinander H, (C₁-C₄)Alkyl, das unsubstituiert oder durch ein oder mehrere Halogenatome substituiert ist, oder (C₃-C₄)-Alkenyl oder (C₃-C₄)Alkinyl
bedeuten.

Bevorzugte Verbindungen der Formel (I) oder deren Salze sind solche, bei denen W ein Sauerstoffatom und A einen Rest der Formel 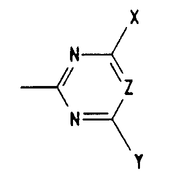 bedeuten, worin X, Y und Z wie oben beschrieben definiert sind.

Besonders bevorzugte Verbindungen der Formel (I) oder deren Salze sind solche, worin
- R⁴: H, (C₁-C₄)Alkyl, Hydroxy oder (C₁-C₄)Alkoxy,
- R⁵: (C₁-C₄)Alkylsulfonyl, CHO, [(C₁-C₄)Alkyl]-carbonyl, das unsubstituiert oder durch ein oder mehrere Halogenatome substituiert ist, oder [(C₁-C₄)Alkoxy]-oxalyl, [(C₃-C₆)Cycloalkyl]-carbonyl oder eine Gruppe der Formel 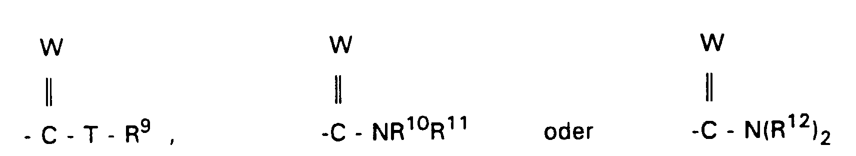 wobei
W, T, R⁹ bis R¹² wie oben in Formel (I) definiert sind,
- R⁶: H, (C₁-C₄)Alkyl, (C₁-C₃)Alkoxy oder Halogen,
- A: einen Rest der Formel 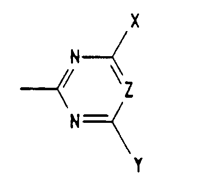
- Z: CH oder N, vorzugsweise CH, und
einer der Reste X und Y Halogen, (C₁-C₂)Alkyl, (C₁-C₂)Alkoxy, OCF₂H, CF₃ oder OCH₂CF₃ und der andere der Reste X und Y (C₁-C₂)Alkyl, (C₁-C₂)Alkoxy oder (C₁-C₂)Haloalkoxy
bedeuten.

Aus Gründen der besseren Herstellbarkeit oder der besseren biologischen Wirkung sind erfindungsgemäße Verbindungen der Formel (II) oder deren Salze, in denen die Gruppe -CR²R³-NR⁴R⁵ in Ortho- oder Parastellung zur Gruppe R¹ oder in Orthostellung zur Sulfogruppe steht, von besonderem Interesse; bevorzugt ist dabei die Parastellung der Gruppe -CR²R³-NR⁴R⁵ zur Gruppe R¹.

Gegenstand der vorliegenden Erfindung sind auch Verfahren zur Herstellung der Verbindungen der allgemeinen Formel (I) oder deren Salze, dadurch gekennzeichnet, daß man
a) eine Verbindung der Formel (II) 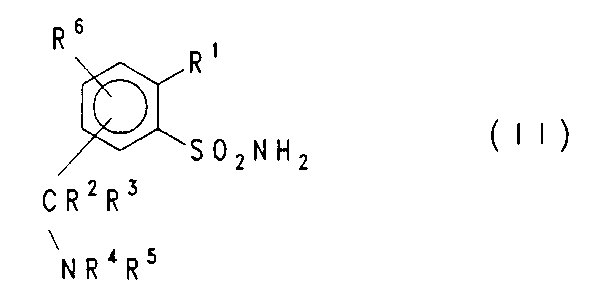 mit einem heterocyclischen Carbamat der Formel (III),

   R* - O - CO - NR⁷ - A (III)

   worin R* gegebenenfalls substituiertes Phenyl oder (C₁-C₄)Alkyl bedeutet, umsetzt oder
b) ein Phenylsulfonylcarbamat der Formel (IV) 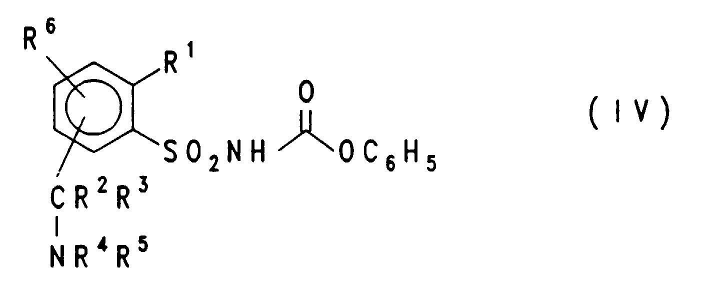 mit einem Aminoheterocyclus der Formel (V)

   H - NR⁷ - A (V)

   umsetzt oder
c) ein Sulfonylisocyanat der Formel (Vl) 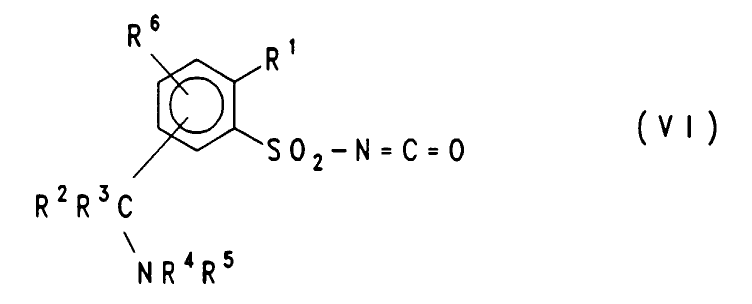 mit einem Aminoheterocyclus der Formel H-NR⁷-A (V) umsetzt oder
d) in einer Eintopfreaktion zunächst einen Aminoheterocyclus der Formel H-NR⁷-A (V) in Gegenwart einer Base mit Phosgen umsetzt und das gebildete Intermediat mit einem Phenylsulfonamid der Formel (II) umsetzt oder
e) ein Sulfonylchlorid der Formel (VII) 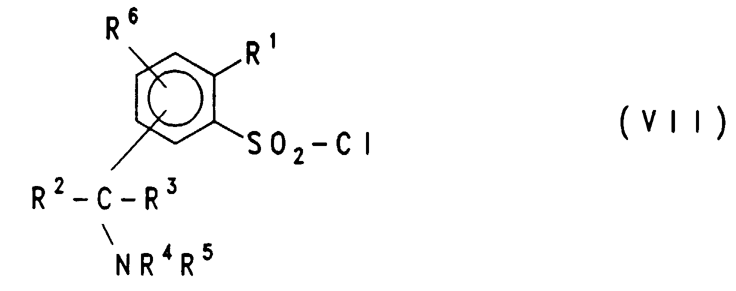 mit einem Cyanat M-OCN, worin M = NH₄, Na oder K bedeutet, und mit einem Aminoheterocyclus der Formel H-NR⁷-A (V) in Gegenwart einer Base umsetzt, oder
f) ein Sulfonamid der genannten Formel (II) mit einem (Thio-)lsocyanat der Formel (V')

   W = C = N - A (V')

   in Gegenwart einer Base umsetzt,
wobei in den Formeln (II)-(VII) und (V') die Reste bzw. Gruppen R¹-R⁷, A oder W wie in Formel (I) definiert sind und wobei in den Varianten a)-e) zunächst Verbindungen der Formel (I) mit W = O erhalten werden.

Die Umsetzung der Verbindungen der Formeln (II) und (III) erfolgt vorzugsweise basenkatalysiert in einem inerten organischen Lösungsmittel, wie z.B. Dichlormethan, Acetonitril, Dioxan oder THF bei Temperaturen zwischen 0°C und dem Siedepunkt des Lösungsmittels. Als Base werden dabei beispielsweise organische Aminbasen, wie 1,8-Diazabicyclo[5.4.0]undec-7-en (DBU), insbesondere bei R* = (subst.) Phenyl (vgl. EP-A-44807), oder Trimethylaluminium oder Triethylaluminium, letztere insbesondere bei R* = Alkyl (vgl. EP-A-166516) verwendet.

Die Sulfonamide (II) sind neue Verbindungen. Sie und ihre Herstellung sind ebenfalls Gegenstand dieser Erfindung.

Am Beispiel der Verbindung (II) mit R² = R³ = H und Q = O werden im folgenden Herstellungsmöglichkeiten näher erläutert, die mit geringfügigen Modifikationen auch für Verbindungen mit R², R³ ungleich Wasserstoff bzw. Q ungleich O eingesetzt werden können.

Ausgehend von gegebenenfalls substituierten Methylanilinsulfonsäuren (VIII) (s. DRP 48583, S. 242), die zunächst durch Diazotierung und Umsetzung mit KCN/CuCN nach Sandmeyer zu den Nitrilen (IX) umgesetzt werden, erhält man nach Verseifung des Nitrils, Veresterung der Carbonsäuregruppe und Überführung der Sulfonsäuregruppe in die tert.-butylgeschützte Sulfonamidgruppe analog literaturbekannter Verfahren das Sulfonamid (X) als Zwischenprodukt (siehe Schema 1).

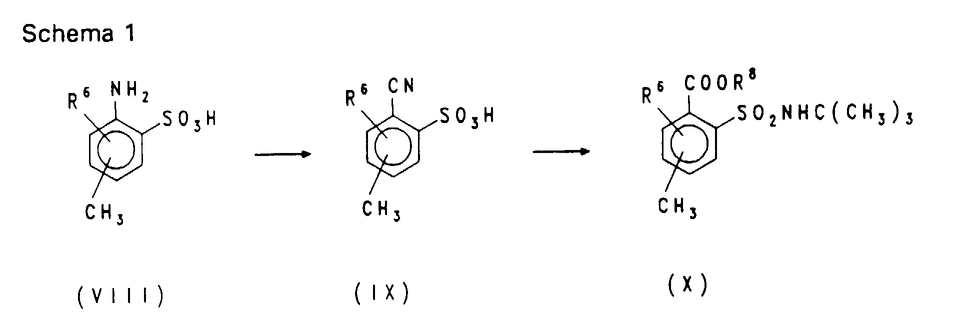

Desweiteren erhält man Sulfonamide (X) ausgehend von gegebenenfalls substituierten Methylnitrobenzoesäuren (XI) via Veresterung, Reduktion der Nitrogruppe, Diazotierung und Kupplung mit SO₂/CuCl (s. H. Meerwein et al., Chem. Ber. 90, 841-1178 (1957)) und Aminolyse mit tert.-Butylamin (siehe Schema 2).

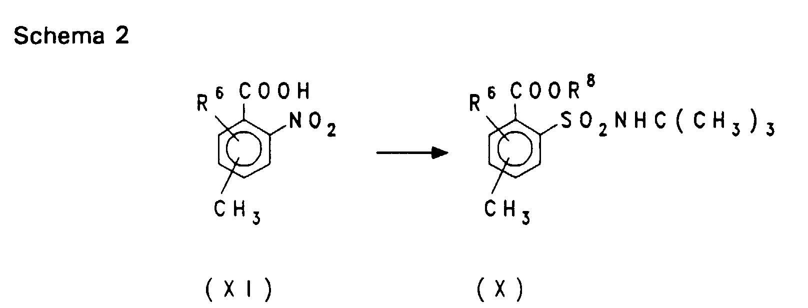

Ebenso lassen sich Sulfonamide (X) ausgehend von gegebenenfalls substituierten Methylnitroanilinen (XII) darstellen. Diazotierung und Kupplung mit KCN/CuCN liefert die entsprechenden Nitrile, die durch Hydrolyse der Cyanogruppe und Veresterung zu Nitrobenzoesäureestern (XIII) umgesetzt werden können.

Die Nitrogruppe kann dann, wie zu Schema 2 beschrieben, in die tert.-Butylaminosulfonylgruppe umgewandelt werden (siehe Schema 3).

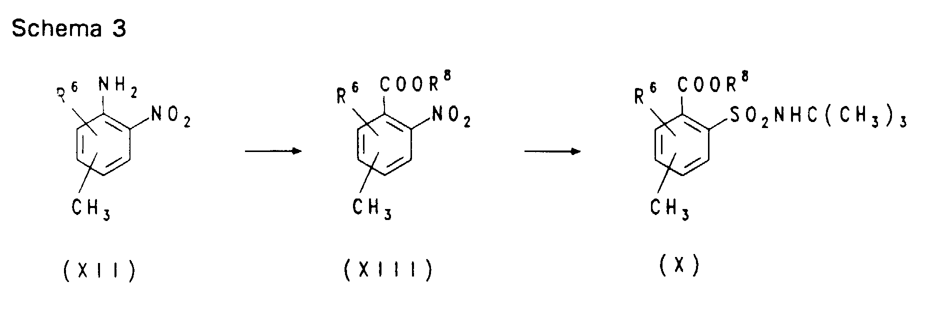

Zur Herstellung der Sulfonamide (II) werden Verbindungen der Formel (X) via Seitenkettenhalogenierung zu (XIV), anschliessender Substitution des Halogenatoms in (XIV) gegen Amine oder gegen Azid mit nachfolgender Reduktion zu Benzylaminen (XV') und weiterer Funktionalisierung der Aminogruppe sowie Abspaltung der tert. Butylschutzgruppe analog bekannter Verfahrensweise (z.B. mit CF₃COOH) zu den Sulfonamiden (II') umgesetzt (siehe Schema 4).

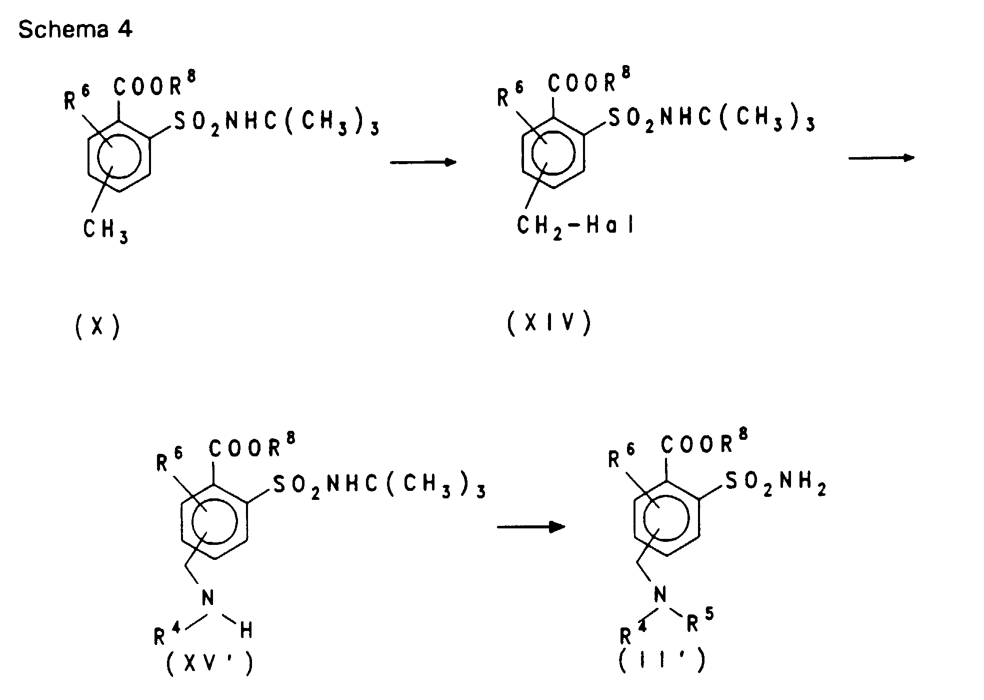

Die Verfahrensweise läßt sich analog auch zur Herstellung anderer Verbindungen der Formel (II) einsetzen, wobei im letzten Schritt die Verbindung der Formel (XV) 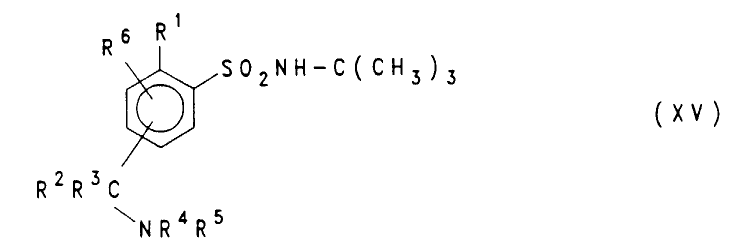 eingesetzt wird.

Die Carbamate der Formel (III) können nach Methoden hergestellt werden, die in den südafrikanischen Patentanmeldungen 82/5671 und 82/5045 bzw. EP-A 70804 (US-A-4 480 101) oder RD 275056 beschrieben sind.

Die Umsetzung der Verbindungen (IV) mit den Aminoheterocyclen (V) führt man vorzugsweise in inerten, aprotischen Lösungsmitteln wie z.B. Dioxan, Acetonitril oder Tetrahydrofuran bei Temperaturen zwischen 0°C und der Siedetemperatur des Lösungsmittels durch. Die benötigten Ausgangsmaterialien (V) sind literaturbekannt oder können nach literaturbekannten Verfahren hergestellt werden. Die Phenylsulfonylcarbamate der Formel (IV) erhält man analog US-PS-4 684 393 oder US-PS-4 743 290.

Die Phenylsulfonylisocyanate der Formel (VI) lassen sich analog US-PS-4 481 029 herstellen und mit den Aminoheterocyclen (V) umsetzen.

Die Phosgenierung von Verbindungen der Formel (V) nach Variante d) kann vorzugsweise in Gegenwart von Basen, wie sterisch gehinderten organischen Aminbasen, beispielsweise Triethylamin, erfolgen. Die anschließende Umsetzung mit Verbindungen der Formel (II) nach Variante d) kann analog bekannten Verfahren durchgeführt werden (vgl. EP-A-232 067).

Die Sulfochloride (VII) können aus entsprechenden Sulfonsäuren erhalten werden, z.B. nach Standardmethoden wie der Umsetzung des Kaliumsalzes mit Phosphoroxychlorid oder Thionylchlorid in inerten Solventien wie Acetonitril und/oder Sulfolan oder in Substanz durch Erwärmen zum Rückfluß (vgl. Houben-Weyl-Klamann, "Methoden der organischen Chemie", 4. Aufl., Bd. E XI/2, S. 1067-1073, Thieme Verlag Stuttgart, 1985).

Die entsprechenden Sulfonsäuren sind aus entsprechenden Nitroverbindungen analog der Umsetzung von Verbindungen (Xl) erhältlich.

Alternativ sind in Einzelfällen Sulfochloride (VII) durch Sulfonierung (+ Chlorierung) oder Sulfochlorierung geeigneter substituierter Benzoesäureester herstellbar; Sulfochlorierung analog Houben-Weyl-Klamann, "Methoden der organischen Chemie", 4. Aufl., Bd E XI/2, S. 1067ff., Thieme Verlag Stuttgart, 1985; Houben-Weyl-Müller, "Methoden der organischen Chemie", 4. Aufl., Bd. IX, S. 563ff., Thieme Verlag Stuttgart, 1955; Sulfonierung analog Houben-Weyl-Klamann, "Methoden der organischen Chemie, 4. Aufl., Bd. E XI/2, S. l055ff., Thieme Verlag Stuttgart, 1985; Houben-Weyl-Müller, "Methoden der organischen Chemie", 4. Aufl., Bd. IX, S. 435ff., Thieme Verlag Stuttgart, 1955.

Die (Thio-)Isocyanate der Formel (V') sind nach literaturbekannten Verfahren erhältlich (EP-A-232067, EP-A-166516). Die Umsetzung der (Thio)-lsocyanate (V') mit Verbindungen (II) erfolgt bei -10°C bis 100°C, vorzugsweise 20 bis 100°C, in einem inerten aprotischen Lösungsmittel, wie z.B. Aceton oder Acetonitril, in Gegenwart einer geeigneten Base, z.B. N(C₂H₅)₃ oder K₂CO₃.

Die Salze der Verbindungen der Formel (I) werden vorzugsweise in inerten polaren Lösungsmitteln wie z.B. Wasser, Methanol oder Aceton bei Temperaturen von 0-100°C hergestellt. Geeignete Basen zur Herstellung der erfindungsgemäßen Salze sind beispielsweise Alkalicarbonate, wie Kaliumcarbonat, Alkali- und Erdalkalihydroxide, z. B. NaOH oder KOH, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono-und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyo>:ethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.
Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d. h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d. h. Ammoniumsulfamat; anilofos; asulam; atrazin; azimsulfurone (DPX-A8947); aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoyl-prop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d. h. 2-Chlor-N,N-di-2-propenylacetamid; CDEC, d. h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyl)-N-methyl-1 H-pyrazole-4-carboxamid; endothal; EPTC; esprocarb; ethalfluralin; ethametsulfuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosaten; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d. h. N-[3-Chlor-4-(1-methylethyl)-phenyl]-2-methylpentanamid; naproanilide; napropamide; naptalam; NC 310, d. h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d. h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d. h. 2-[[7-[2-Chlor-4-(trifluor-methyl)-phenoxy]-2-naphthalenyl]-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d. h. N,N-Diethyl-3-[(2-ethyl-6-methylphenyl)-sulfonyl]-1H-1,2,4-triazol-1-carboxamid; thenylchlor (NSK-850); thiazafluron; thizopyr (Mon-13200); thidiazimin (SN-124085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d. h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A. Chemische Beispiele

- Abkürzungen:: Die %-Angaben und Mengenverhältnisse beziehen sich auf das Gewicht, wenn nicht näher spezifiziert.
i. Vak. = unter reduziertem Druck
h = Stunde(n)

### Beispiel A1

### N-tert-Butyl-5-brommethyl-2-methoxycarbonylbenzolsulfonamid

Eine Lösung von 54,8 g (192 mmol) N-tert-Butyl-2-methoxycarbonyl-5-methylbenzolsulfonamid in 420 ml Tetrachlormethan wird unter einer Stickstoffschutzgasatmosphäre nach Zugabe von 36 g (202 mmol) N-Bromsuccinimid und 0,5 g Azobisisobutyronitril (AIBN) unter gleichzeitiger Bestrahlung mit einer Tageslichtlampe 6-8 h am Rückfluß erhitzt. Die Lösung wird danach filtriert und sukzessive mit Natriumbisulfitlösung, Natriumhydrogencarbonatlösung und Wasser gewaschen, über Na₂SO₄ getrocknet und i. Vak. vollständig eingeengt. Kristallisation des Rückstandes aus Diisopropylether/Essigsäureethylester liefert 41,9 g (57 %) N-tert-Butyl-5-brommethyl-2-methoxycarbonylbenzolsulfonamid vom Schmp. 88-90°C.

### Beispiel A2

### N-tert-Butyl-5-azidomethyl-2-methoxycarbonylbenzolsulfonamid

Eine Lösung von 25,5 g (70 mmol) N-tert-Butyl-5-brommethyl-2-methoxycarbonylbenzolsulfonamid und 5,9 g (90 mmol) Natriumazid in 240 ml Ethanol wird 6 h am Rückfluss erhitzt. Anschließend wird vollständig eingeengt und der Rückstand wird extraktiv mit Wasser/Essigsäureethylester aufgearbeitet. Digerieren des Rohproduktes mit Diisopropylether ergibt 16,6 g (72,5 %) N-tert-Butyl-5-azidomethyl-2-methoxycarbonylbenzolsulfonamid vom Schmp. 63-65°C.

### Beispiel A3

### N-tert-Butyl-5-aminomethyl-2-methoxycarbonylbenzolsulfonamid

16,3 g (50 mmol) N-tert-Butyl-5-azidomethyl-2-methoxycarbonyl-benzolsulfonamid wird in 300 ml Methanol gelöst und über Pd/C (5 %) hydriert. Der Ansatz wird filtriert und vollständig eingeengt. Das anfallende Rohprodukt wird durch Elution von einer Kieselgelsäule mit Essigsäureethylester/Methanol 4:1 gereinigt. Man erhält 11,2 g (74 %) N-tert-Butyl-5-aminomethyl-2-methoxycarbonylbenzolsulfonamid als zähes Öl.
- ¹H NMR (100 MHz, CDCl₃): δ =: 1,25 (s, 9H, C(CH₃)₃),
1,80 (bs, 2H, NH₂),
3,95 (s, 3H, OCH₃),
4,00 (s, 2H, Ar-CH₂),
6,20 (bs, 1H, SO₂NH),
7,58 (dd, 1H, Ar-H),
7,80 (d, 1H, Ar-H),
8,10 (dd, 1H, Ar-H).

### Beispiel A4

### N-tert-Butyl-5-acetamidomethyl-2-methoxycarbonylbenzolsulfonamid

Zu einer auf 0°C gekühlten Lösung von 2,01 g (6,7 mmol) N-tert-Butyl-5-aminomethyl-2-methoxycarbonylbenzolsulfonamid und 0,93 ml (6,7 mmol) Triethylamin in 30 ml Dichlormethan tropft man 0,63 g (8 mmol) Acetylchlorid, gelöst in 10 ml Dichlormethan, und läßt anschließend 2 h bei Raumtemperatur nachrühren. Die Reaktionslösung wird mit Wasser gewaschen, getrocknet und i. Vak. vollständig eingeengt. Man erhält 2,1 g (91 %) N-tert-Butyl-5-acetamidomethyl-2-methoxycarbonylbenzolsulfonamid als zähes Öl.
- ¹H NMR (100 MHz, CDCl₃): δ =: 1,25 (s, 9H, C(CH₃)₃),
2,05 (s, 3H, COCH₃),
3,95 (s, 3H, OCH₃),
4,50 (s, 2H, Ar-CH₂),
6,15 (s, 1H, SO₂NH),
6,38 (bt, 1H, NH),
7,47 (dd, 1H, Ar-H),
7,75 (d, 1H, Ar-H),
7,95 (dd, 1H, Ar-H).

### Beispiel A5

### 5-Acetamidomethyl-2-methoxycarbonylbenzolsulfonamid

Eine Lösung von 2,09 g (6,1 mmol) N-tert-Butyl-5-acetamidomethyl-2-methoxycarbonylbenzolsulfonamid in 25 ml Trifluoressigsäure wird 14 h bei Raumtemperatur gerührt und dann vollständig eingedampft. Kristallisation des Rückstandes aus Essigsäureethylester liefert 1,33 g (76 %) 5-Acetamidomethyl-2-methoxycarbonylbenzolsulfonamid vom Schmp. 173-175°C.

### Beispiel A6

### N-[4,6-Dimethoxypyrimidin-2-yl)aminocarbonyl]-5-acetamidomethyl-2-methoxycarbonylbenzolsulfonamid

Zu einer Suspension von 1,3 g (4,54 mmol) 5-Acetamidomethyl-2-methoxycarbonylbenzolsulfonamid und 1,25 g (4,54 mmol) N-(4,6-Dimethoxypyrimidin-2-yl)phenylcarbamat in 20 ml Acetonitril gibt man bei 0°C 0,69 g (4,54 mmol) 1,8-Diazabicyclo[5,4,0]undec-7-en (DBU). Nach 2 h bei Raumtemperatur wird mit Wasser und Diethylether verdünnt, mit Salzsäure auf pH 1-2 angesäuert und der ausgefallene Niederschlag abfiltriert und getrocknet. Man erhält 1,32 g (62 %) N-[(4,6-Dimethoxypyrimidin-2-yl)aminocarbonyl]-5-acetamidomethyl-2-methoxycarbonylbenzolsulfonamid vom Schmp. 149-150°C.

Die in den folgenden Tabellen 1-4 aufgeführten Verbindungen der Formel (I) (W = O, A = Pyrimidinyl oder Triazinyl) werden analog zu den Beispielen A1 bis A6 erhalten. In den Tabellen bedeuten:
"Fp." = Schmelzpunkt in °C;
Vb. = Verbindungsnummer = Beispielsnummer.
Me = Methyl = CH₃
Et = Ethyl
Bu = n-Bu = n-Butyl = n-C₄H₉
Ph = Phenyl

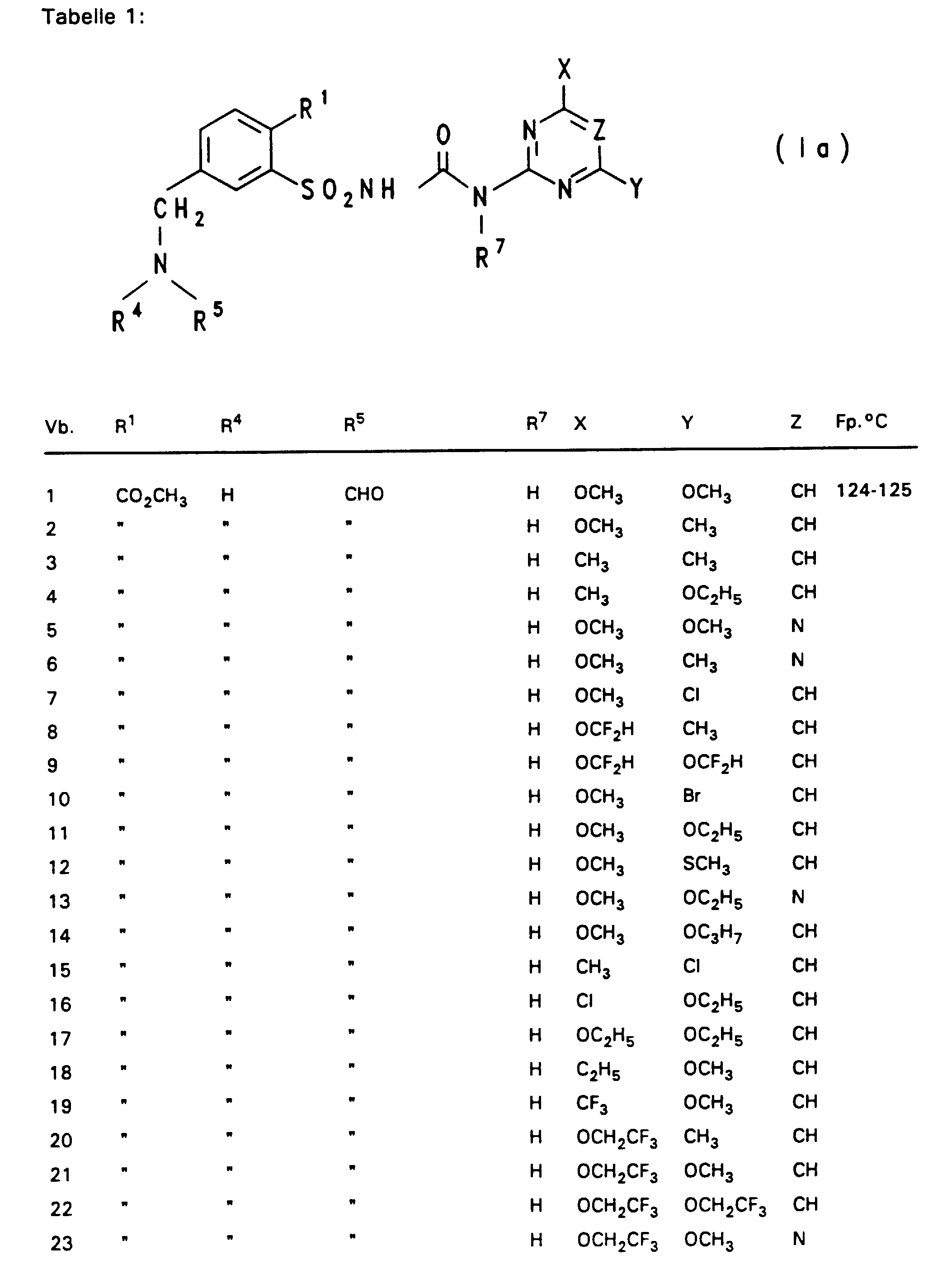

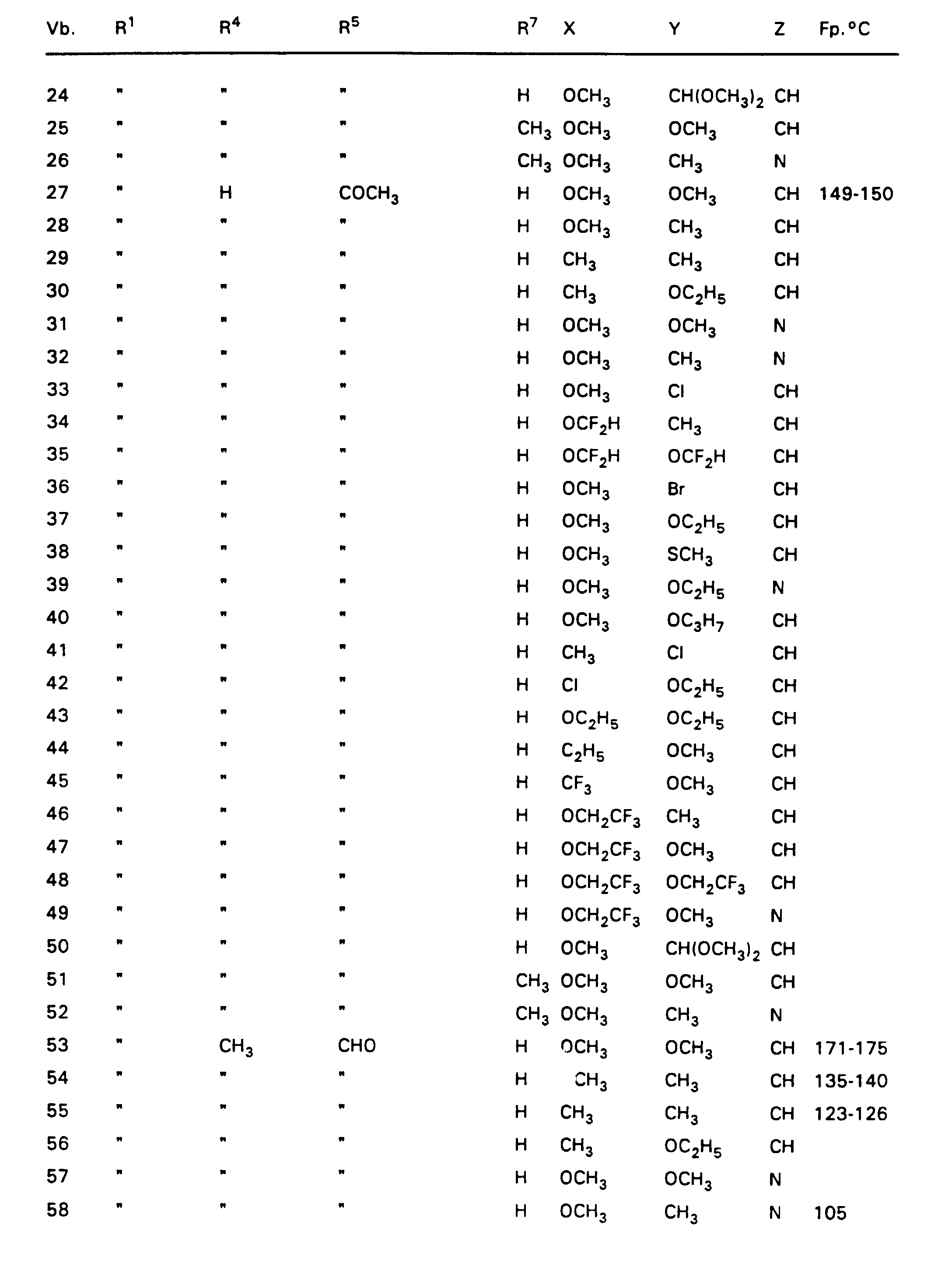

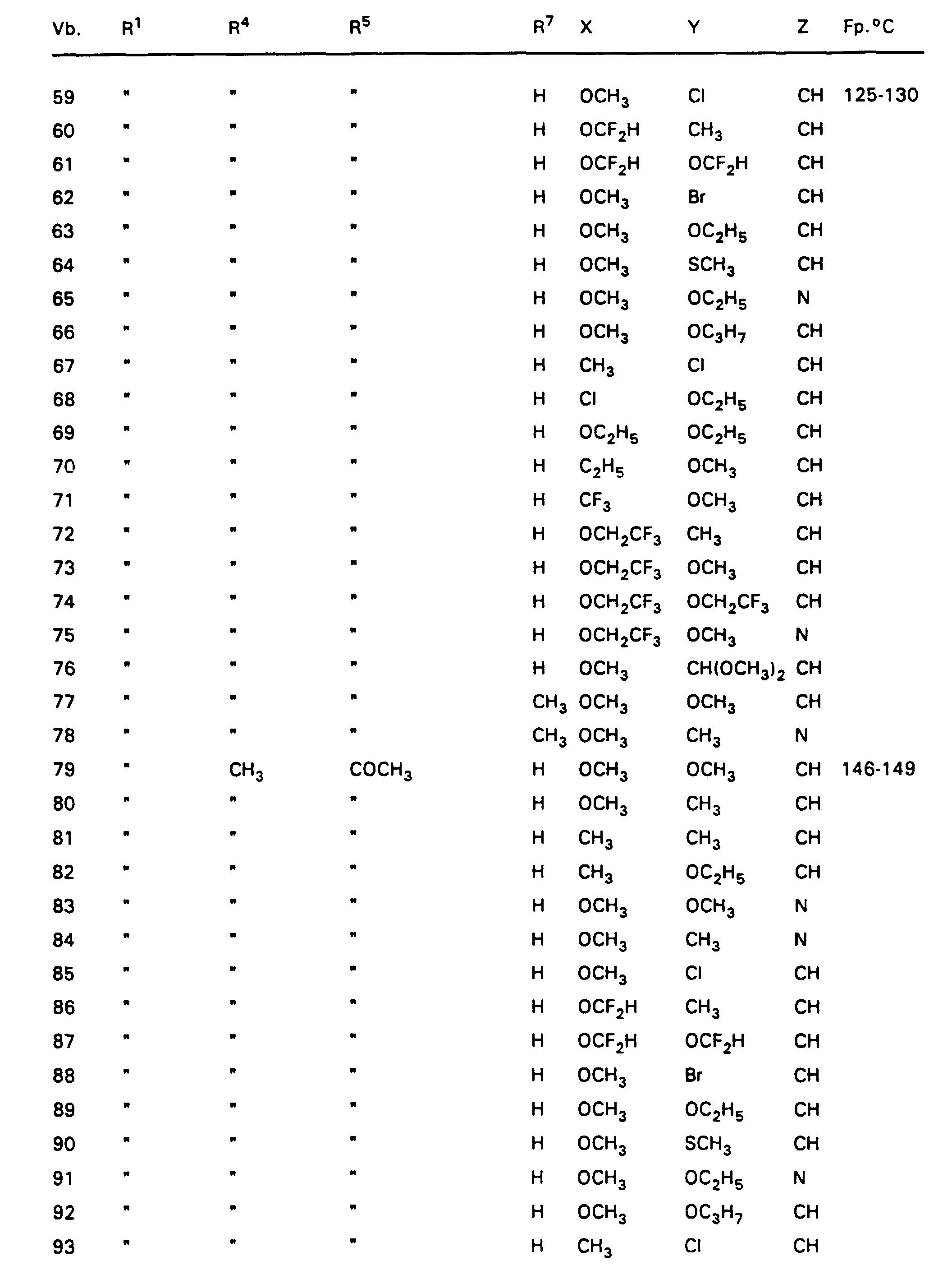

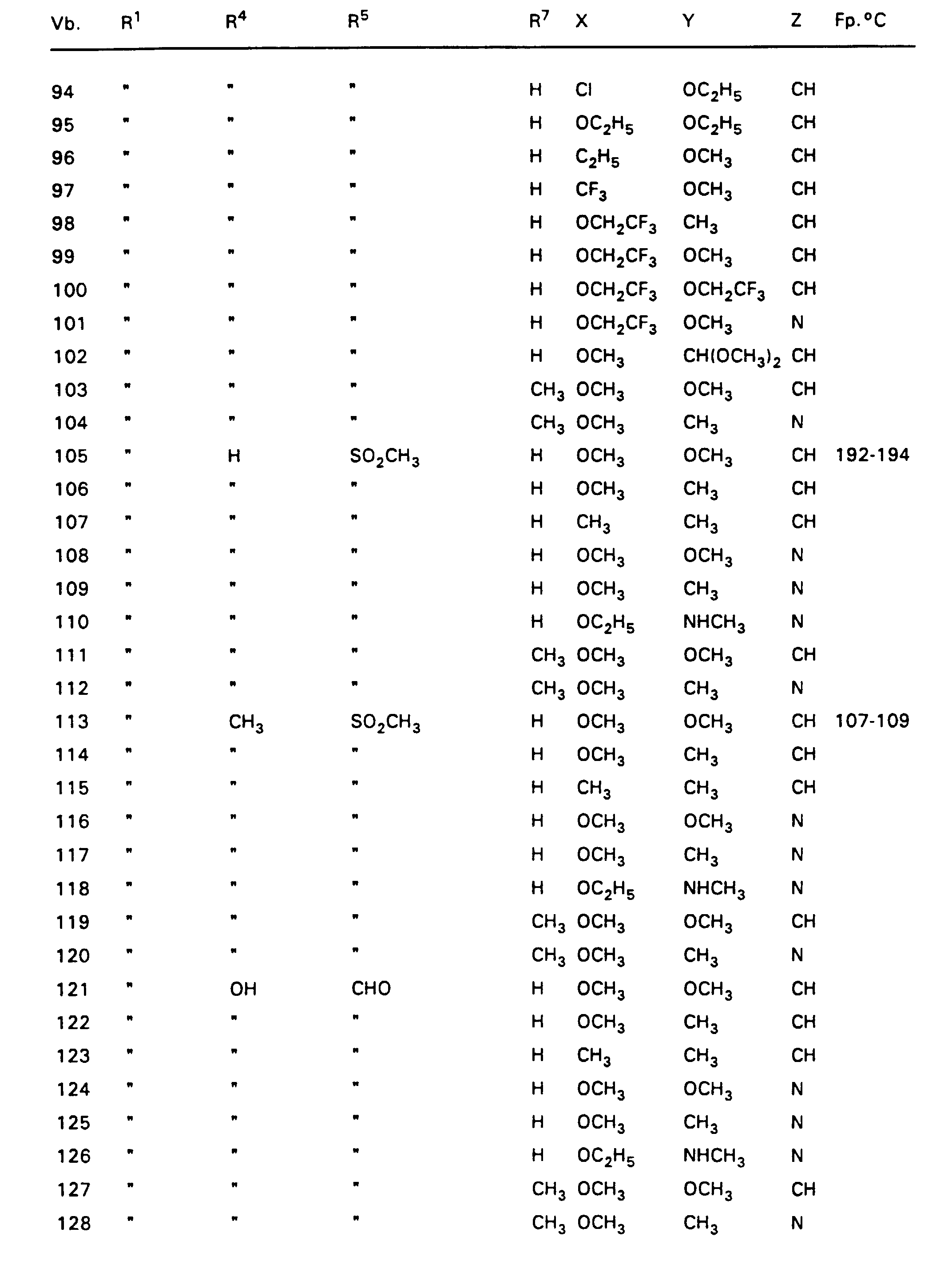

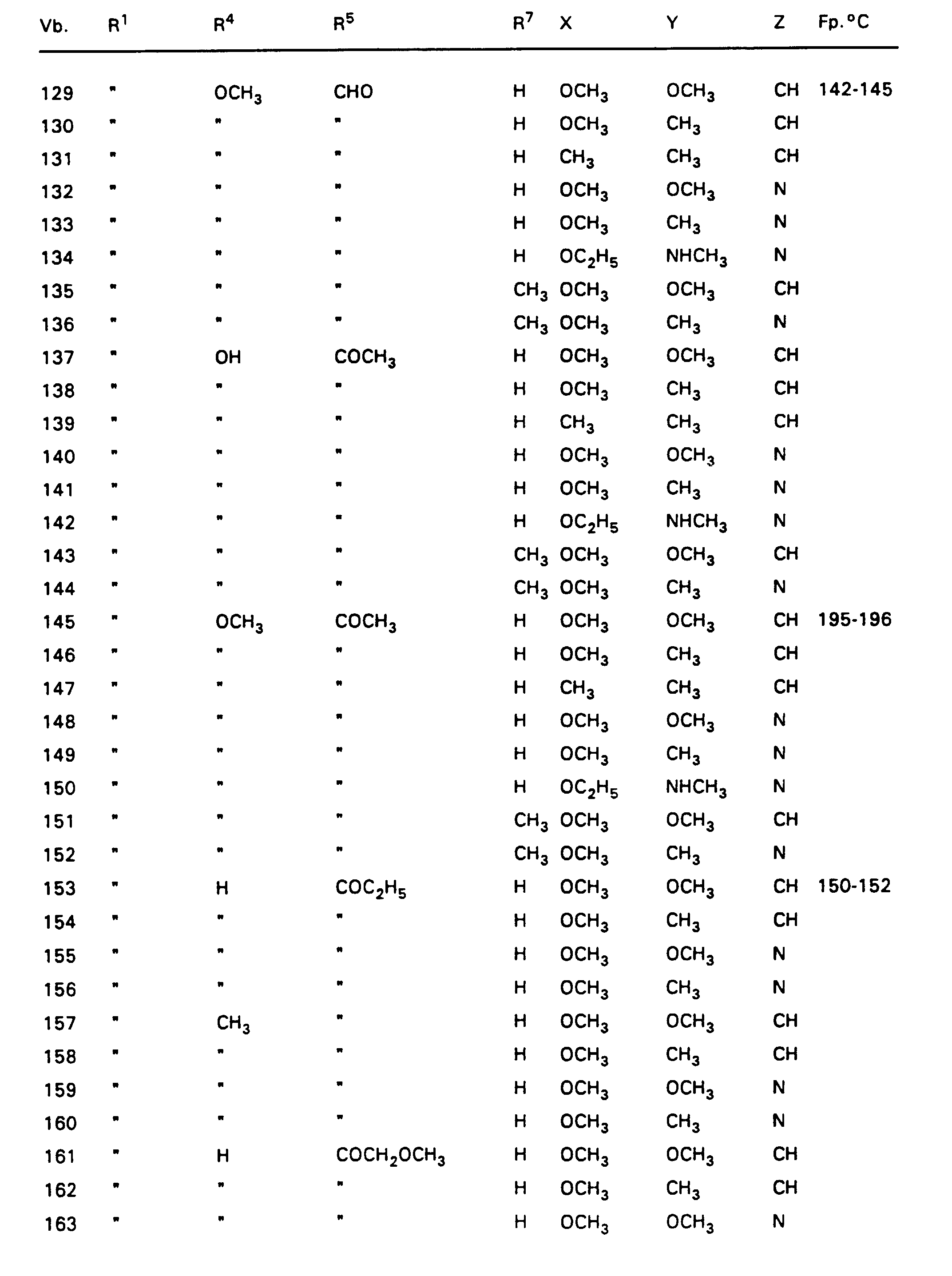

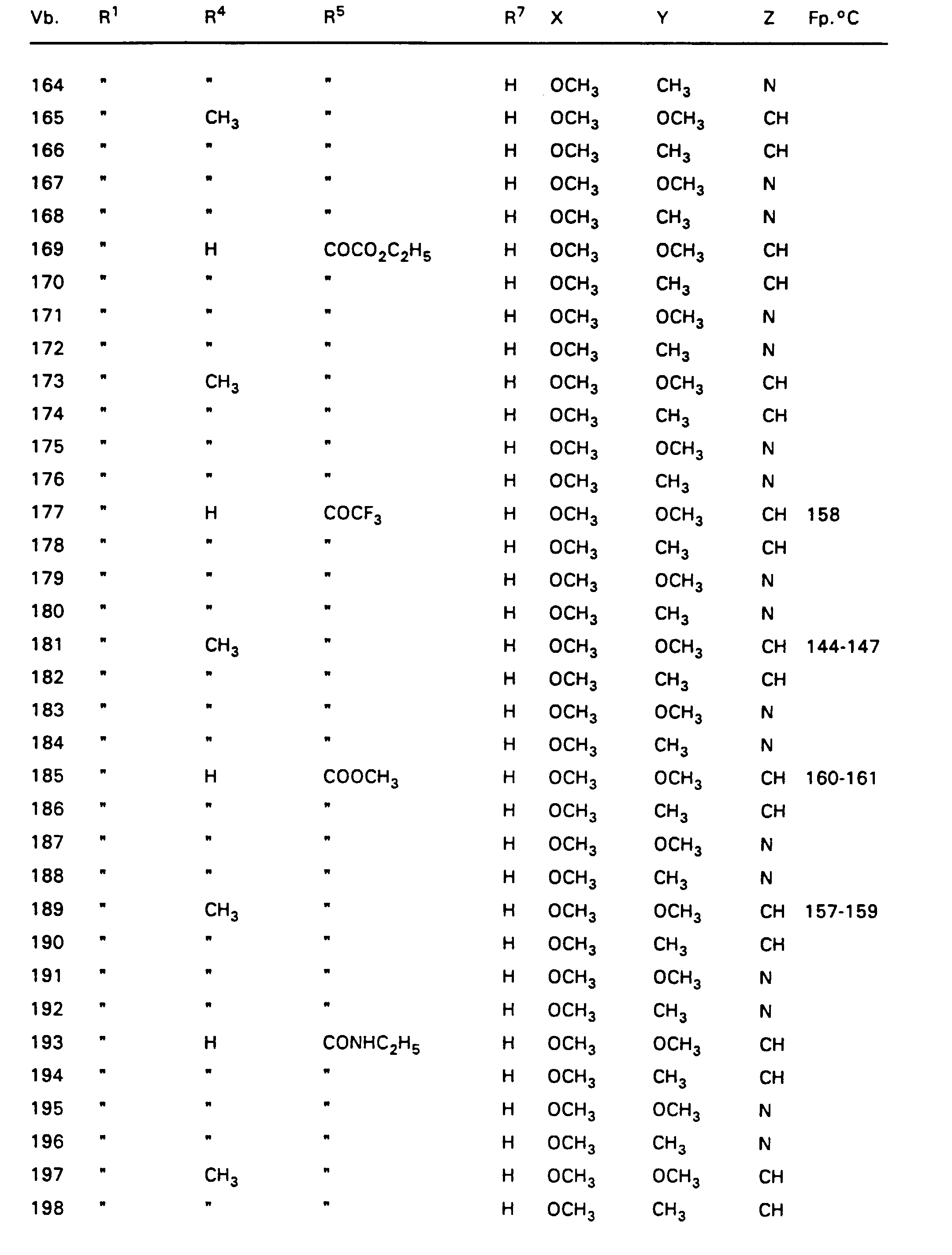

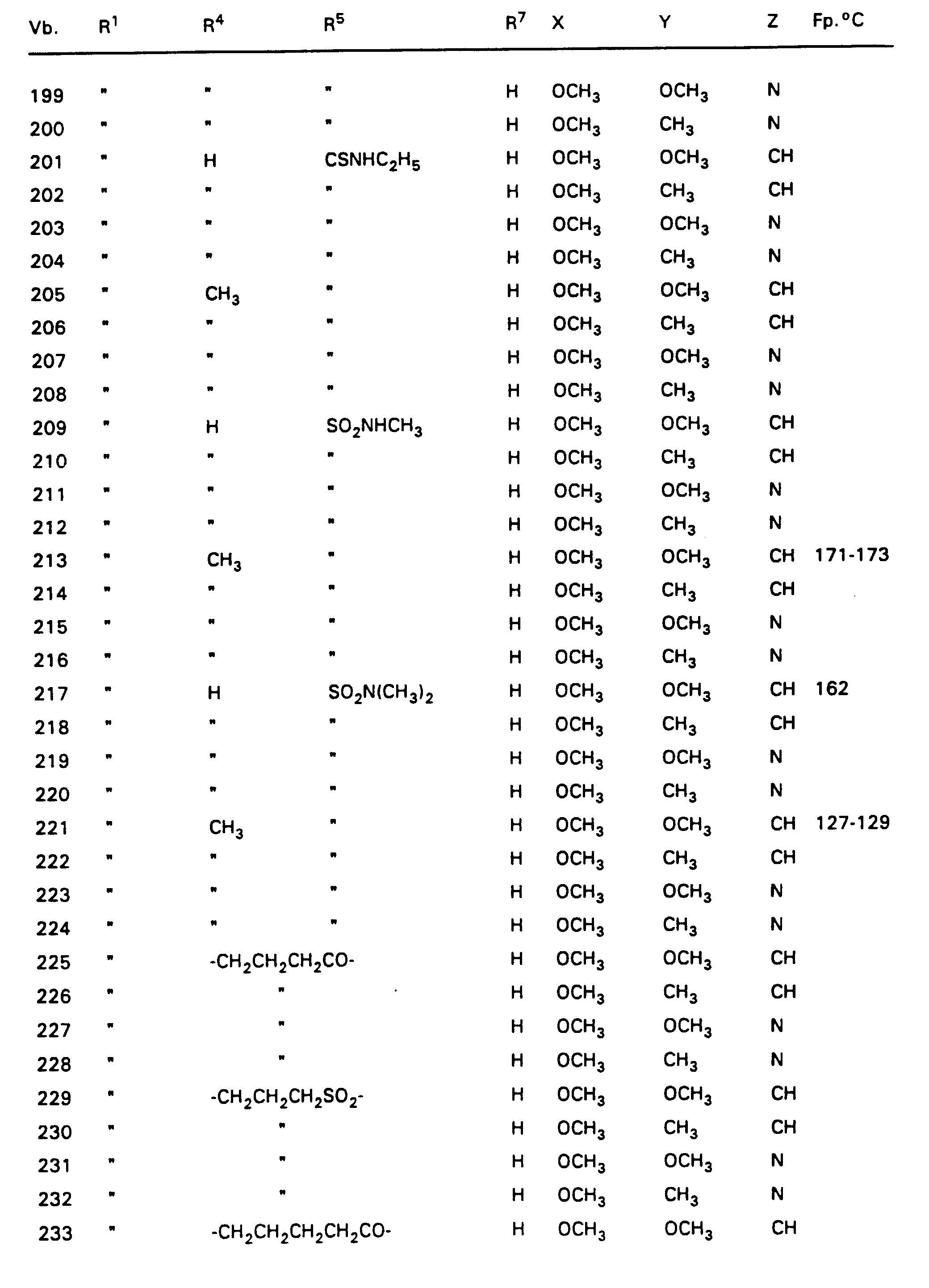

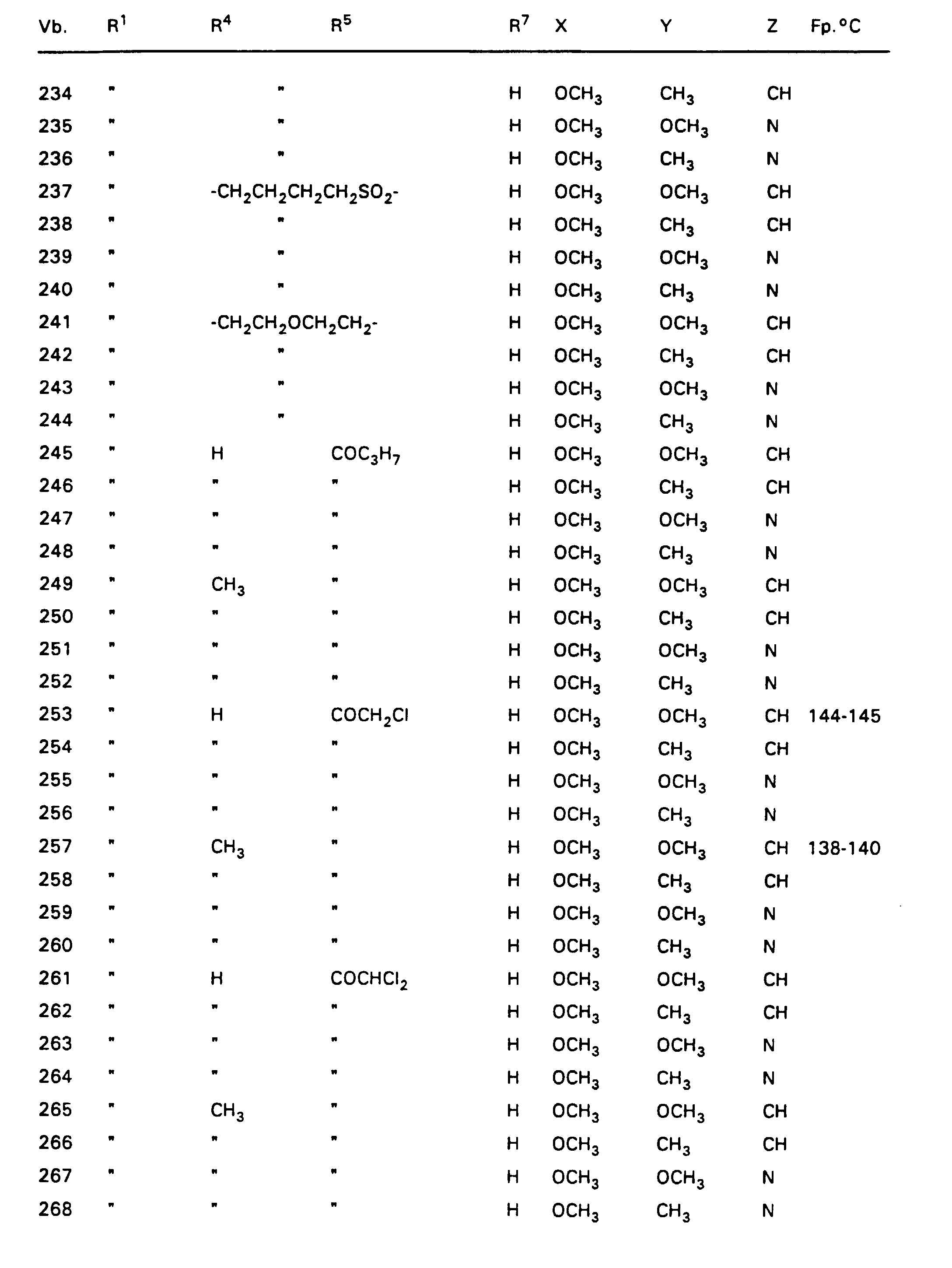

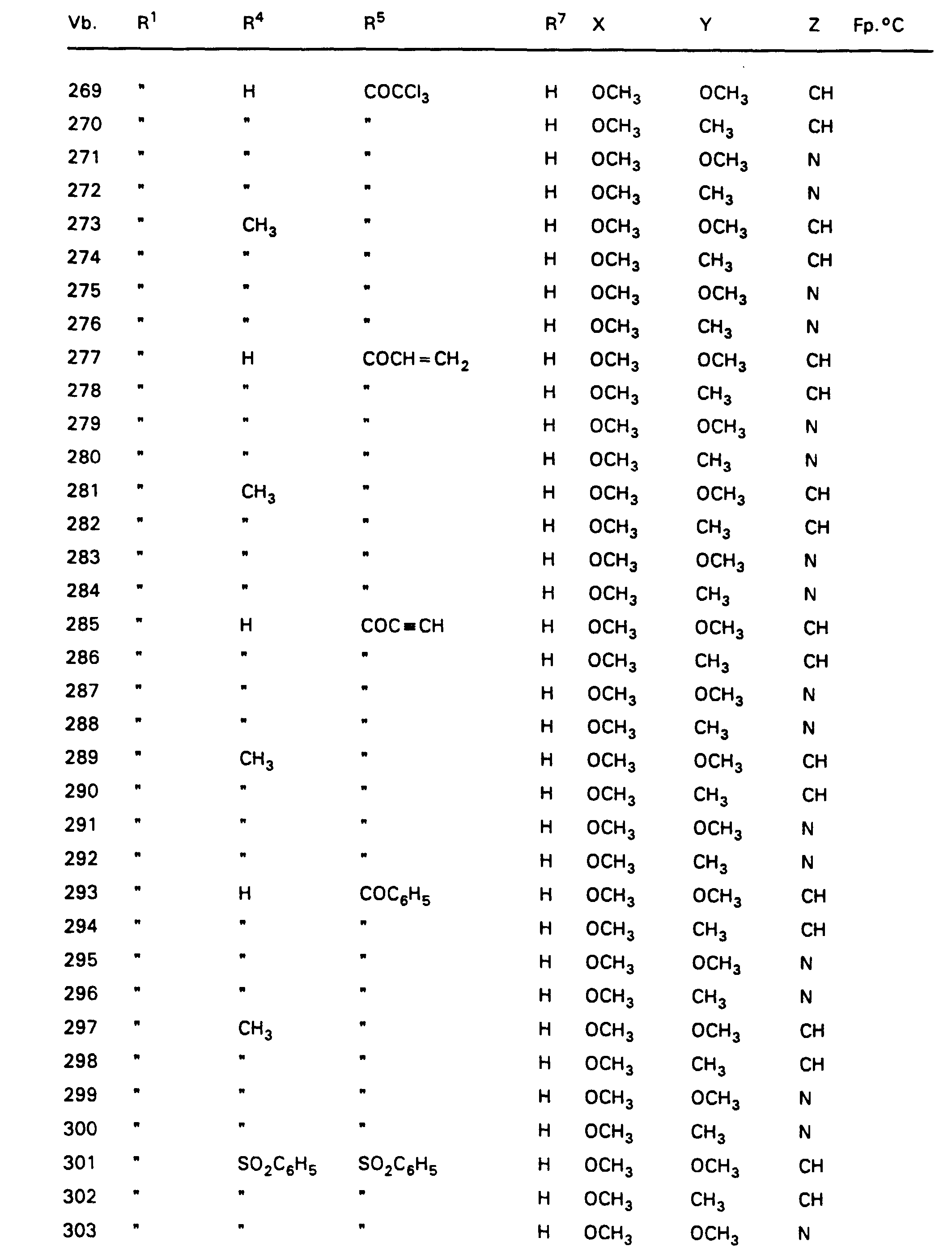

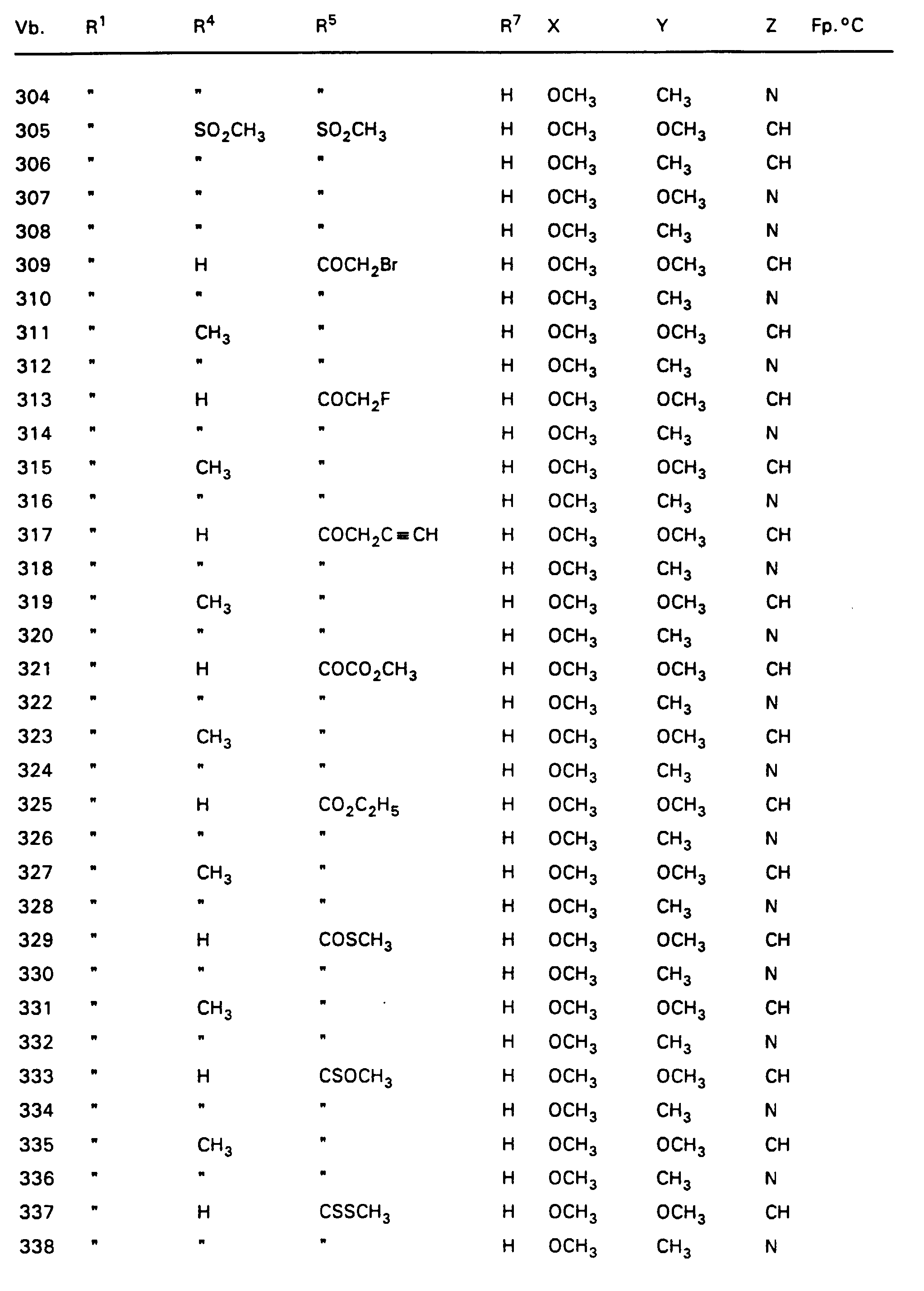

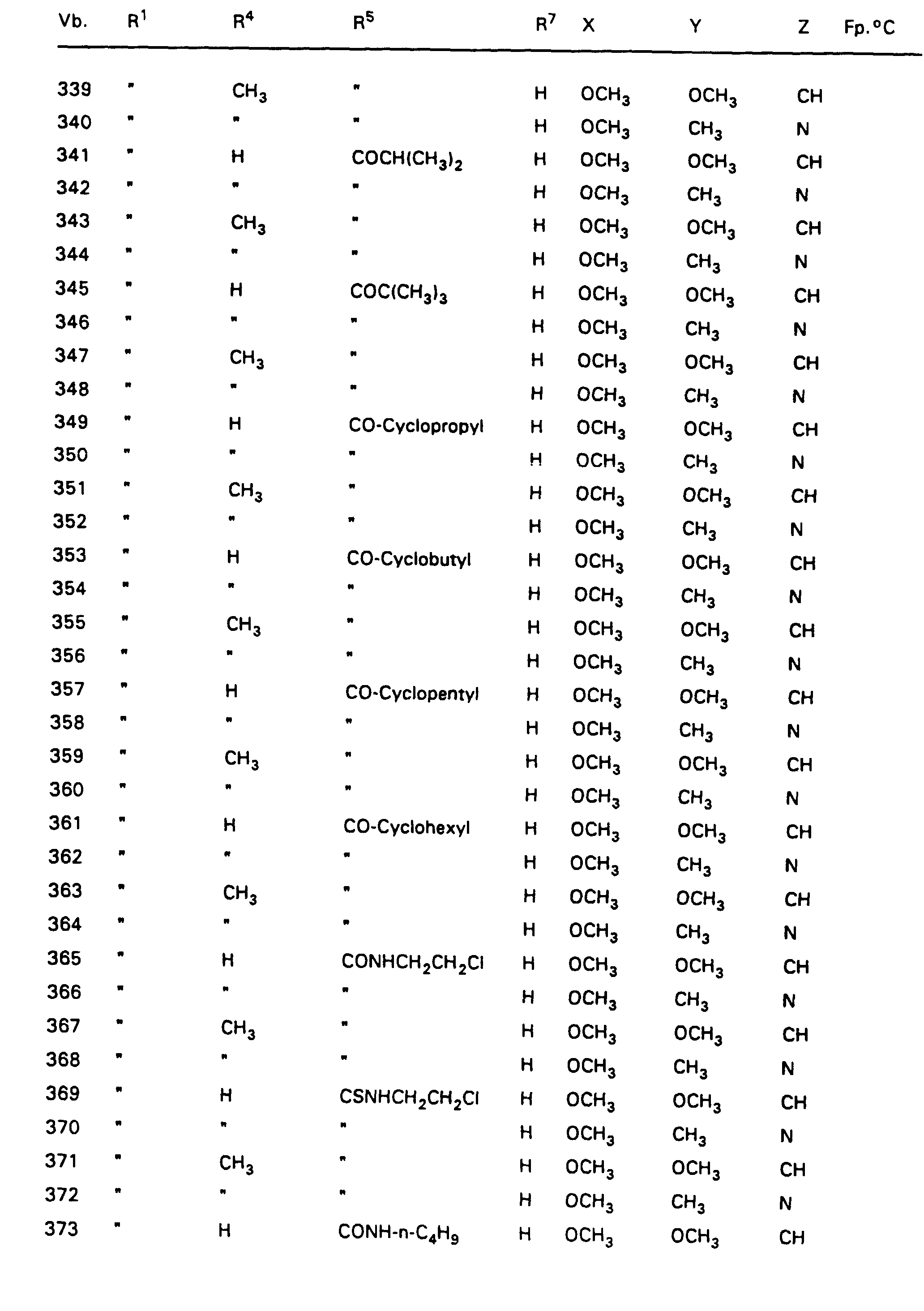

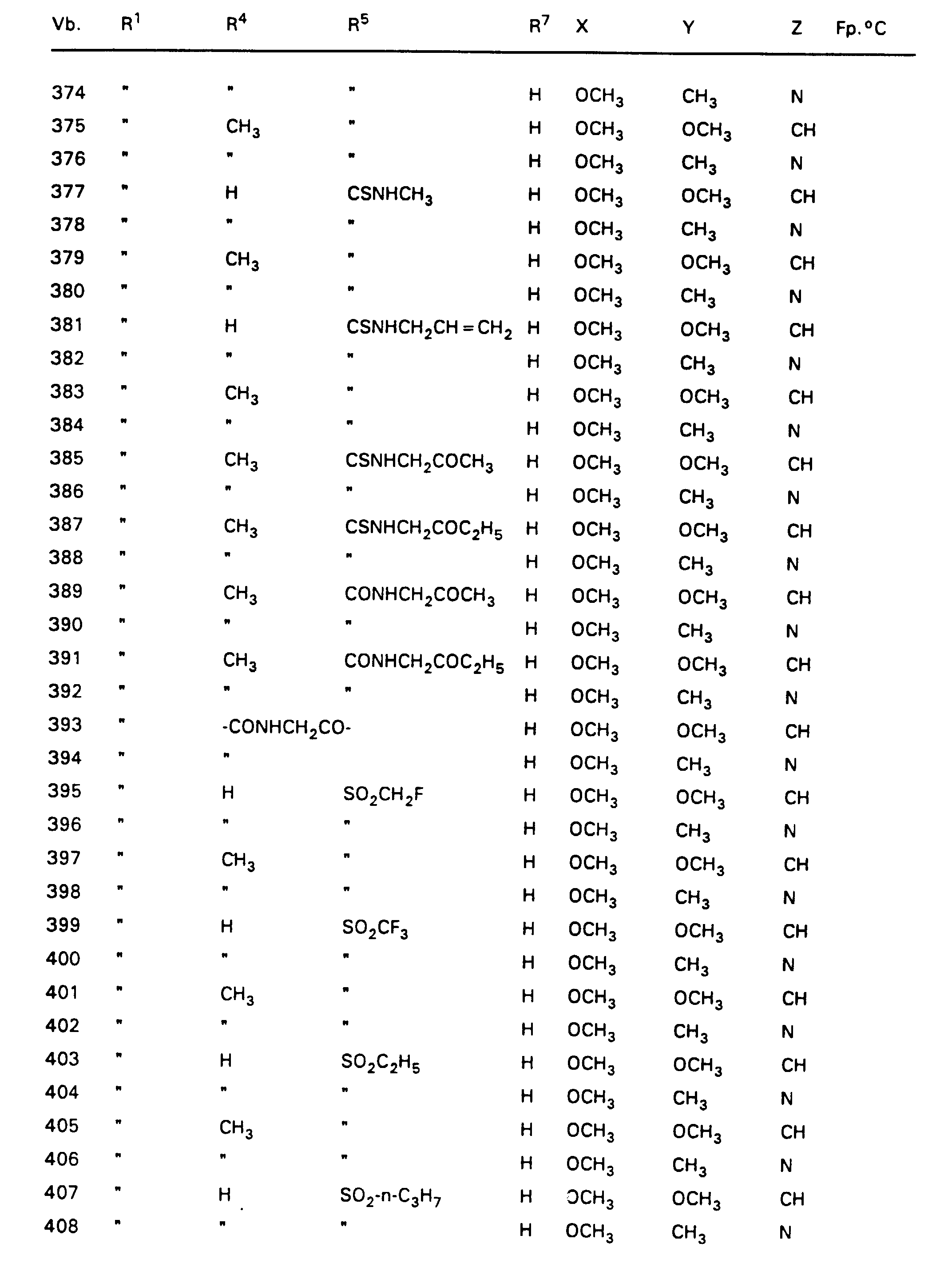

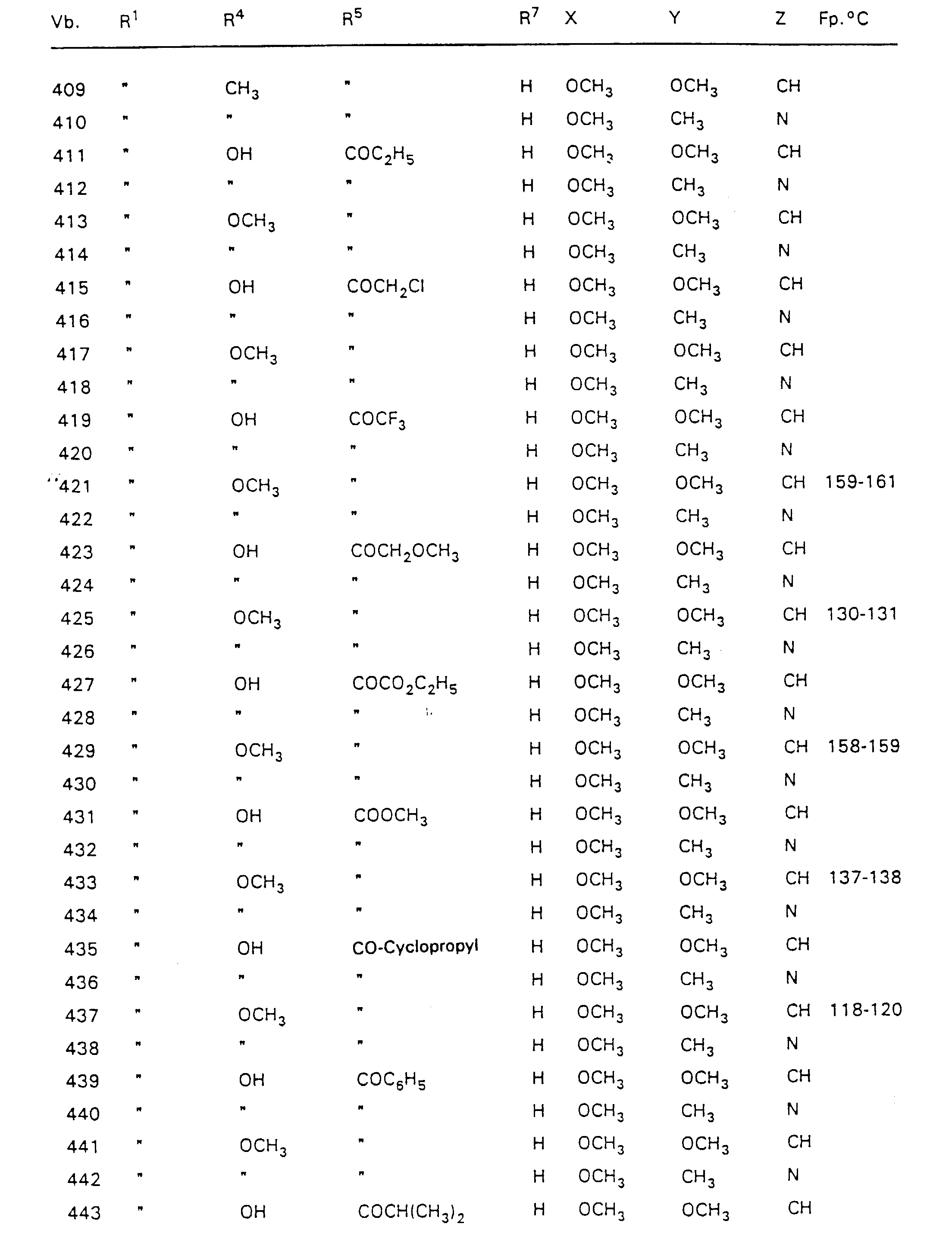

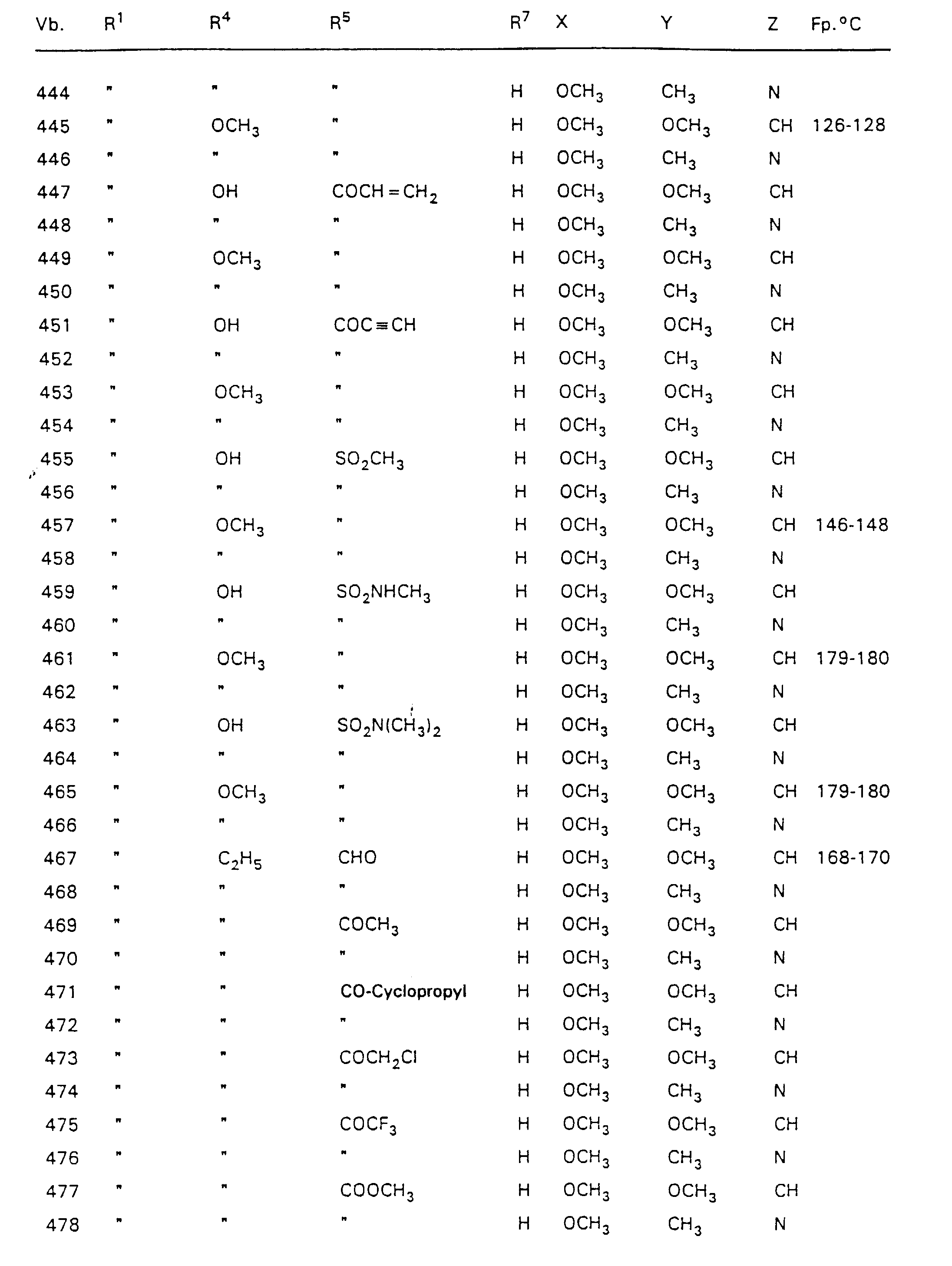

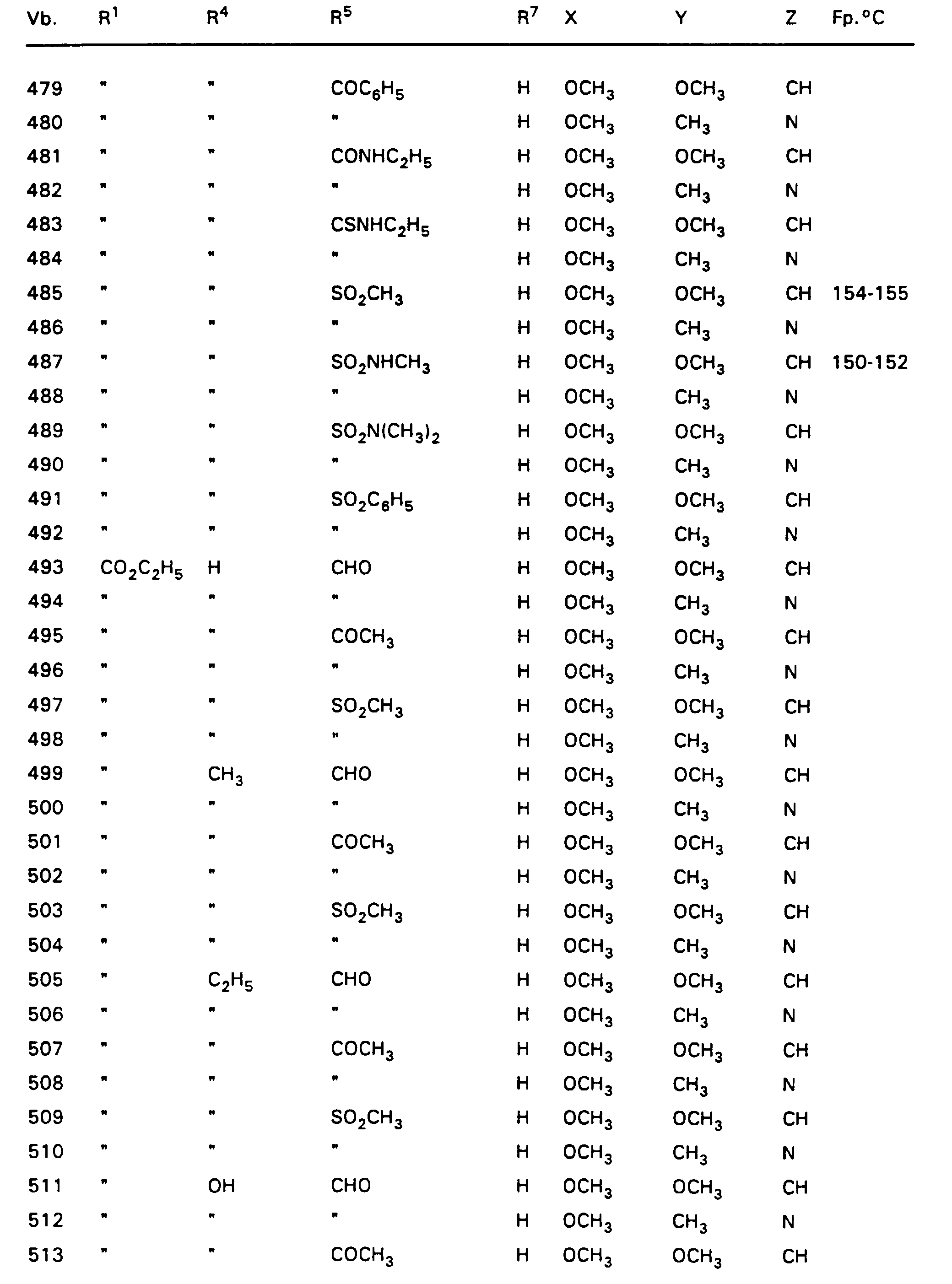

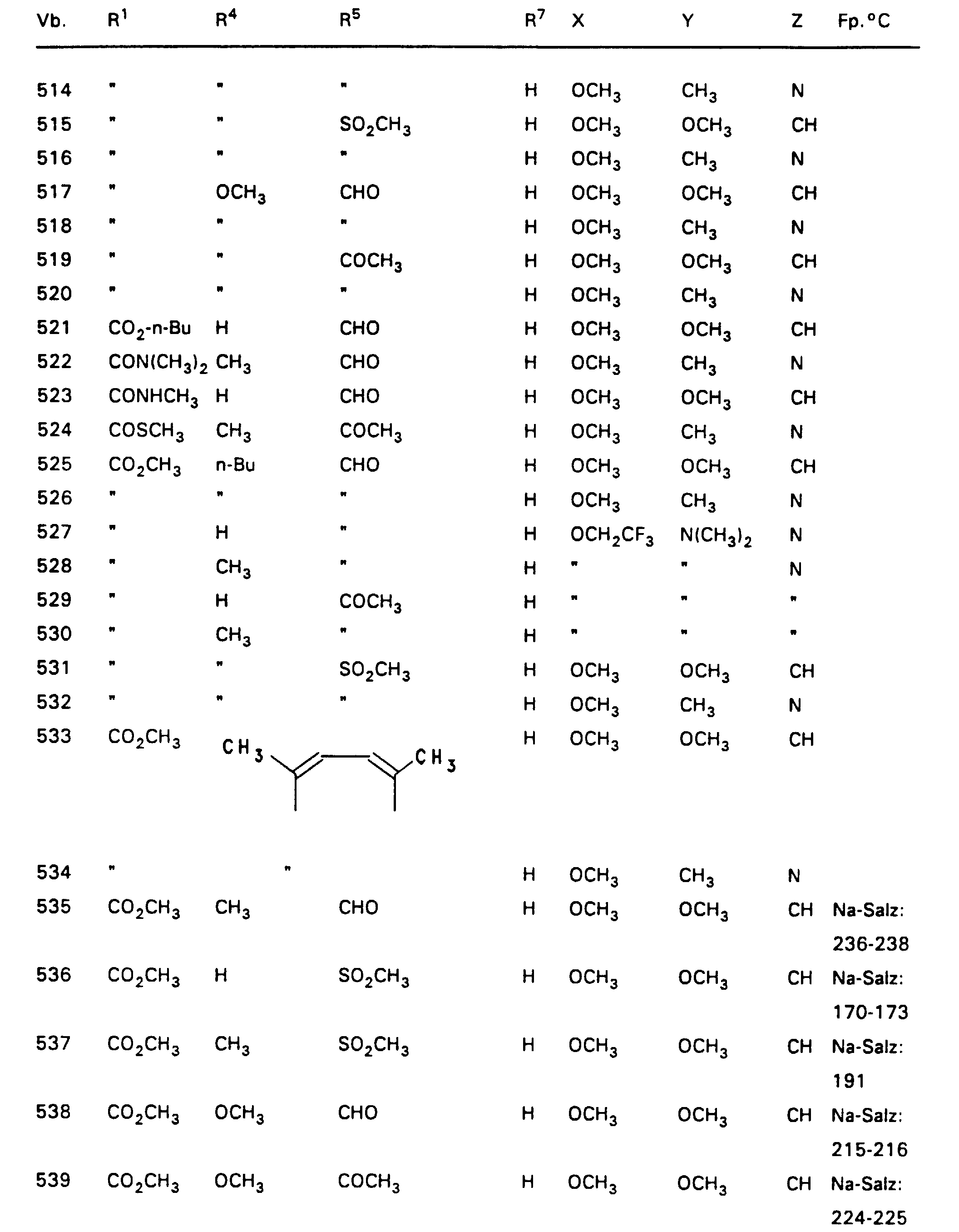

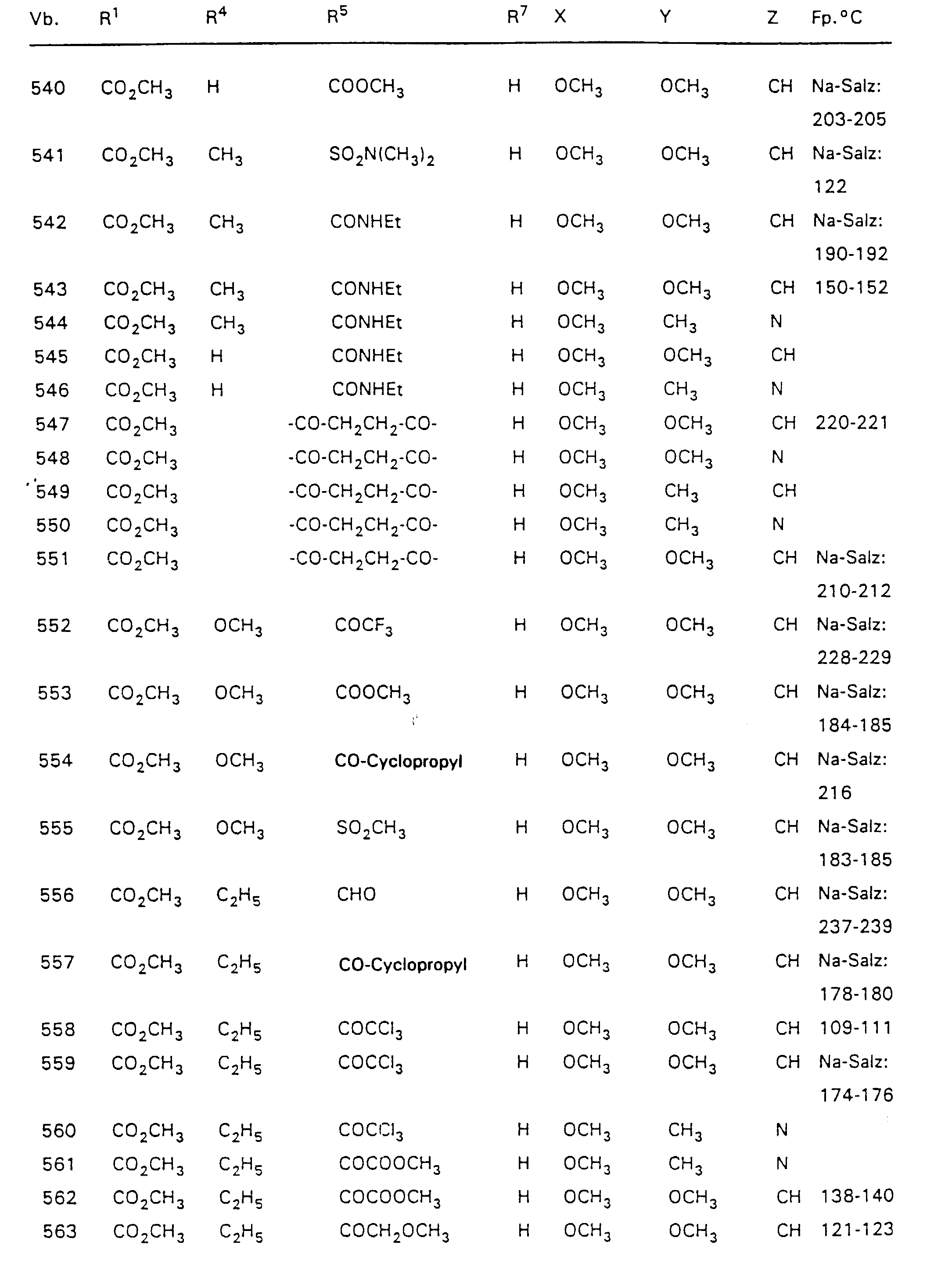

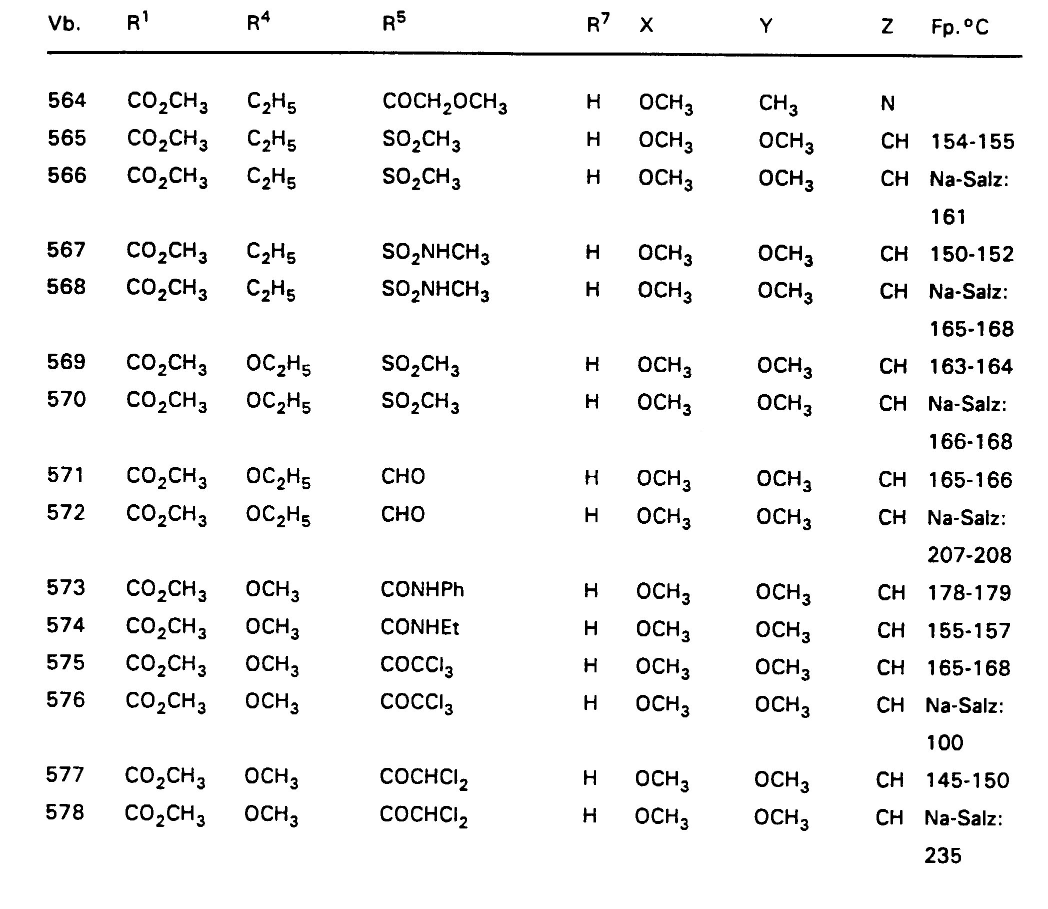

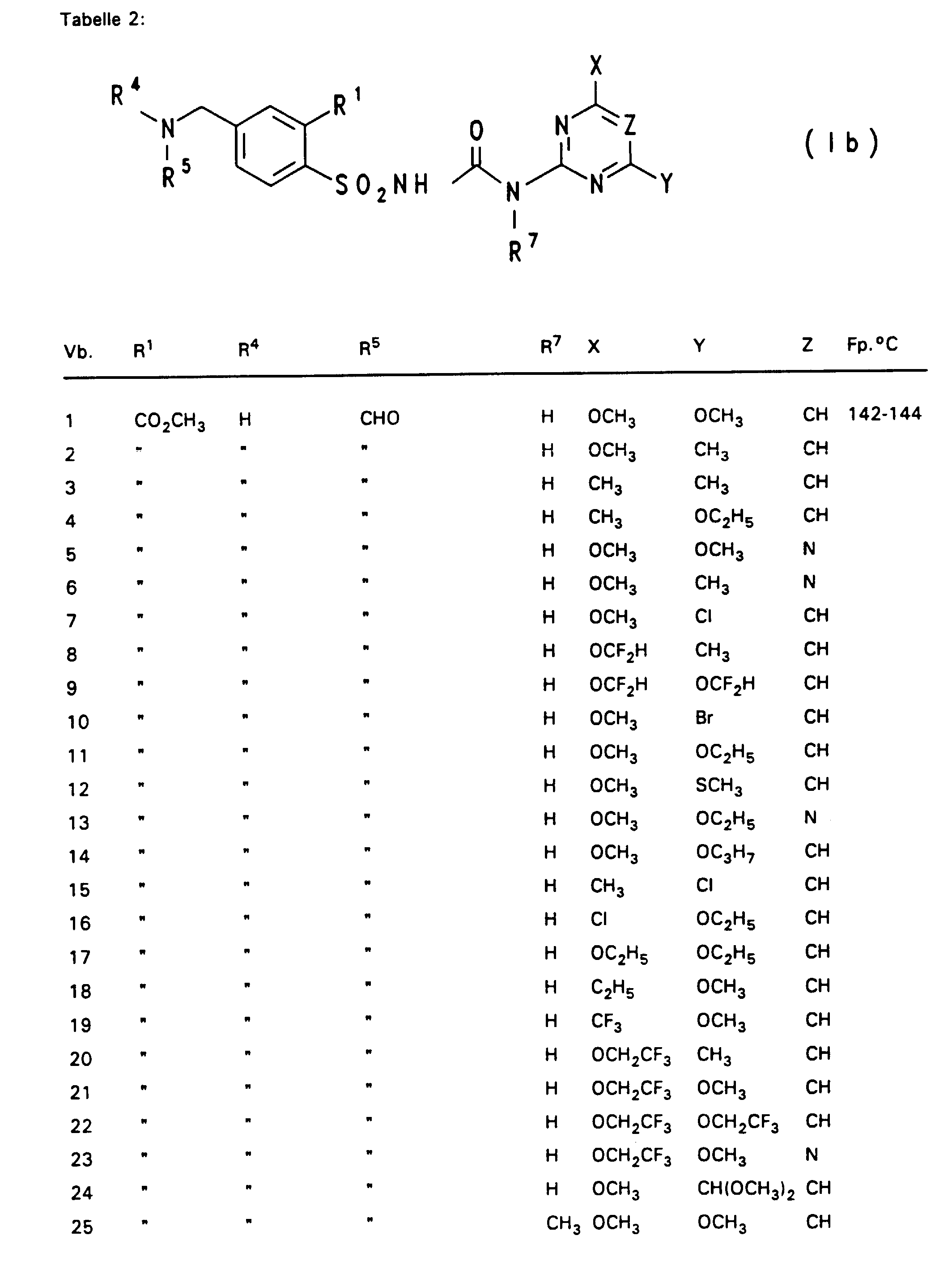

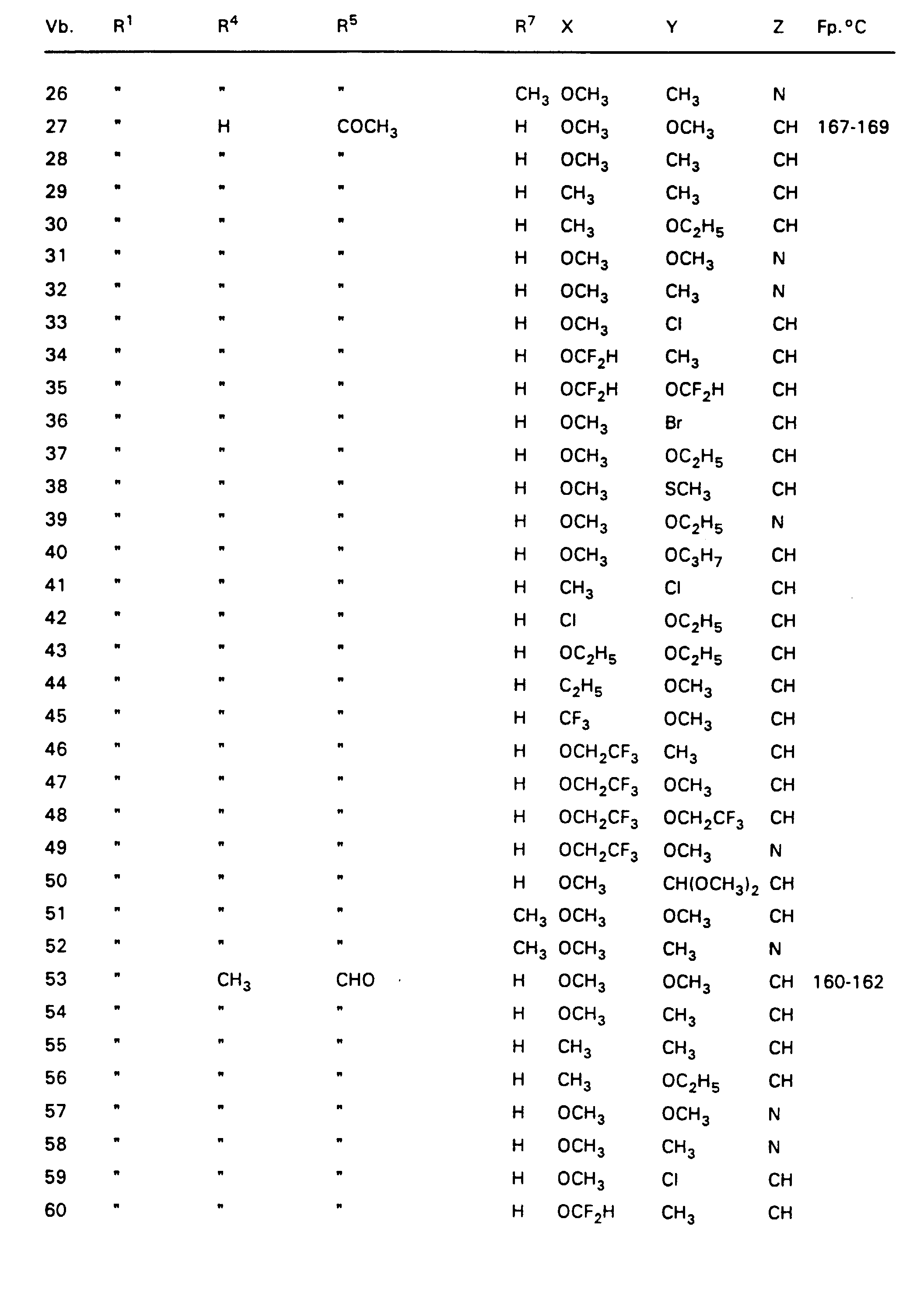

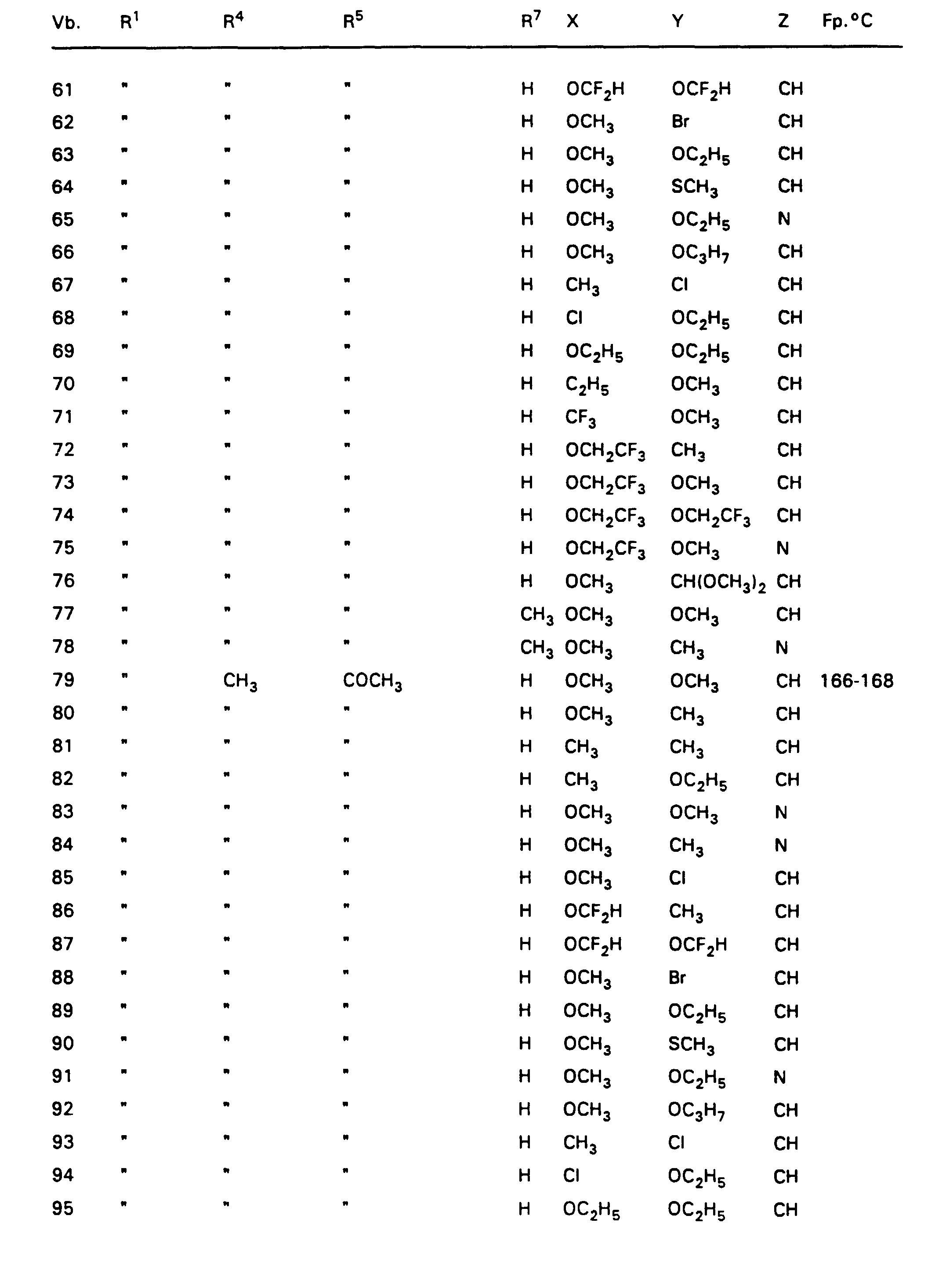

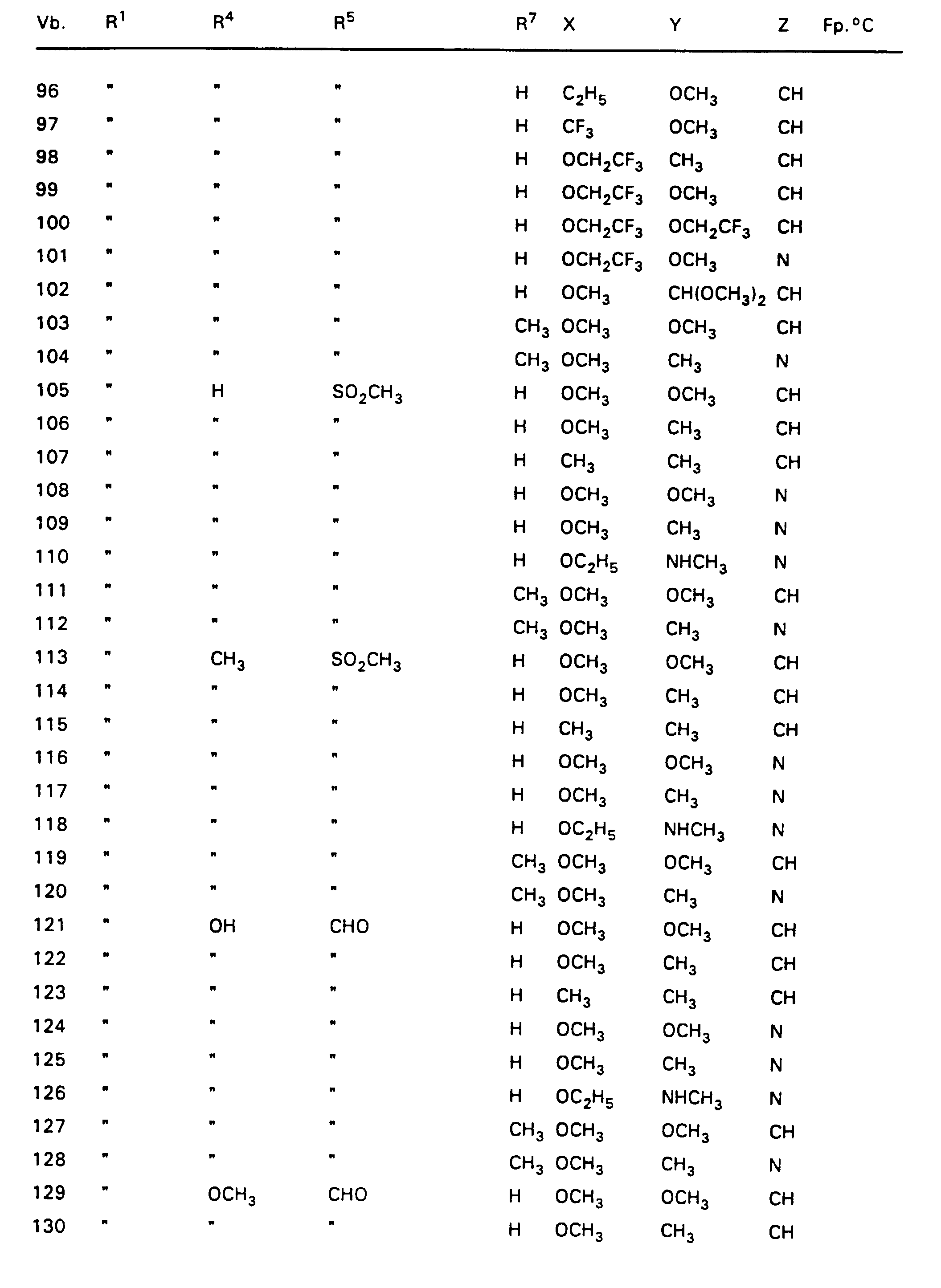

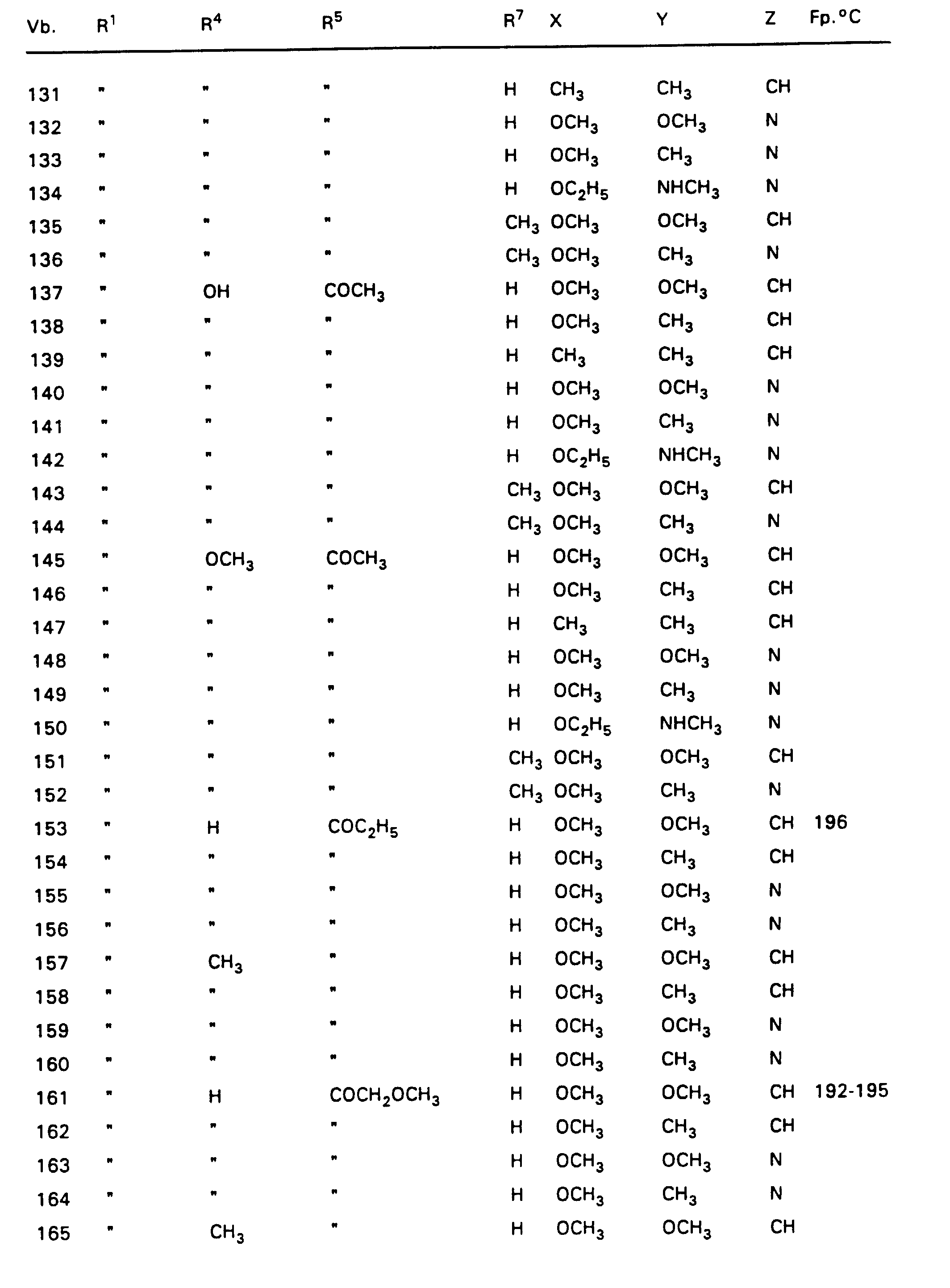

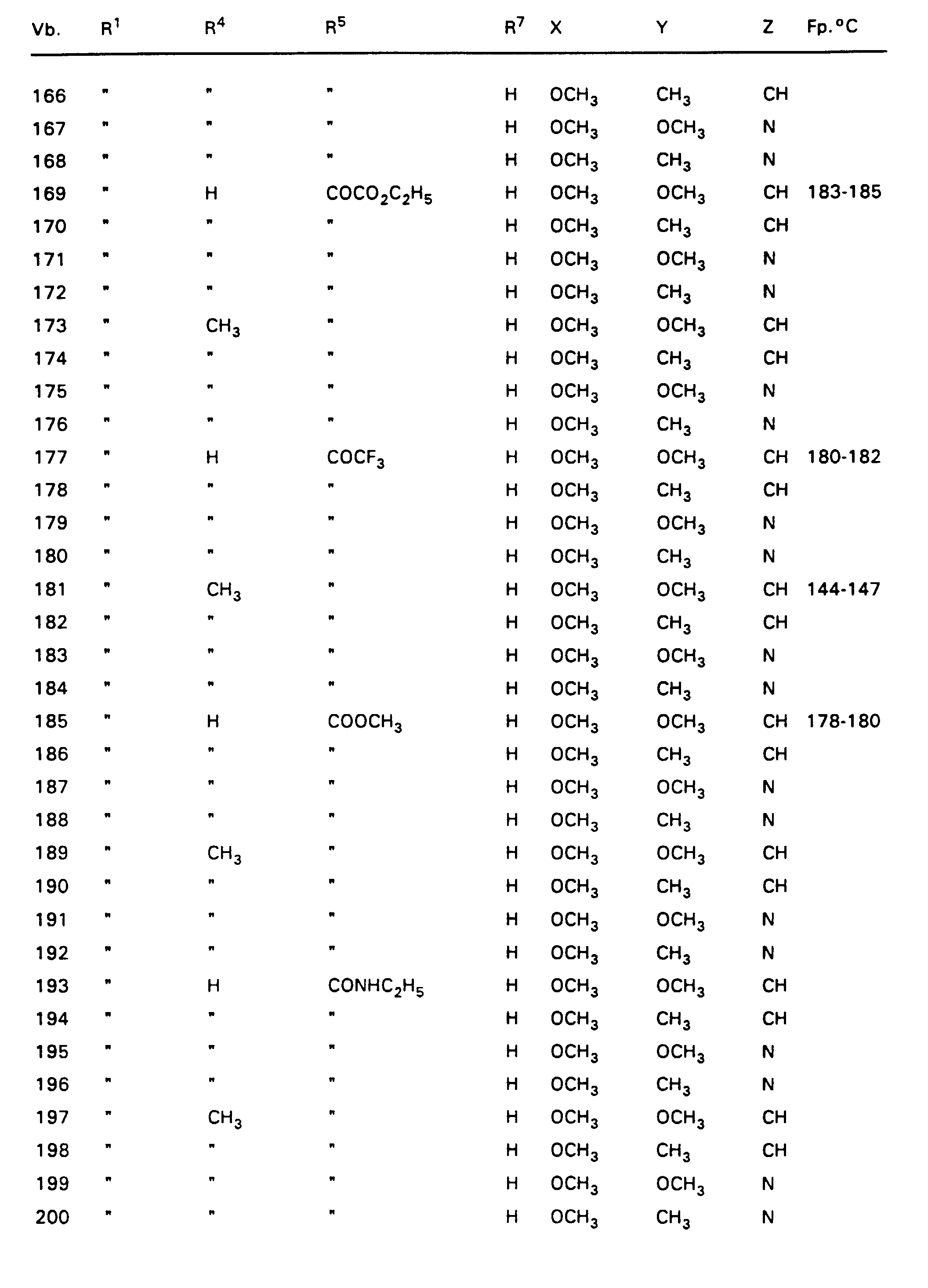

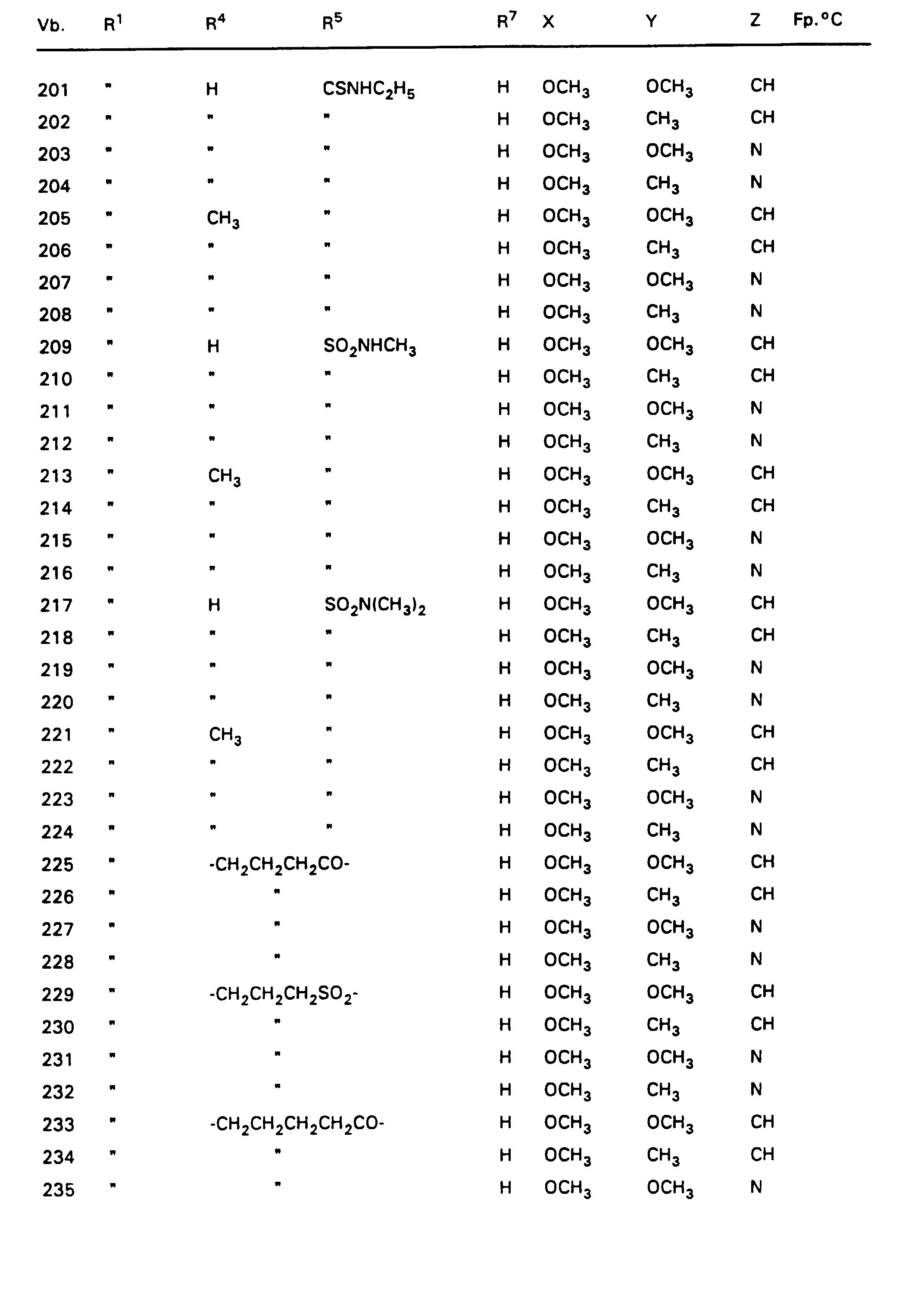

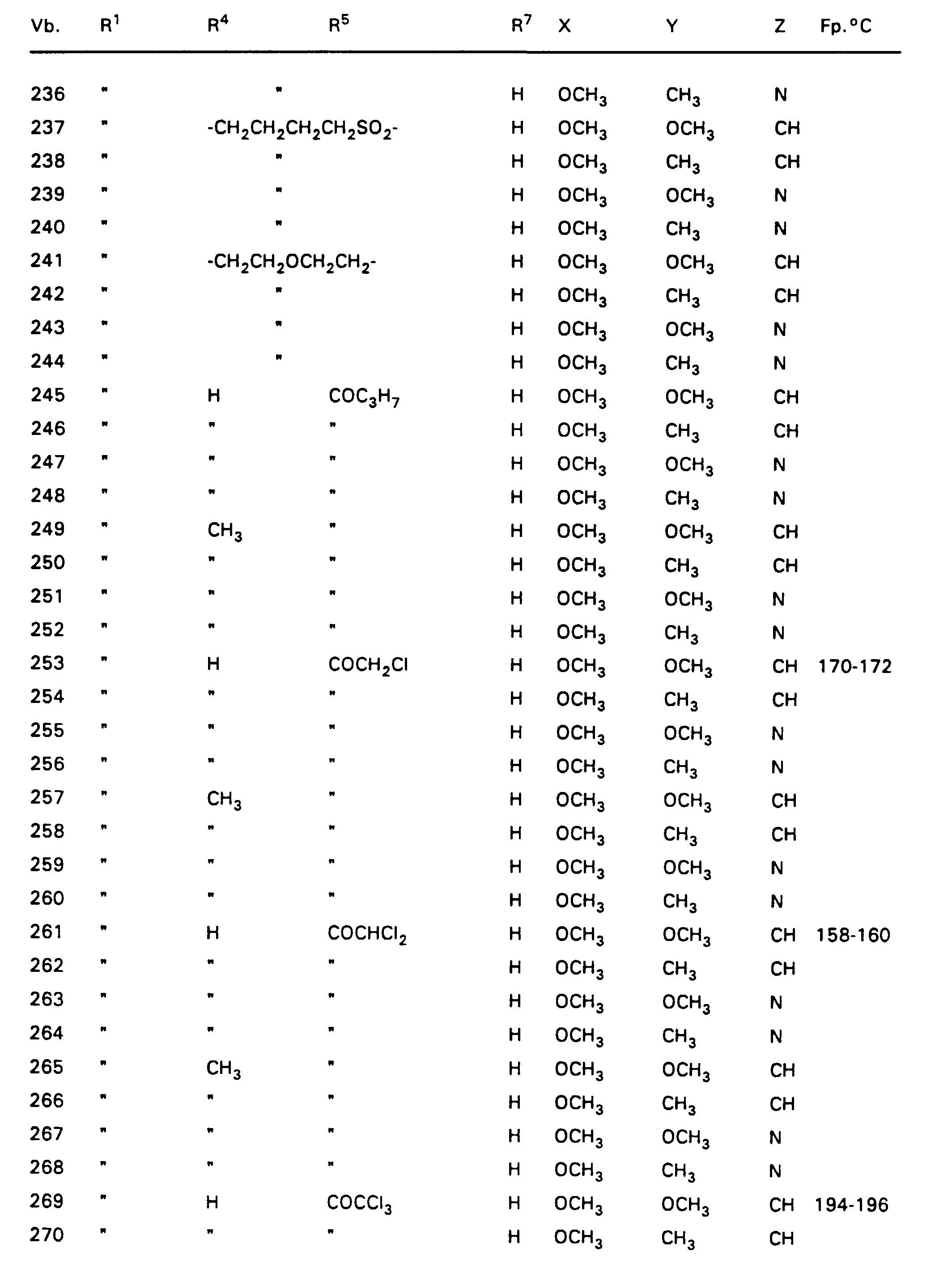

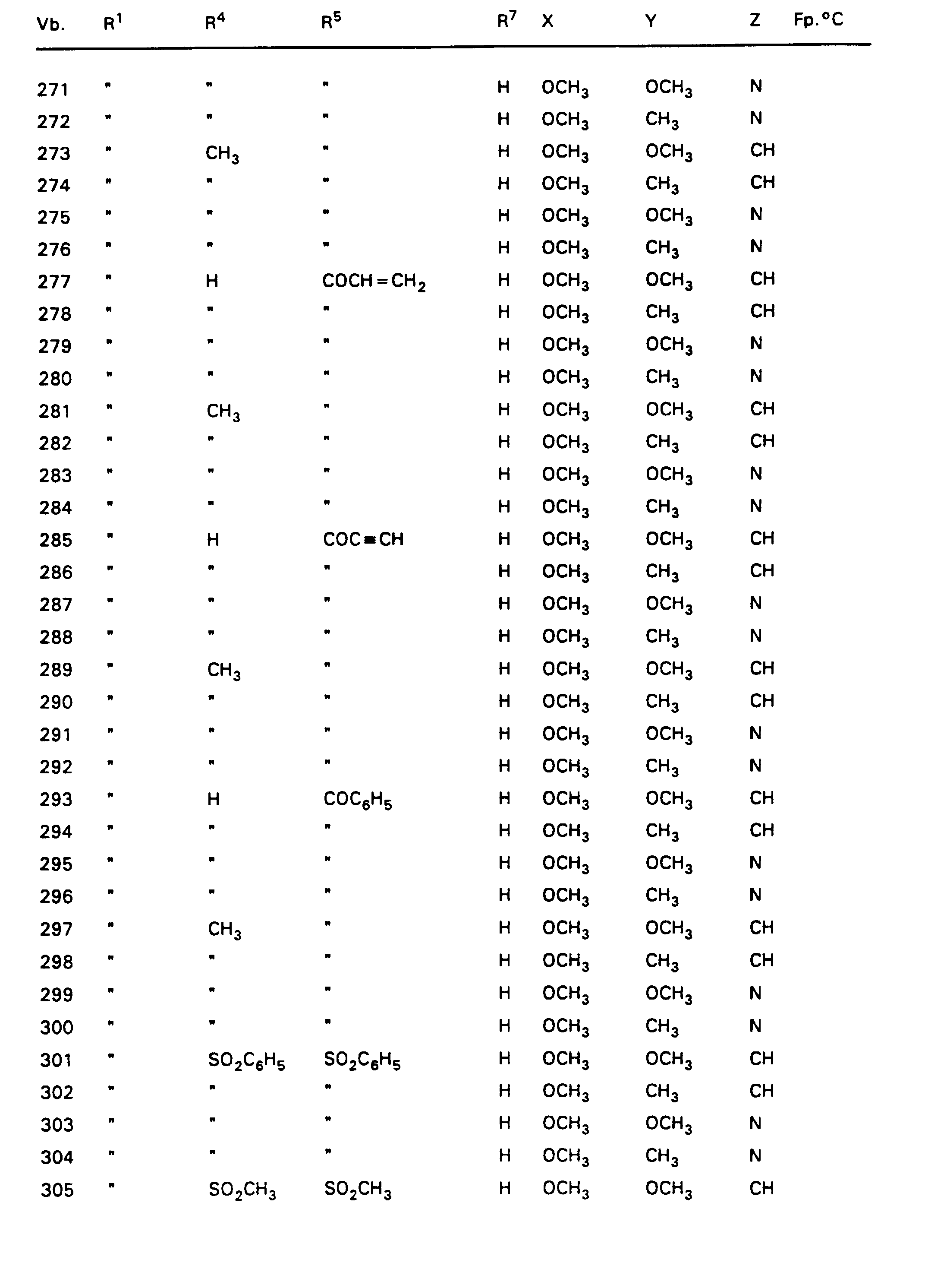

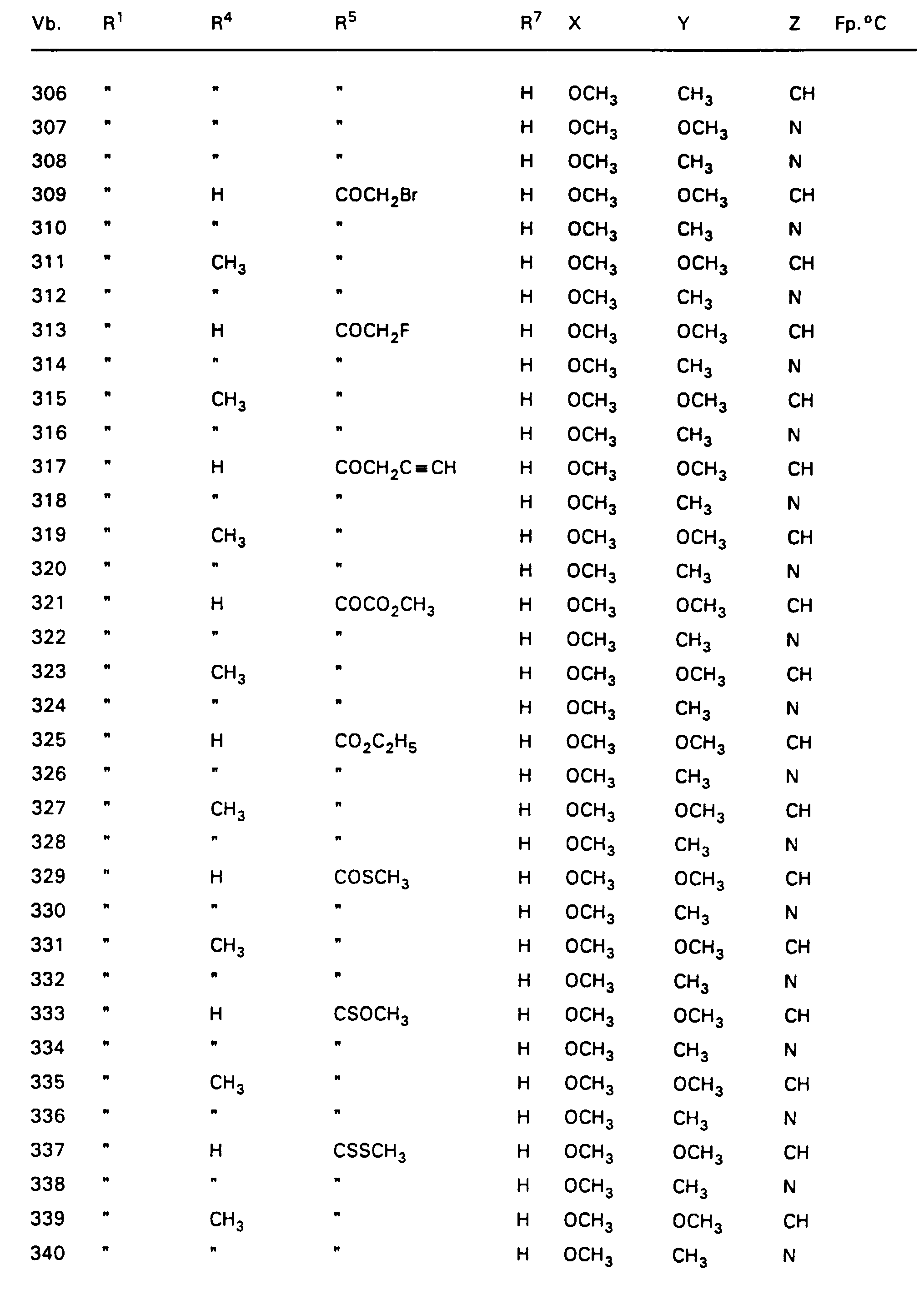

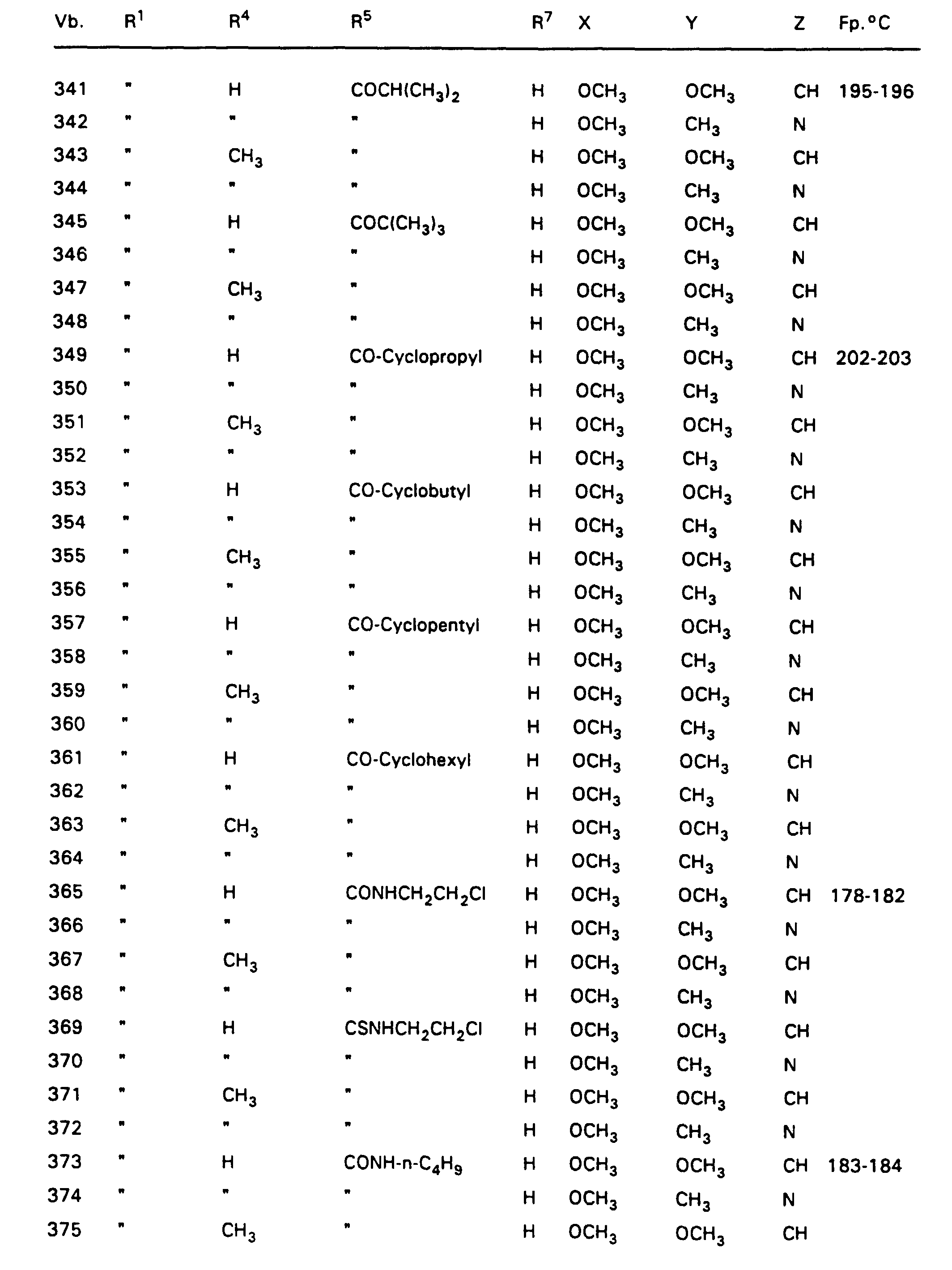

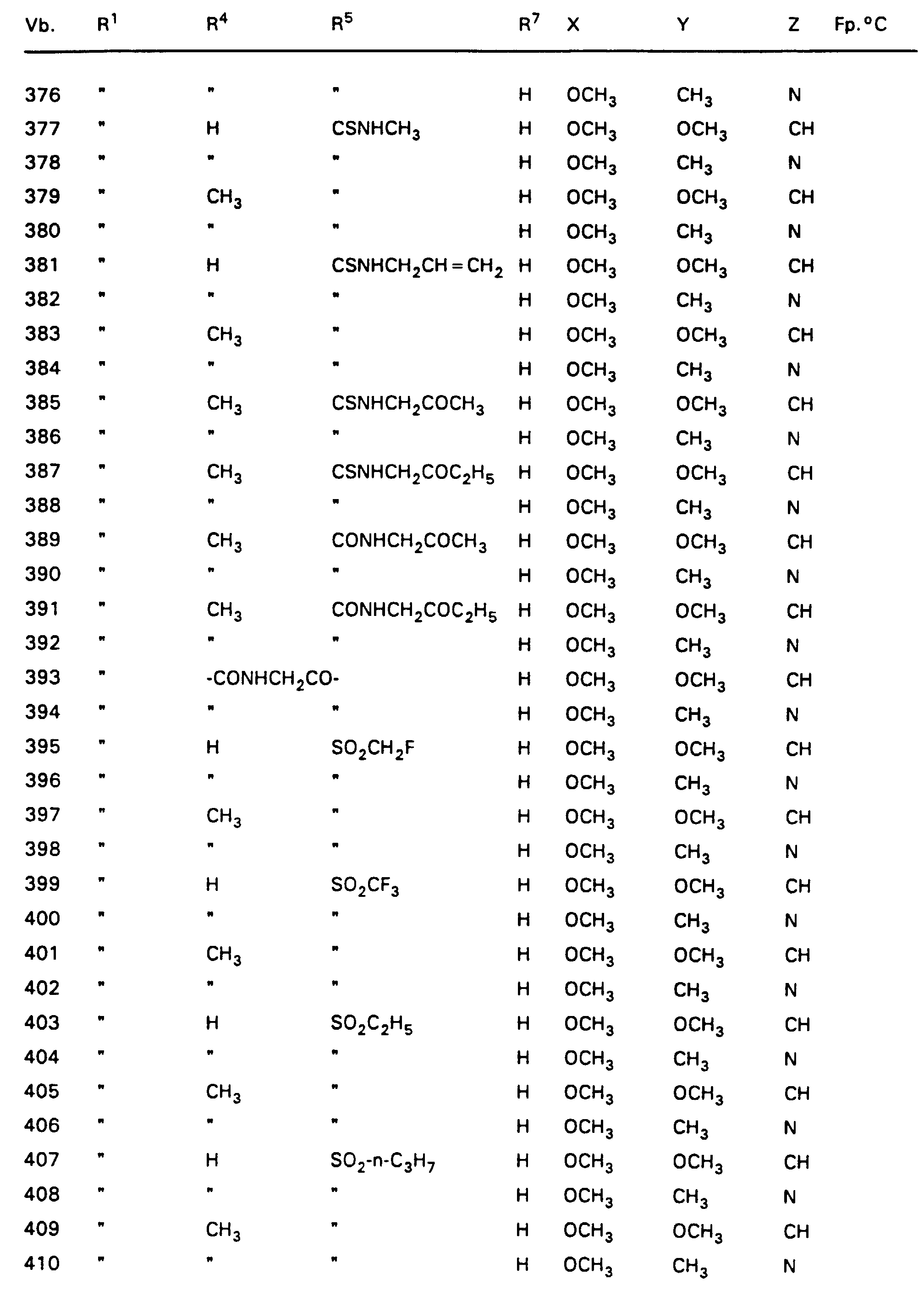

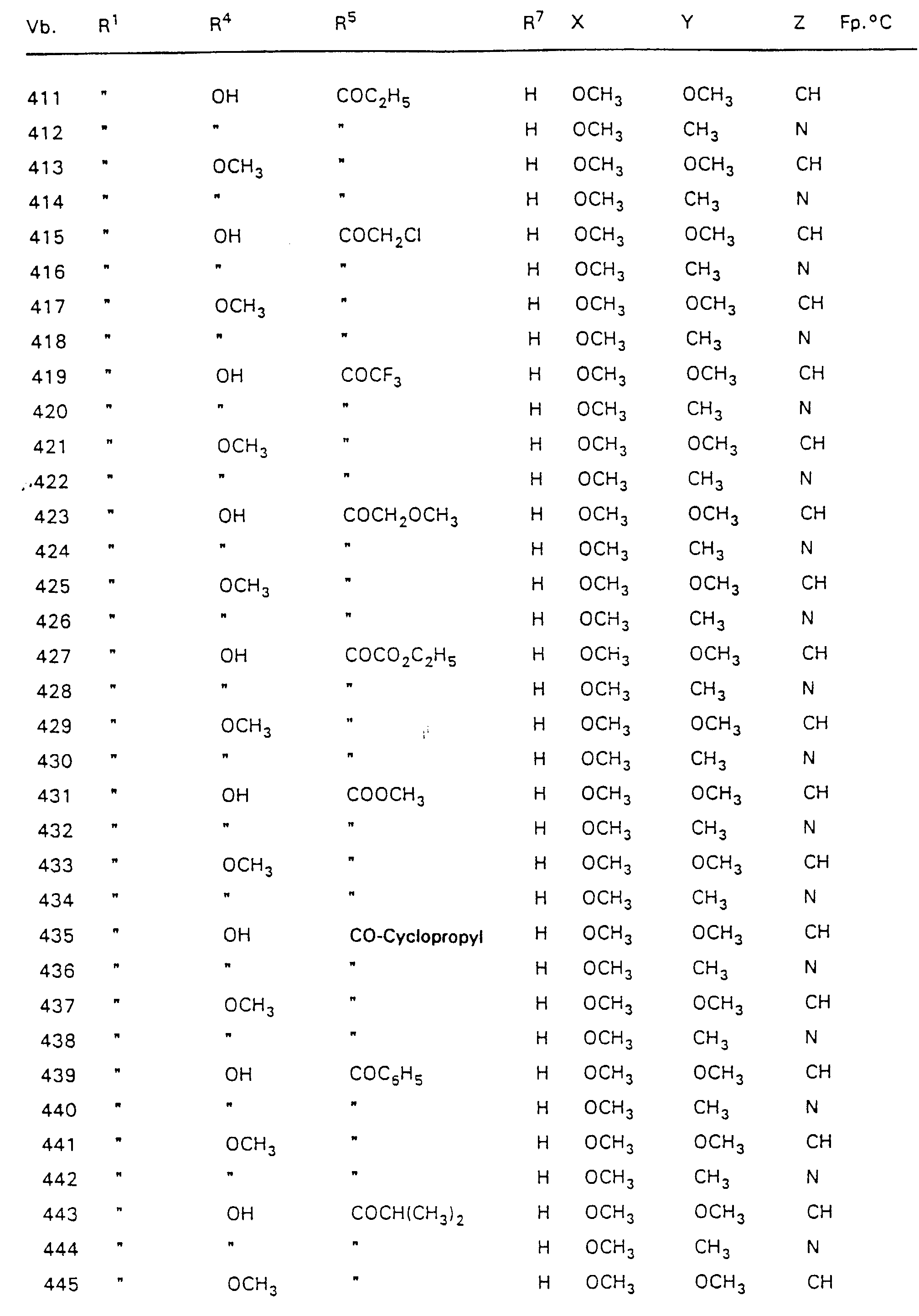

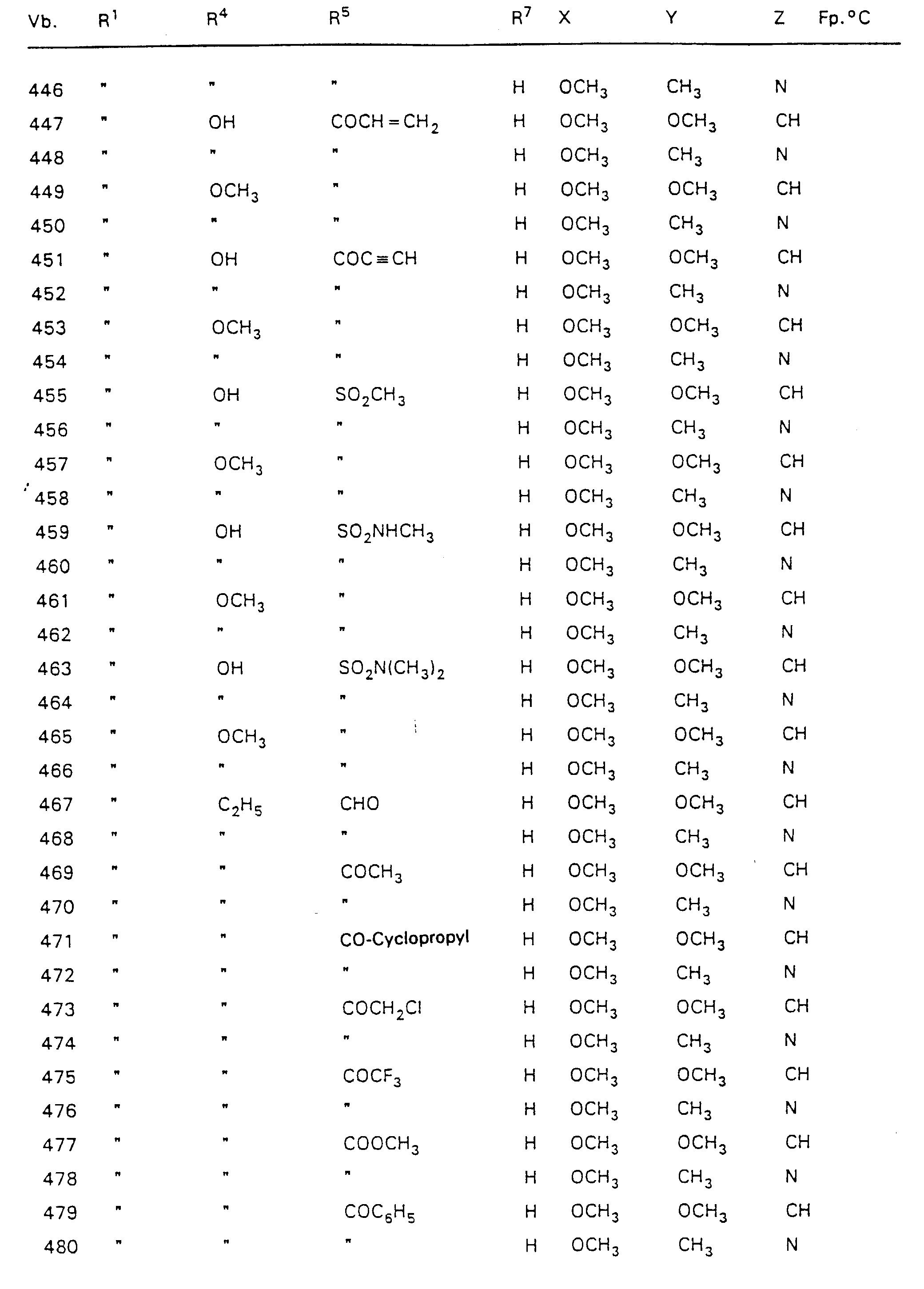

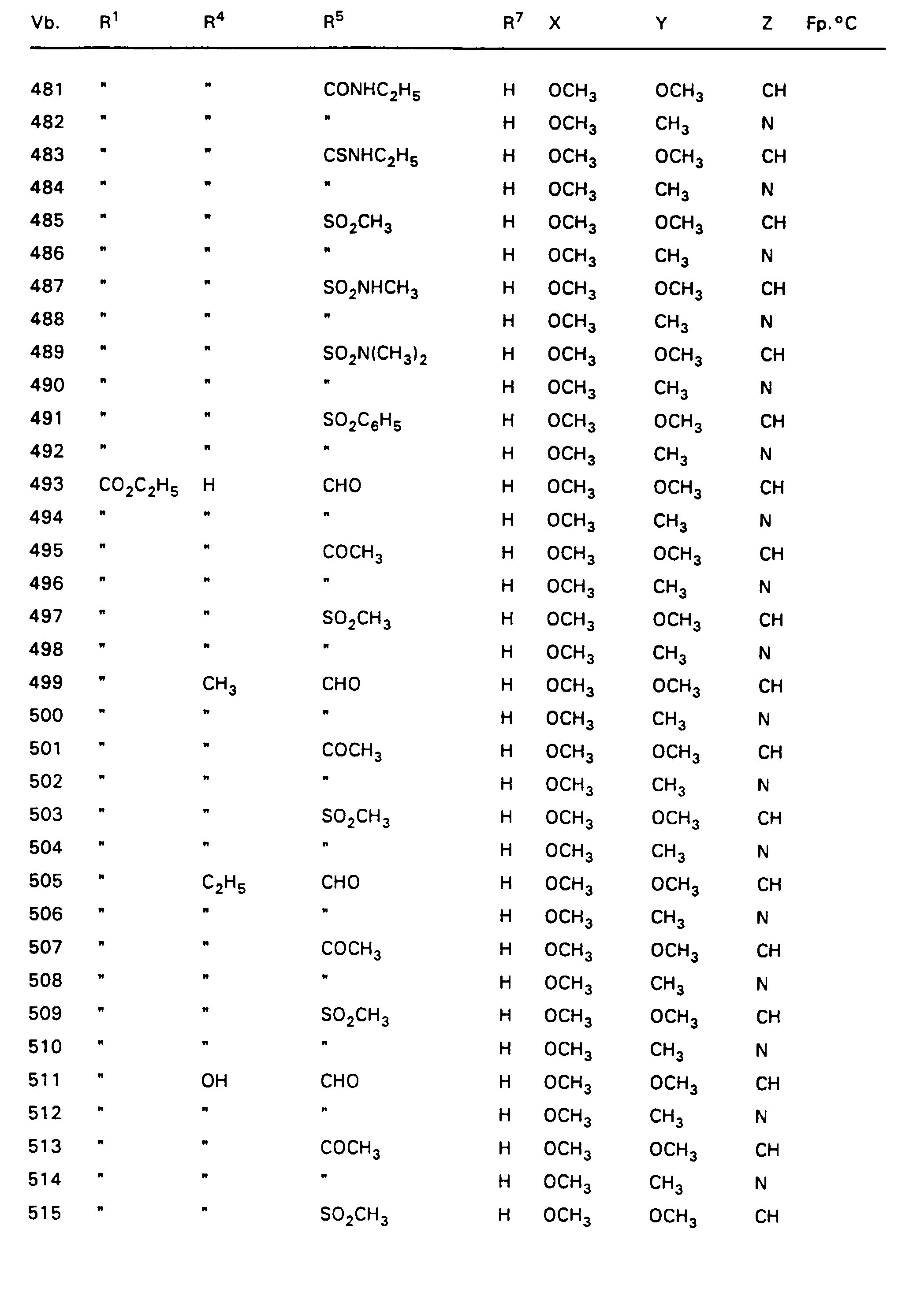

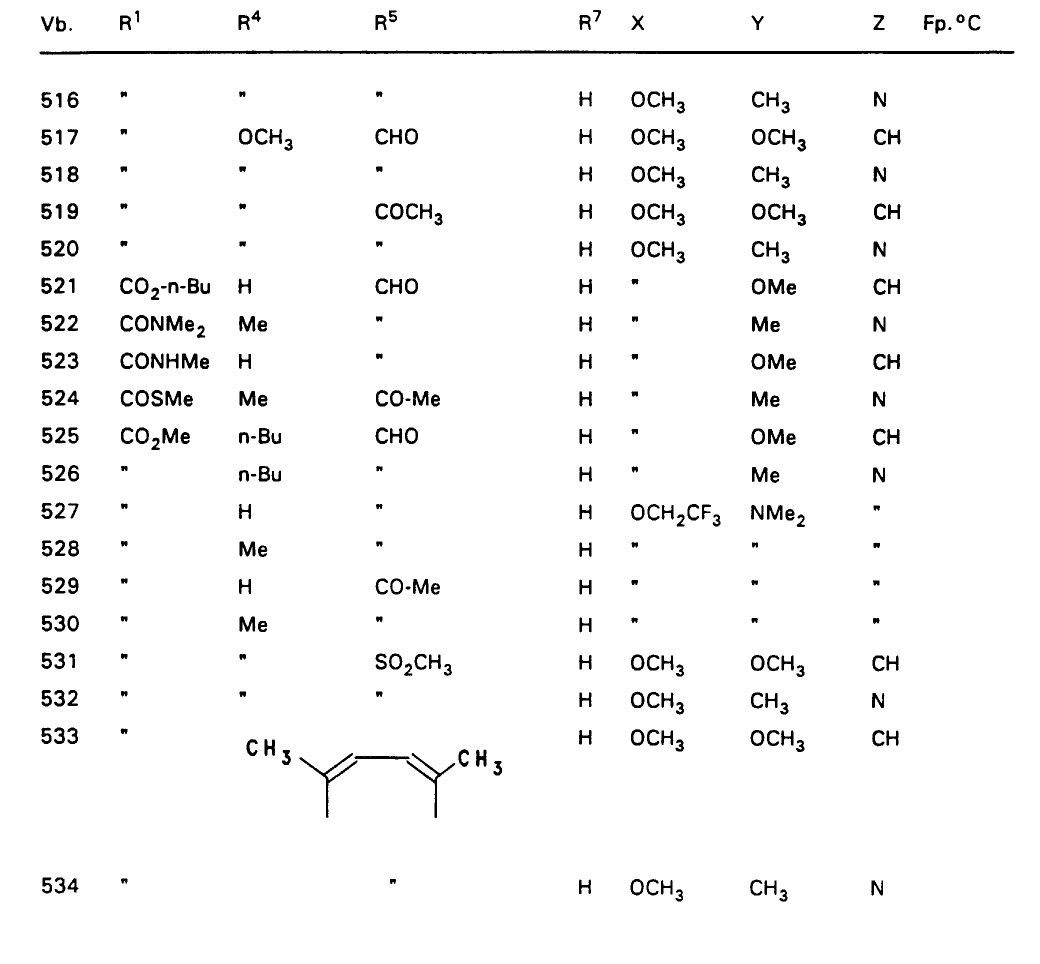

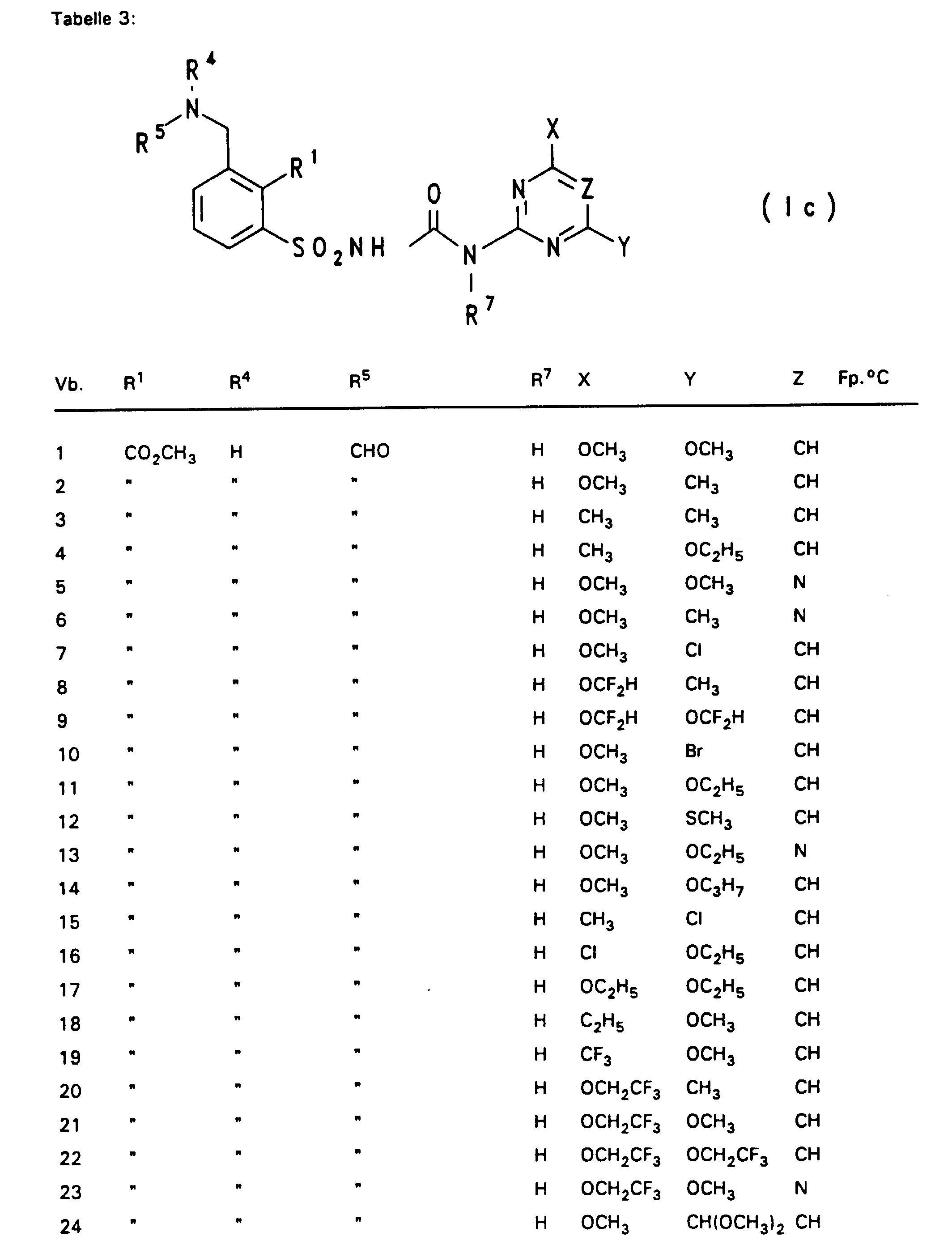

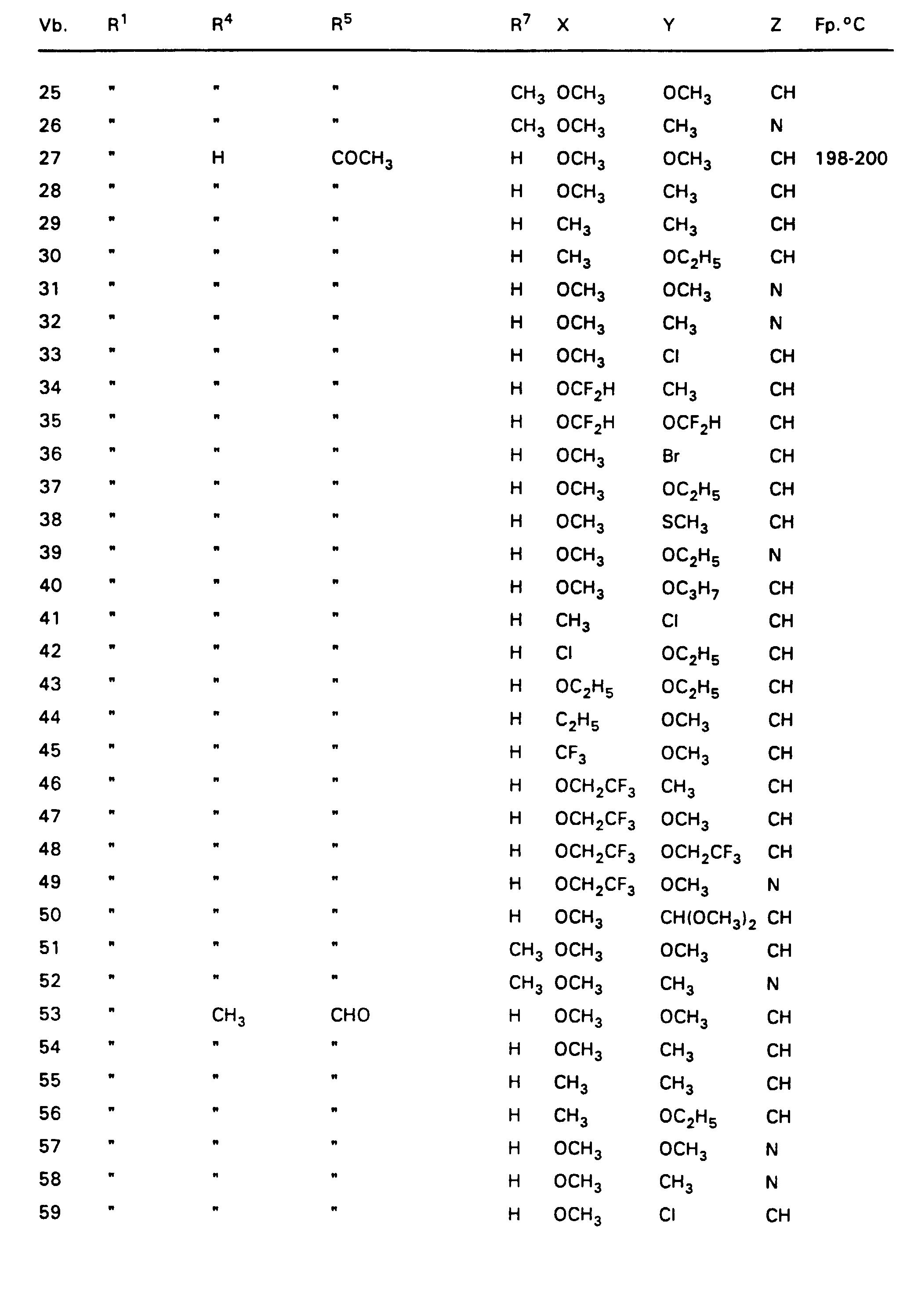

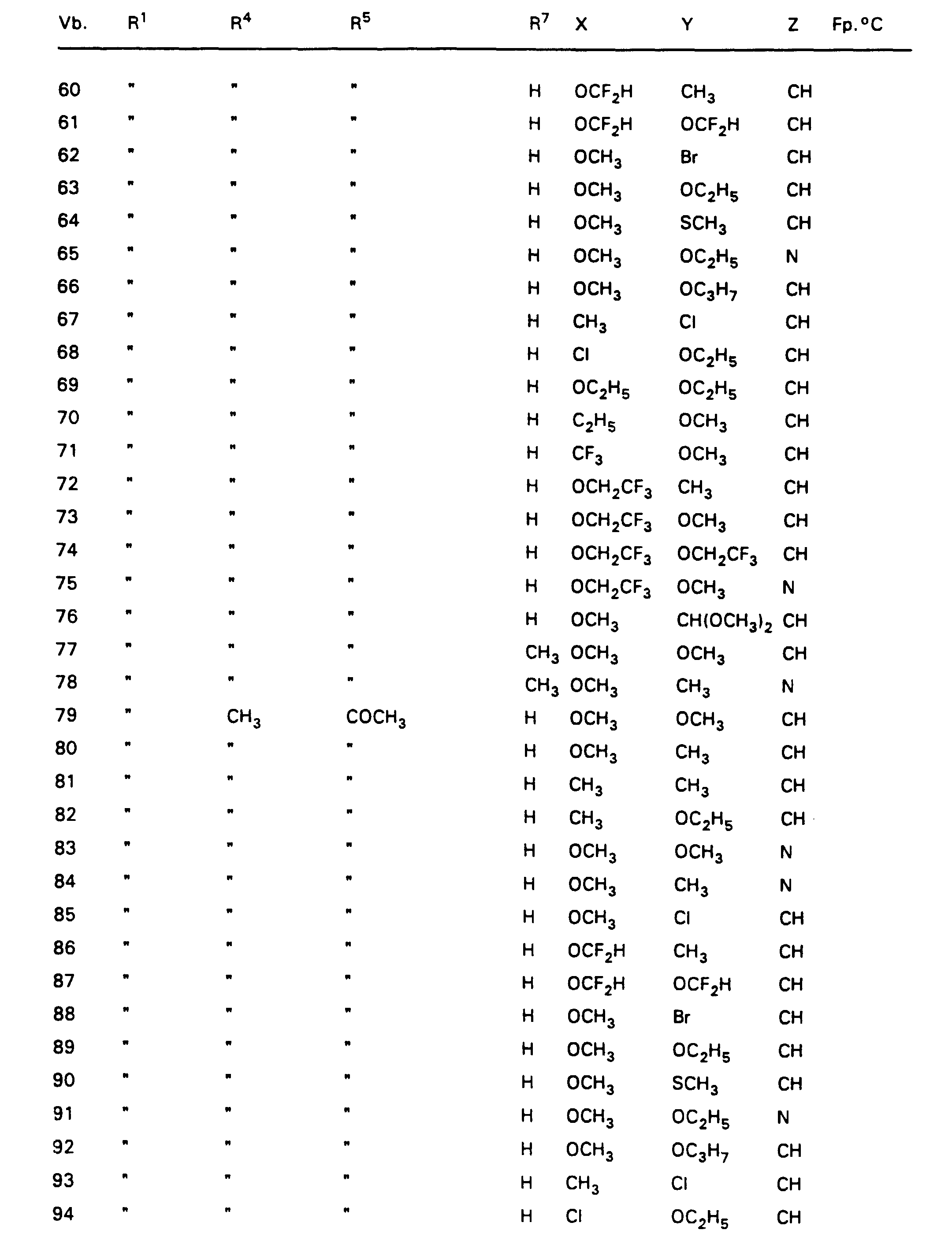

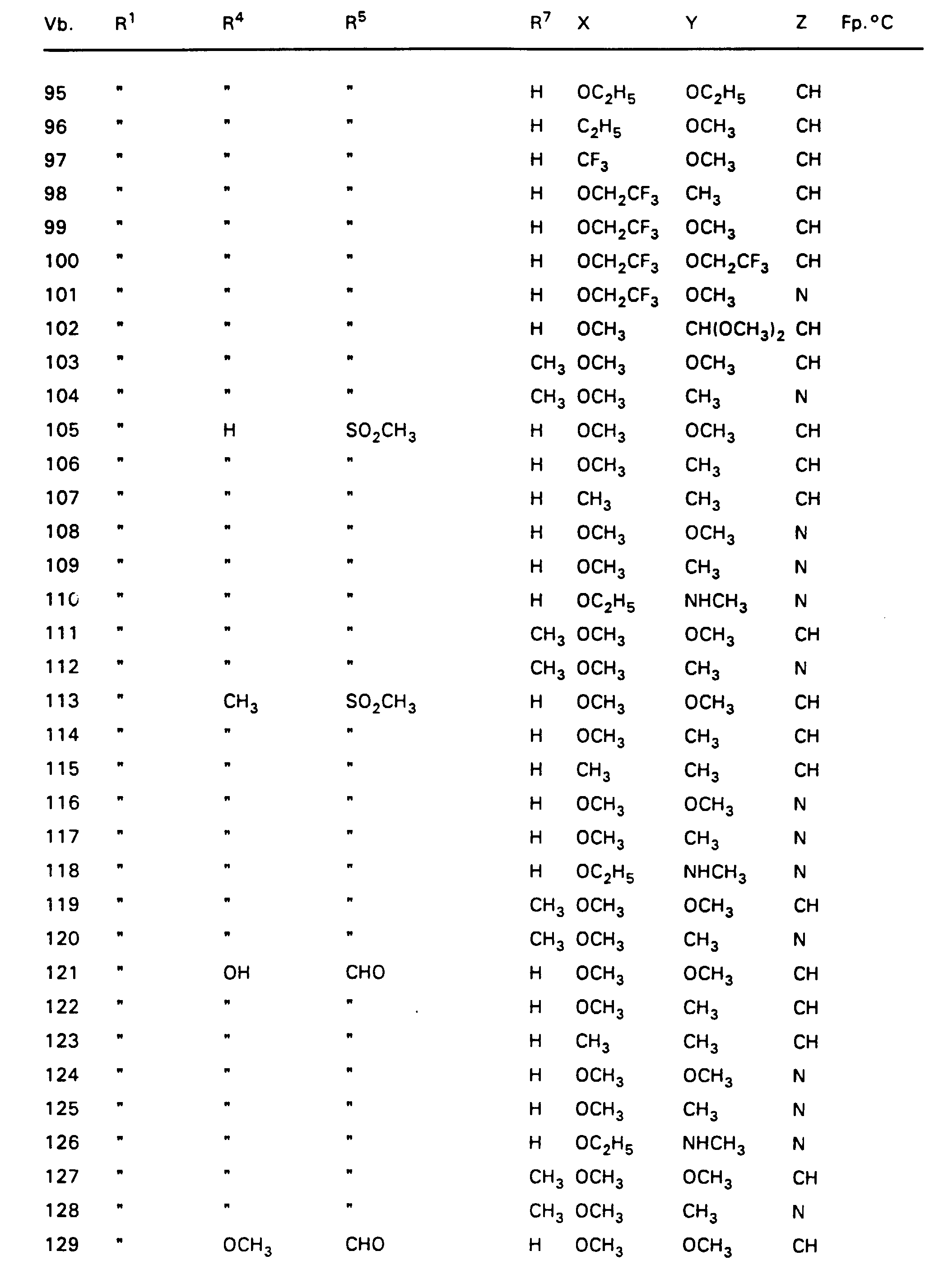

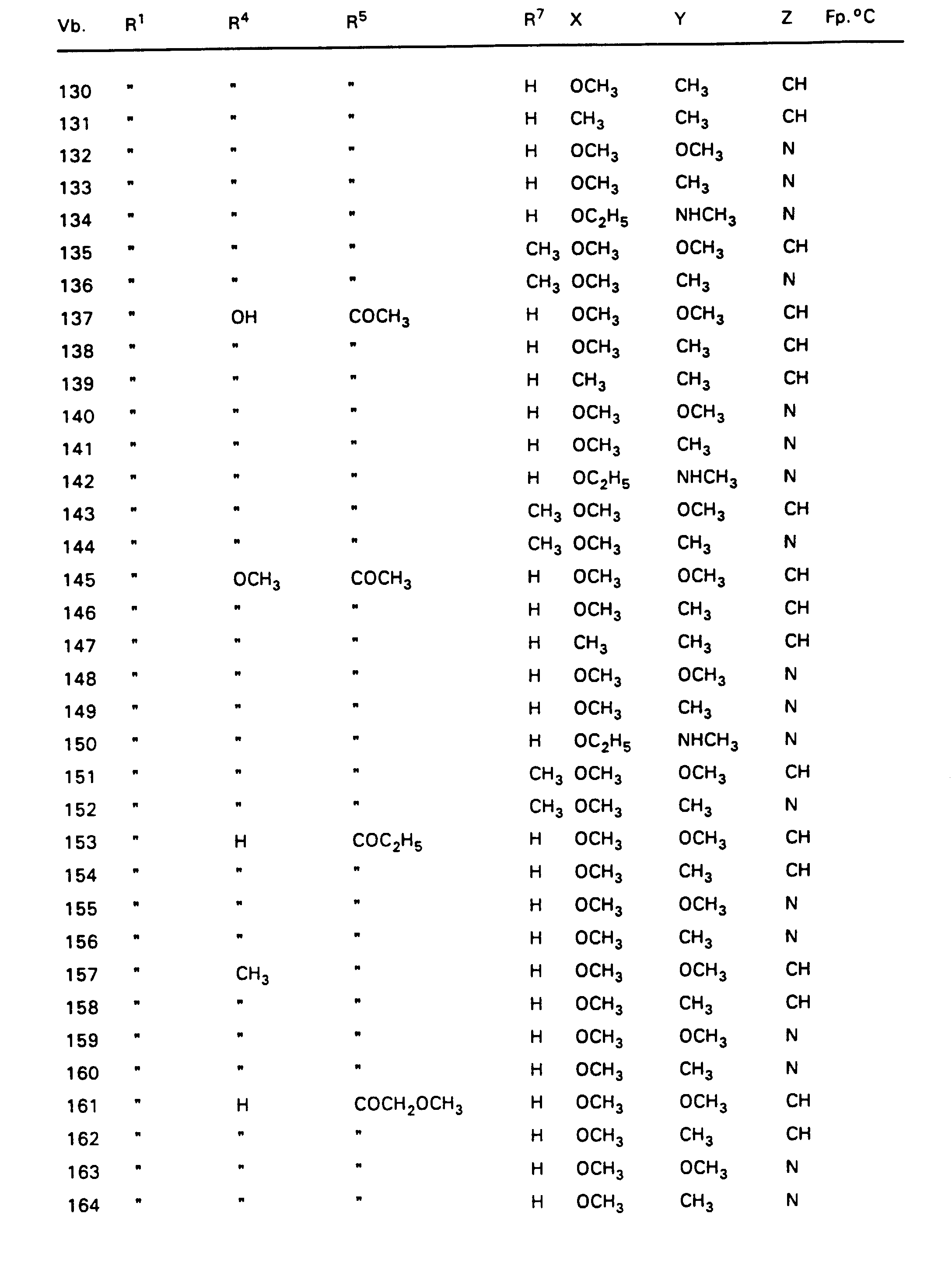

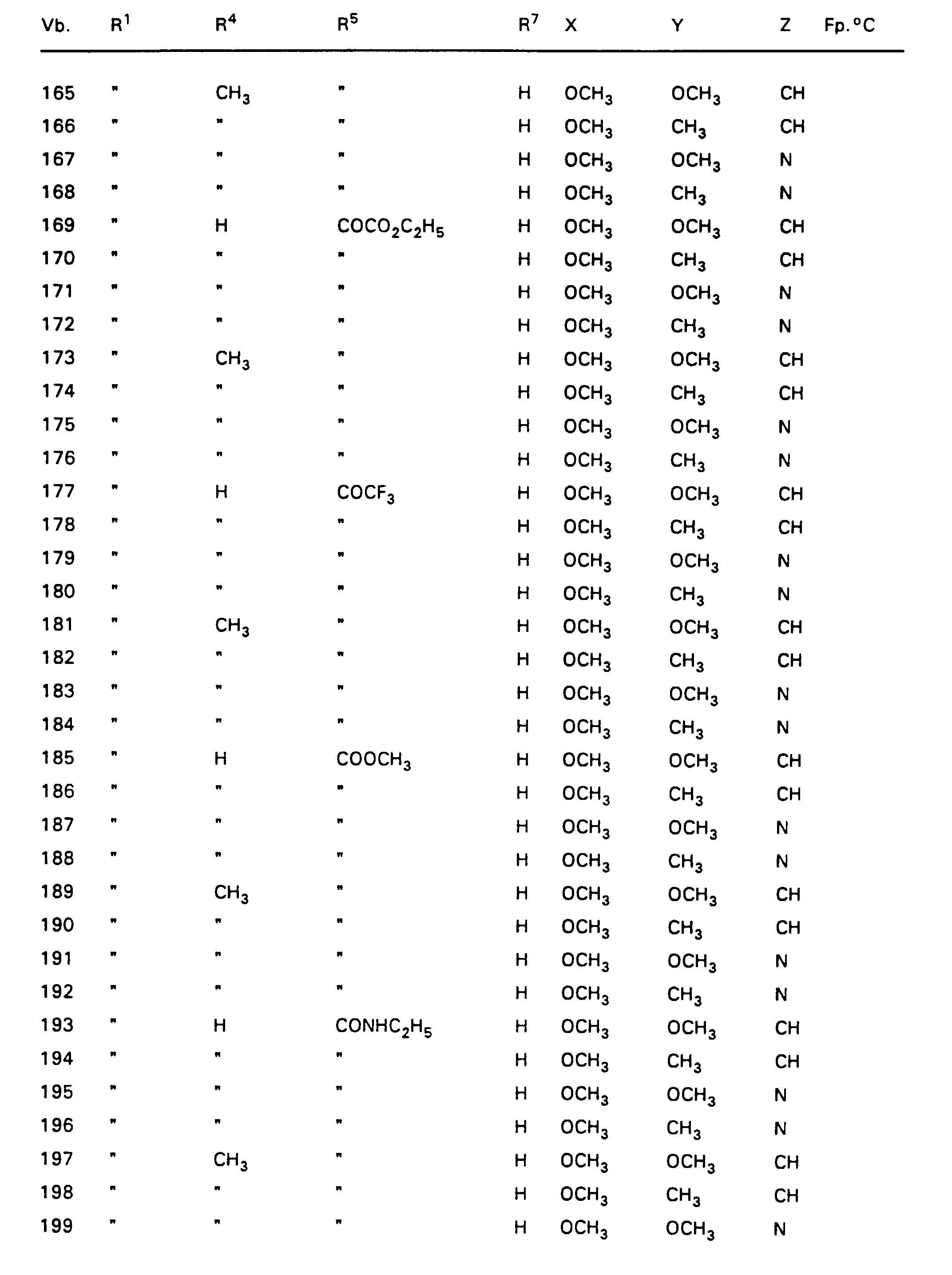

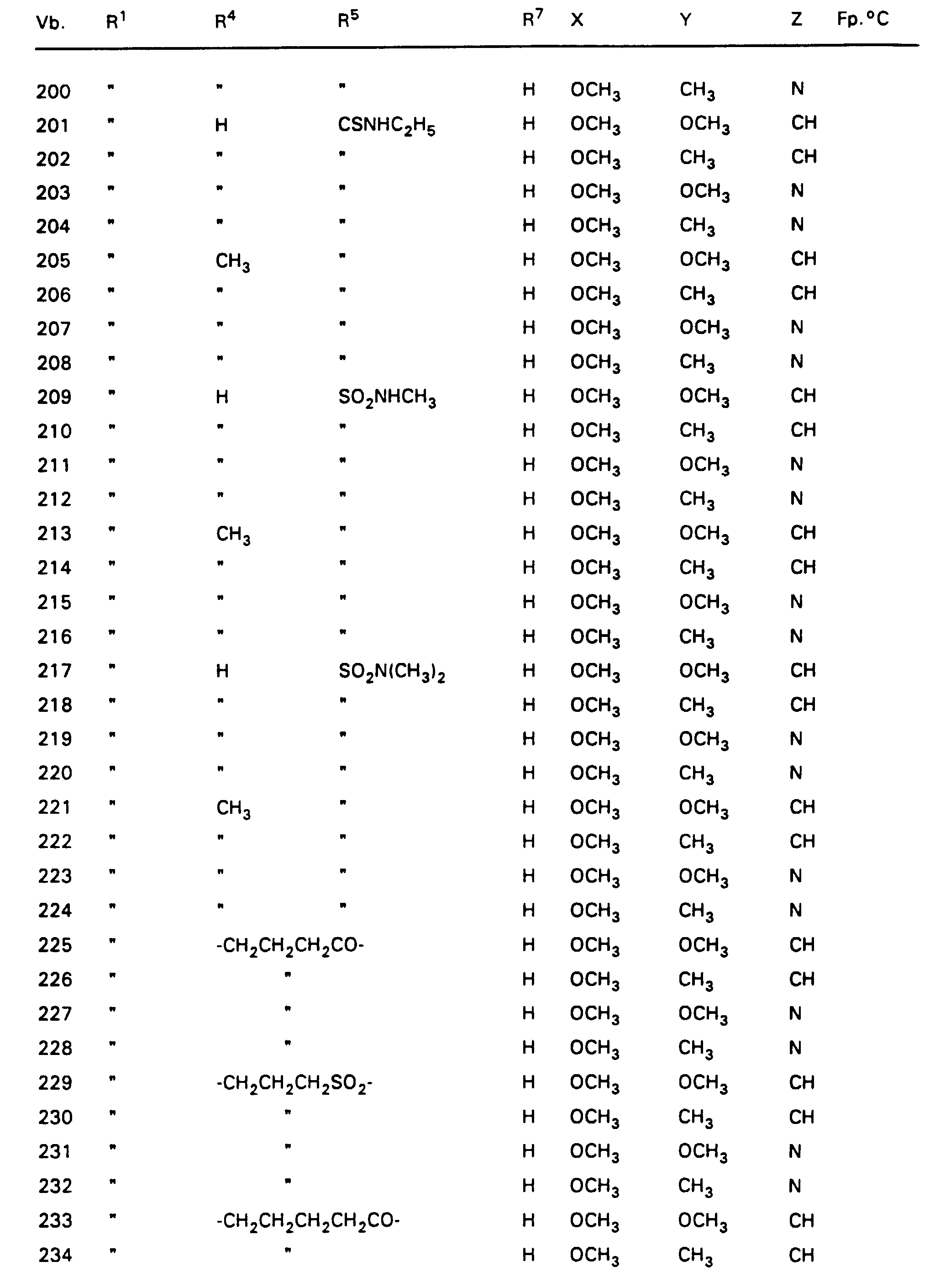

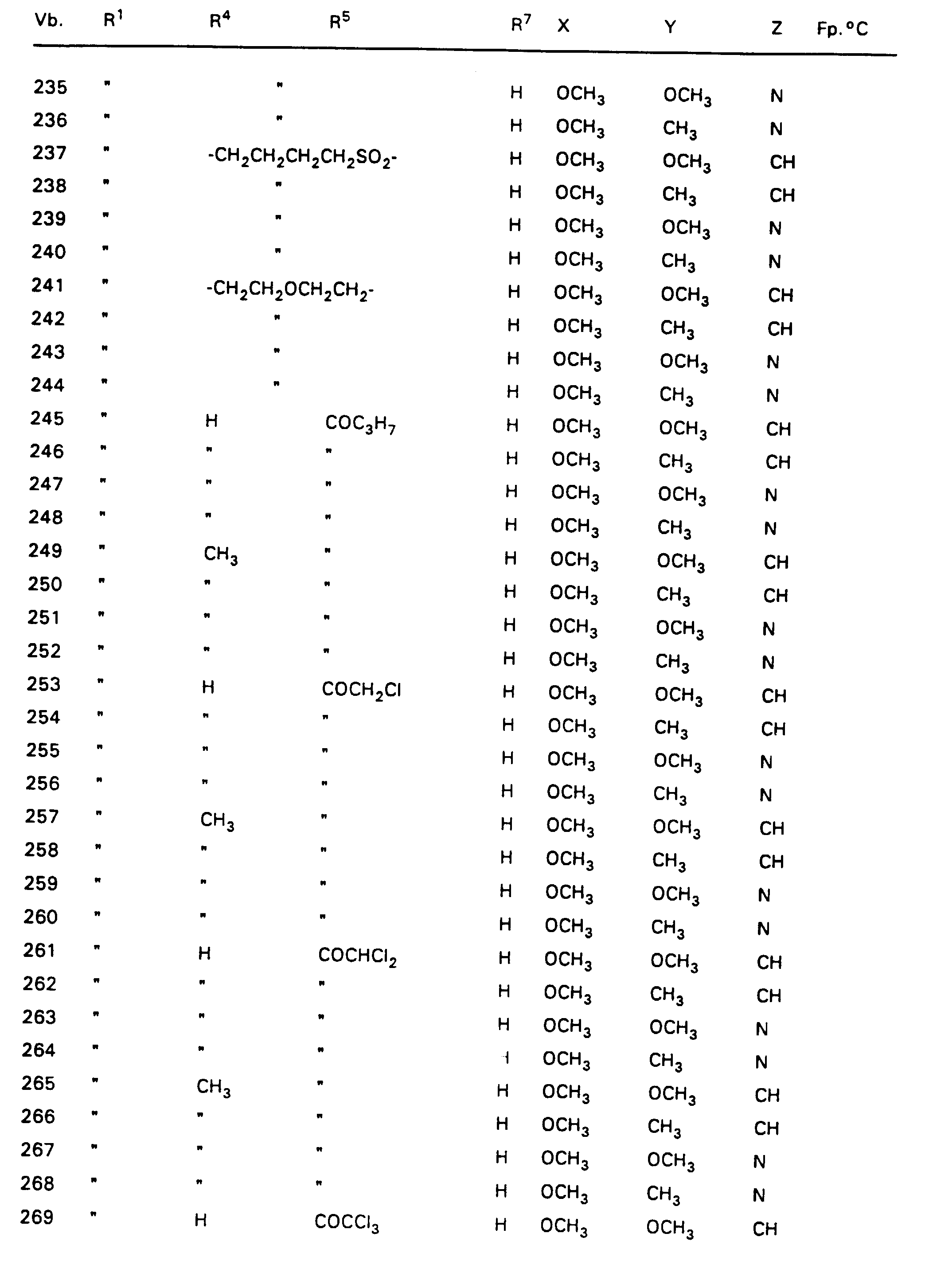

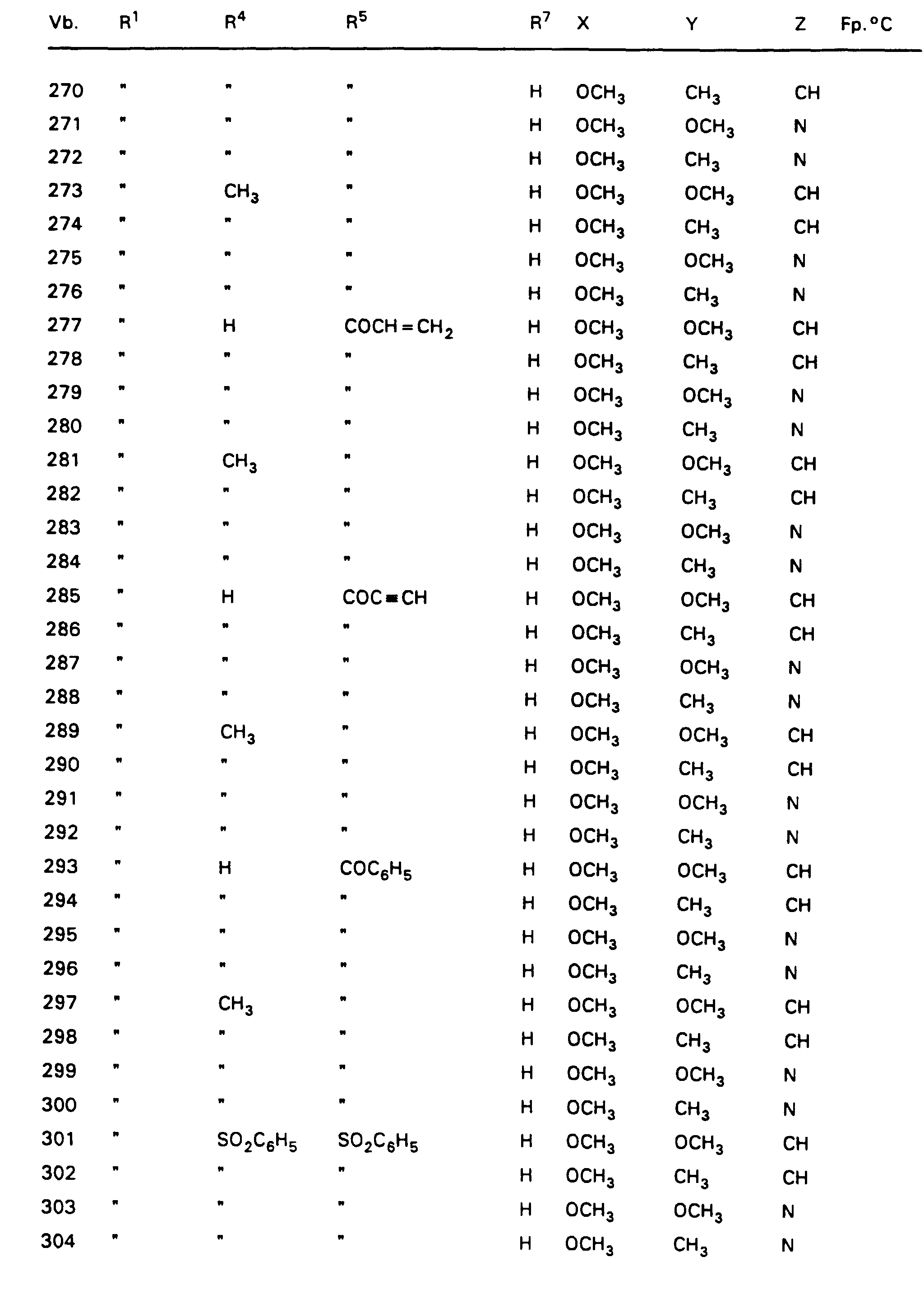

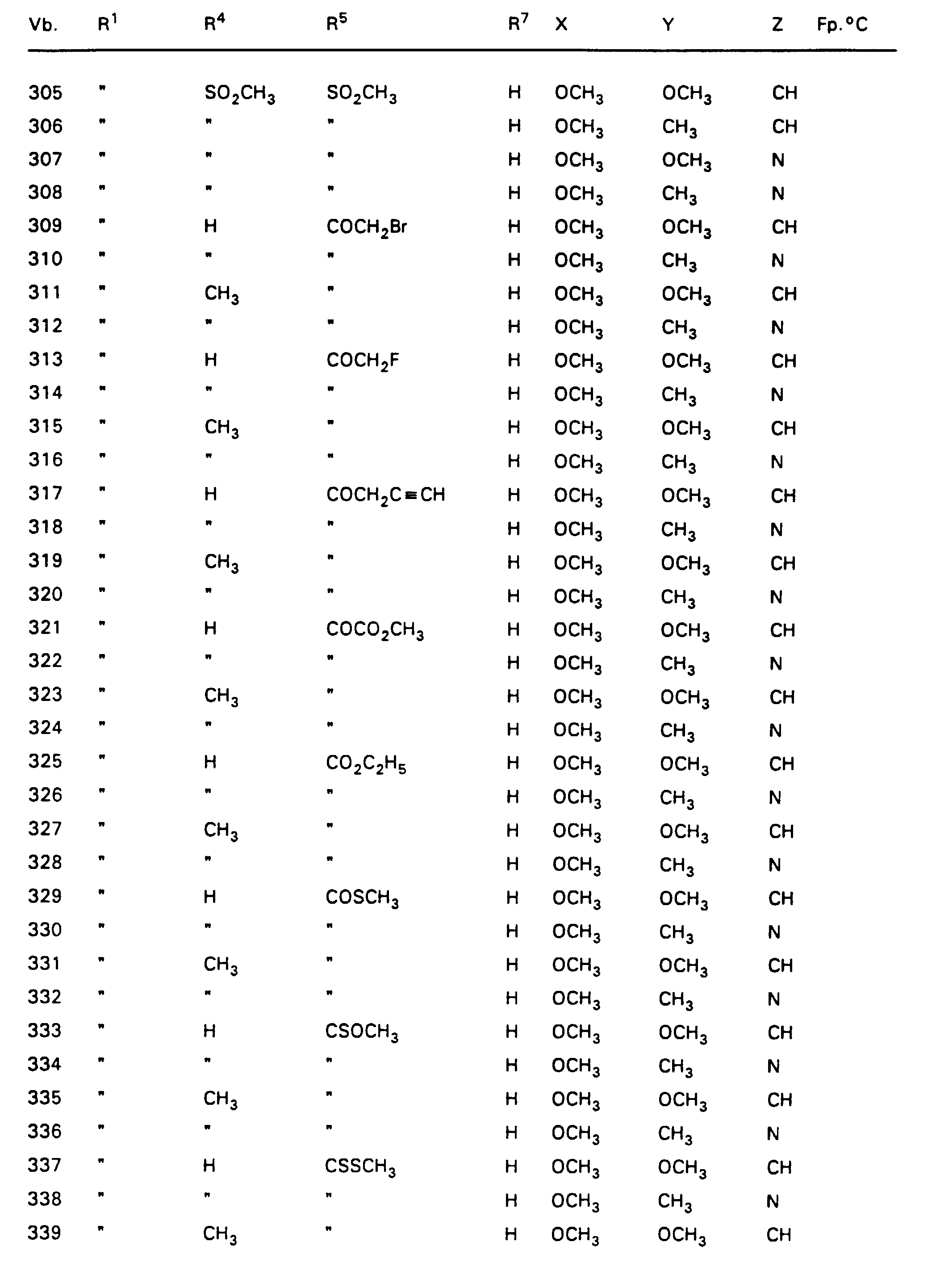

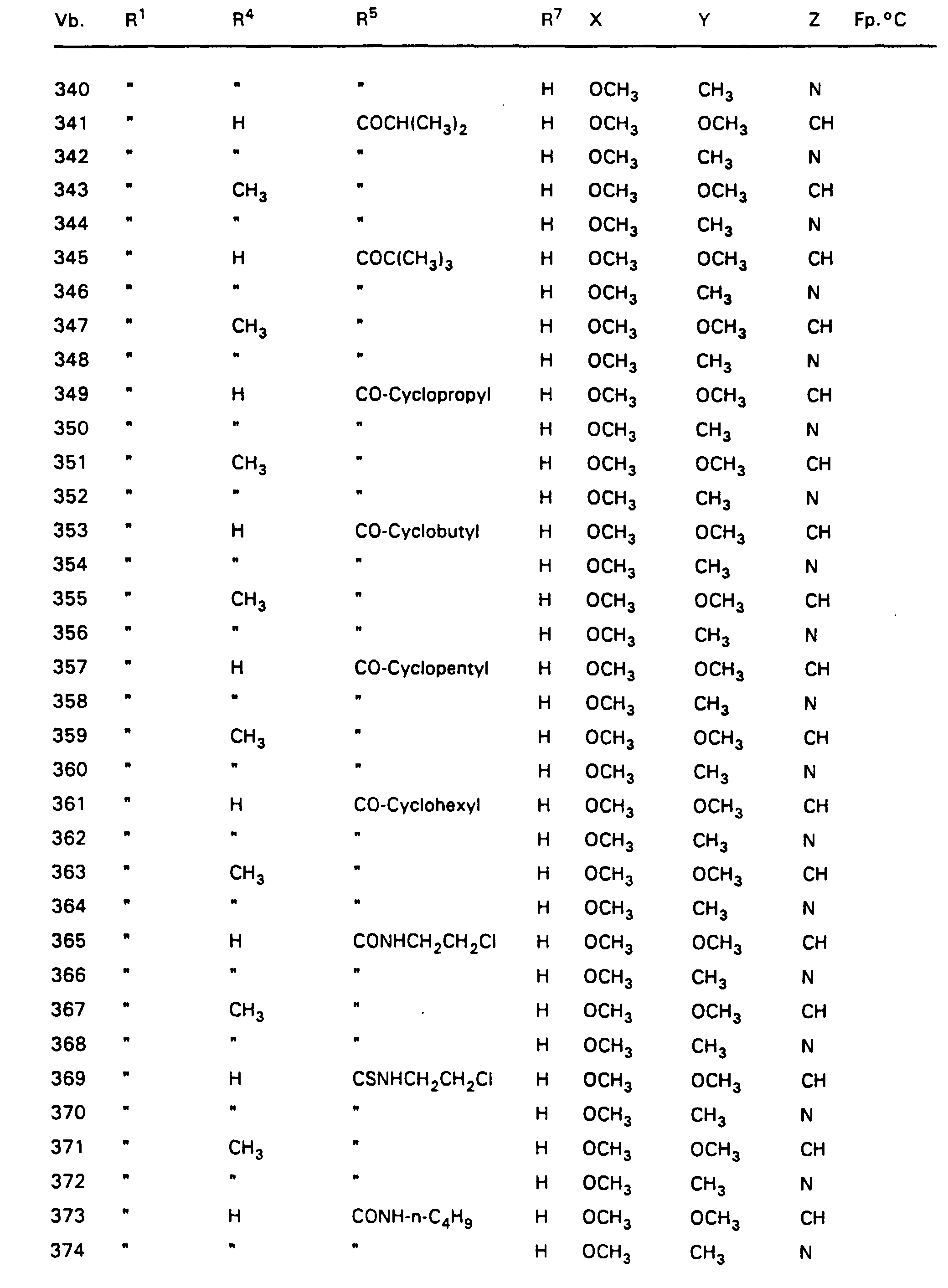

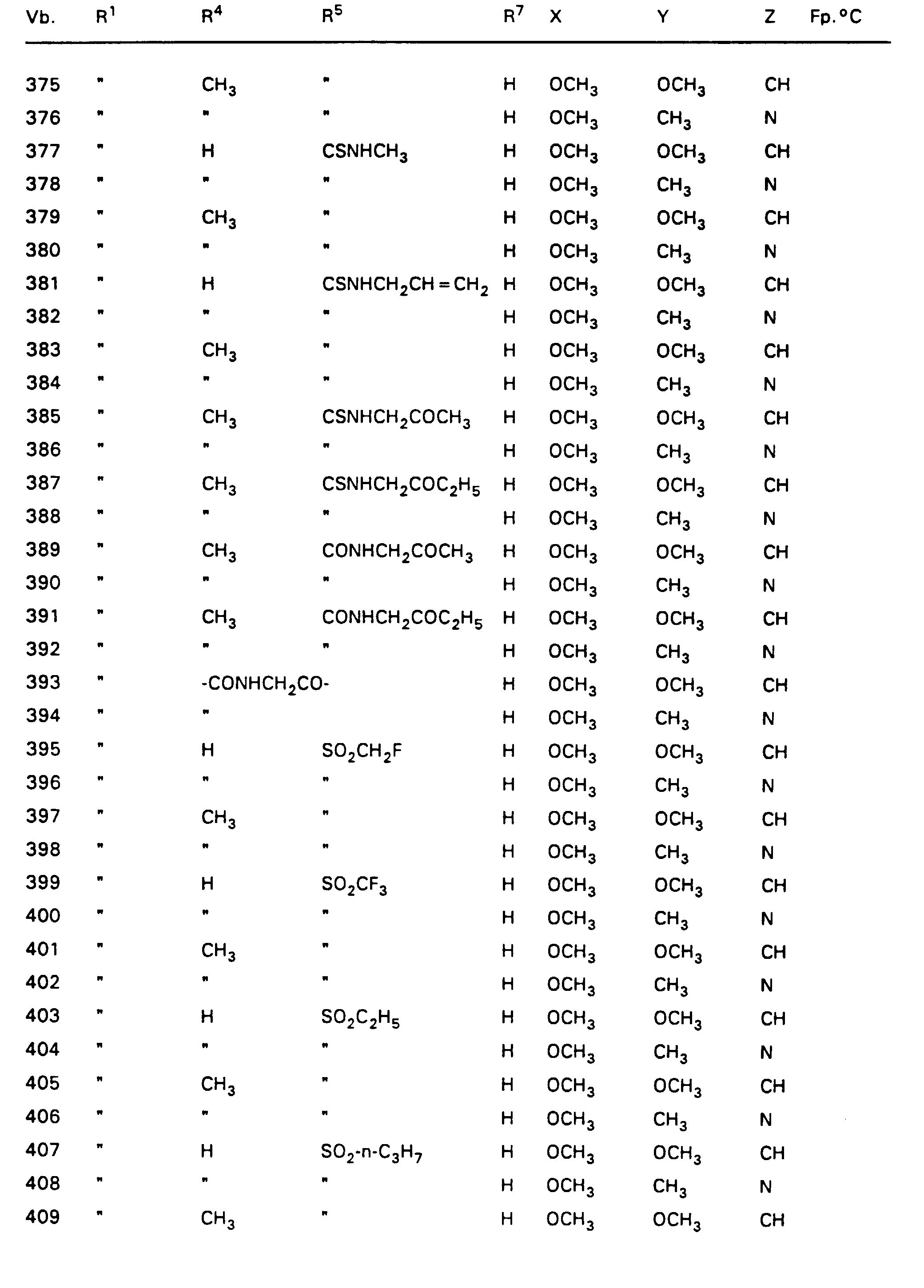

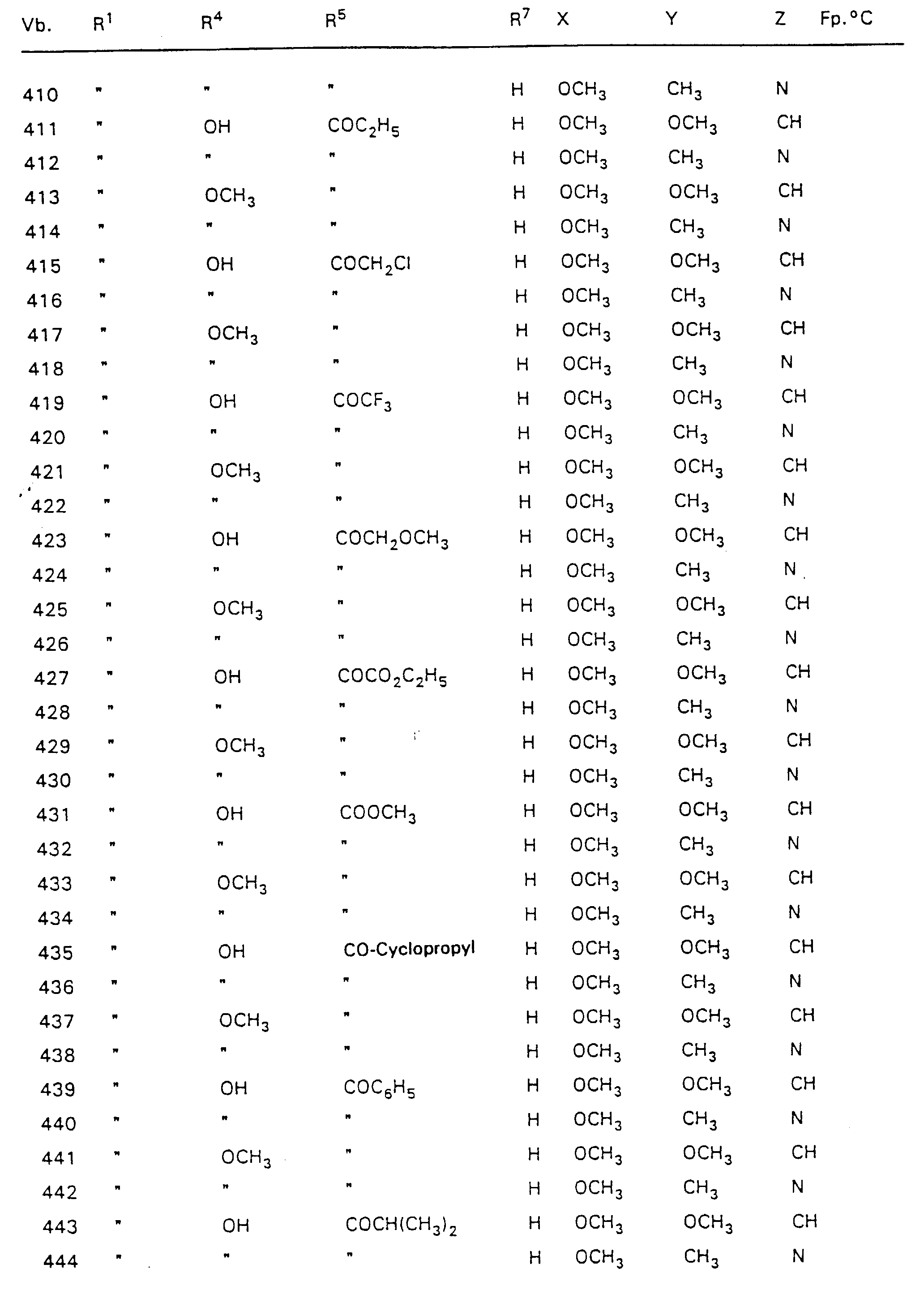

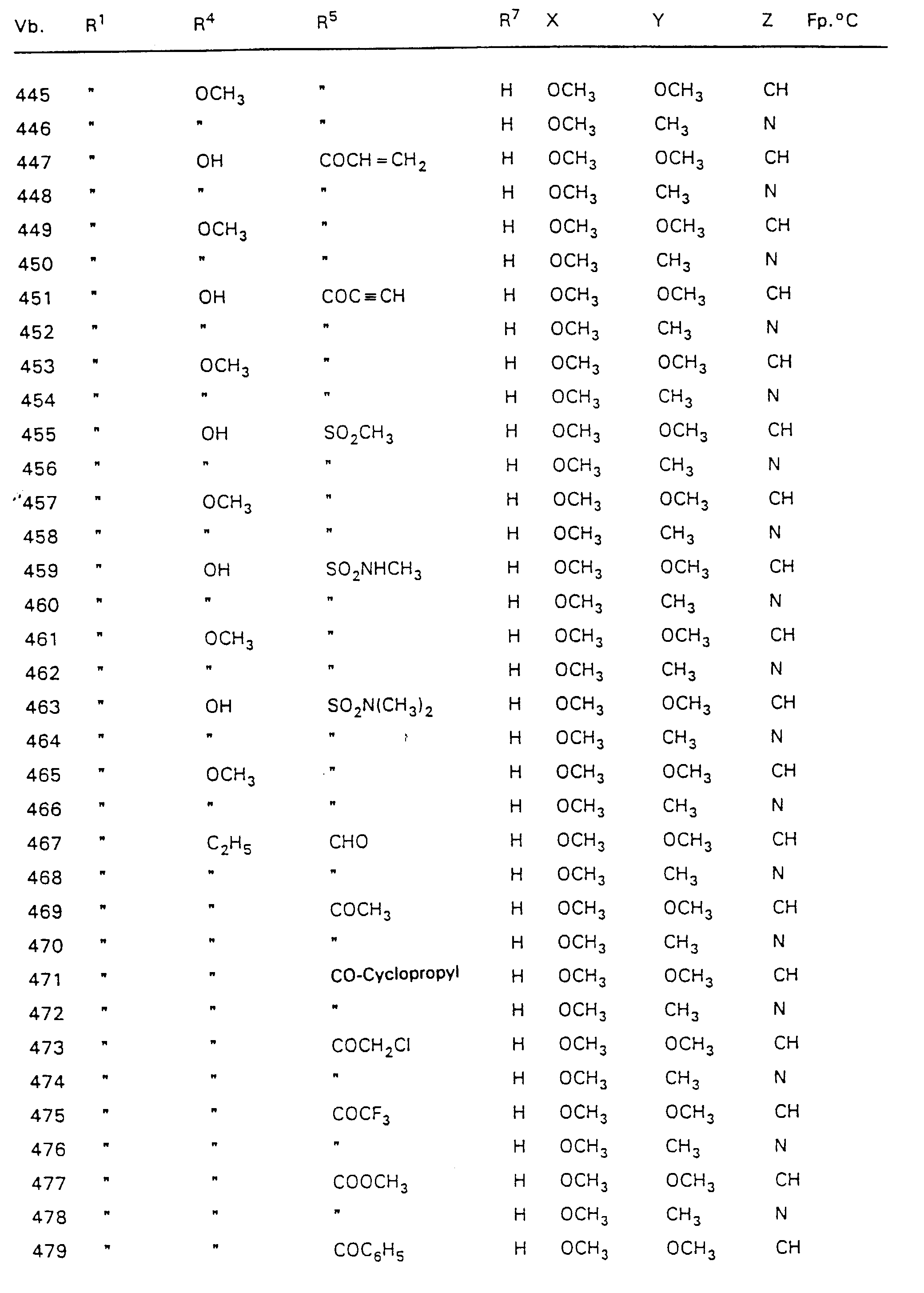

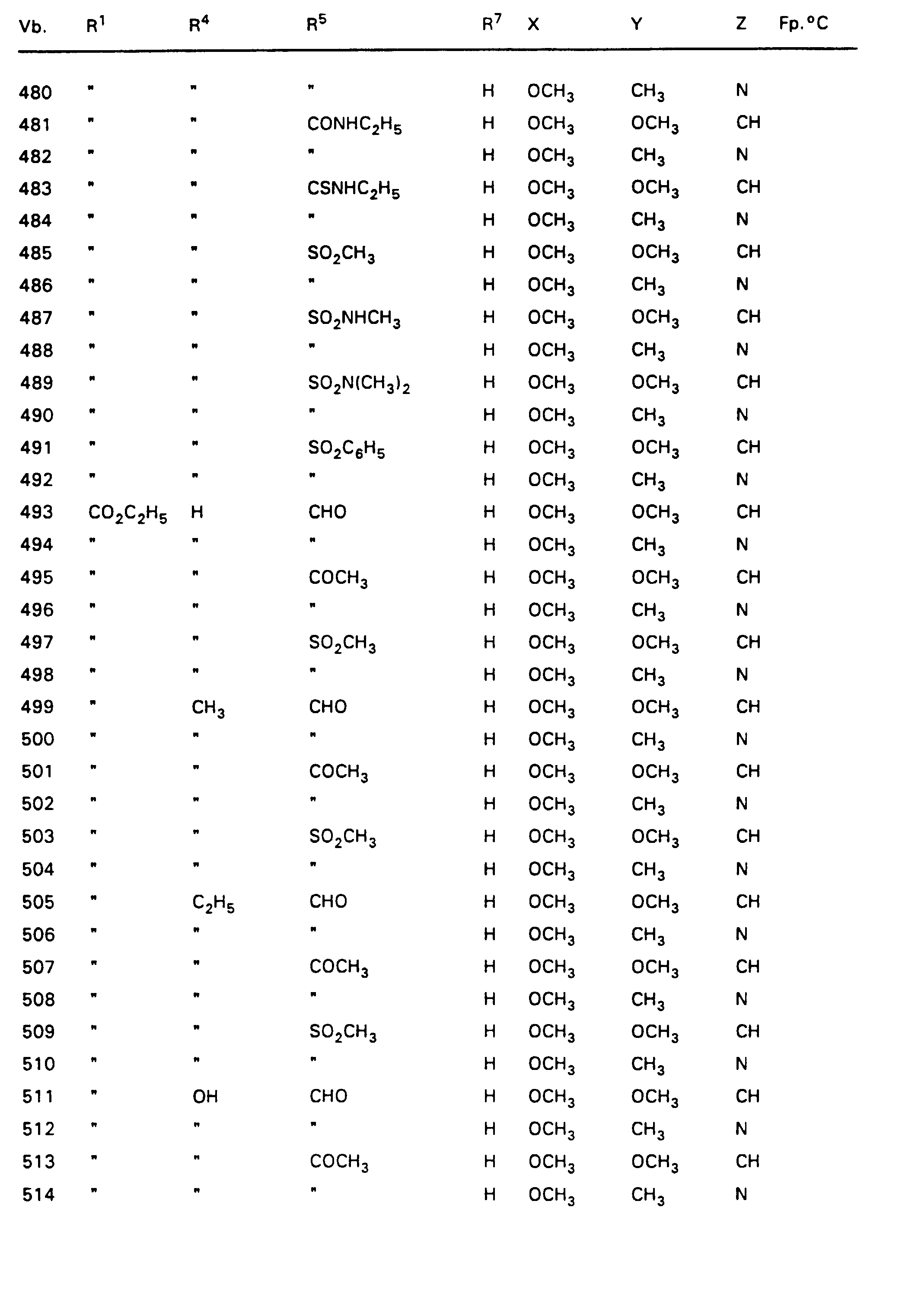

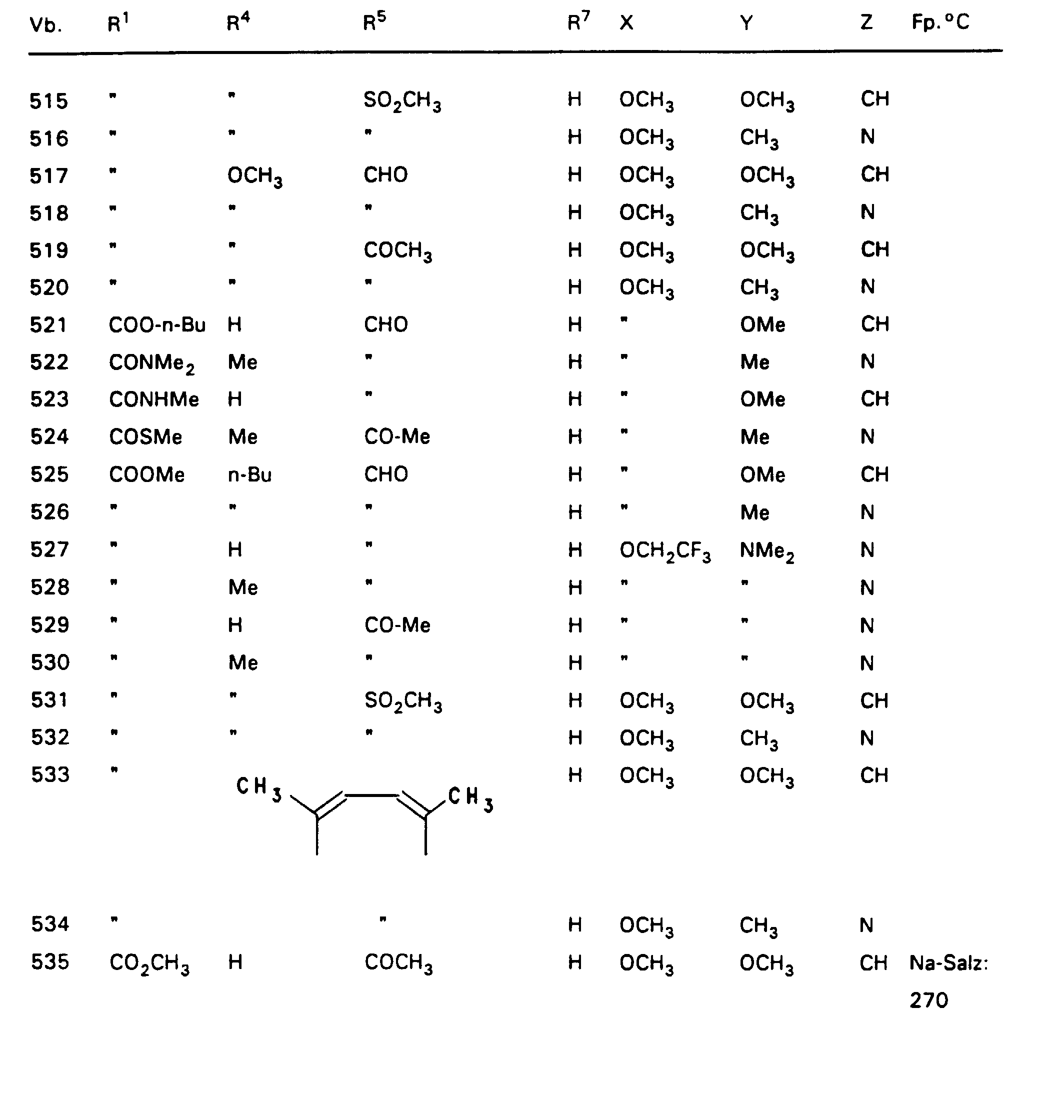

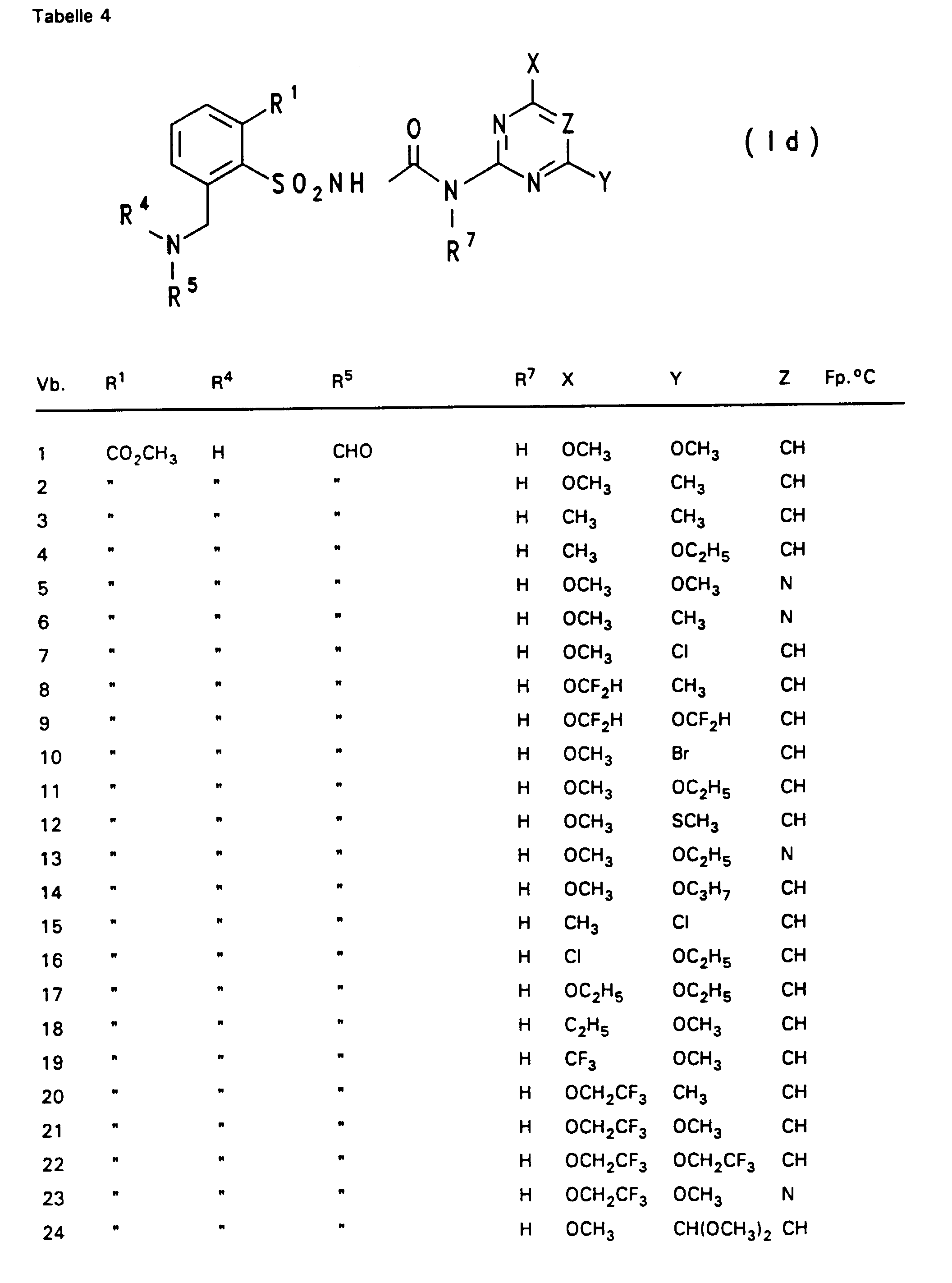

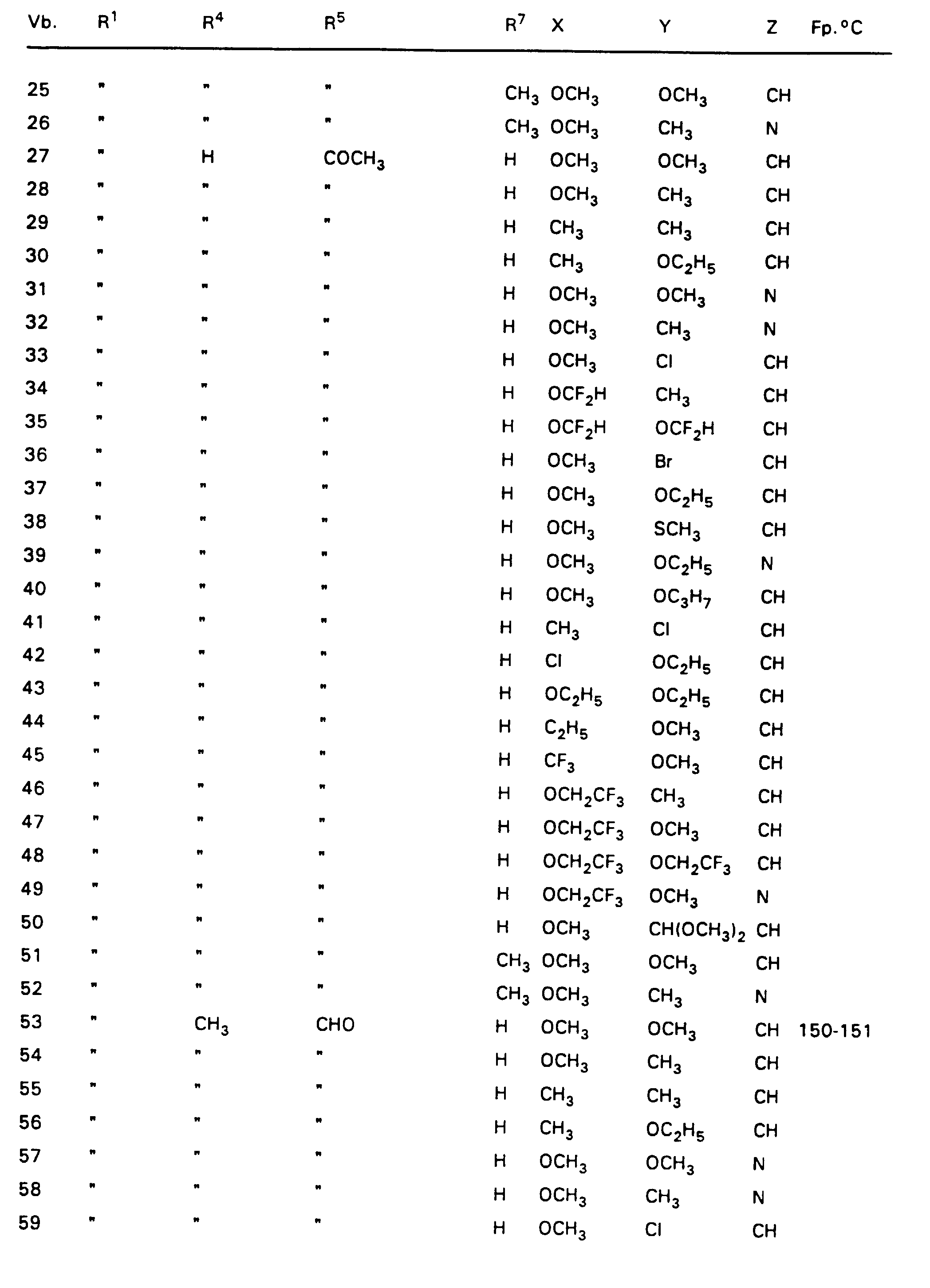

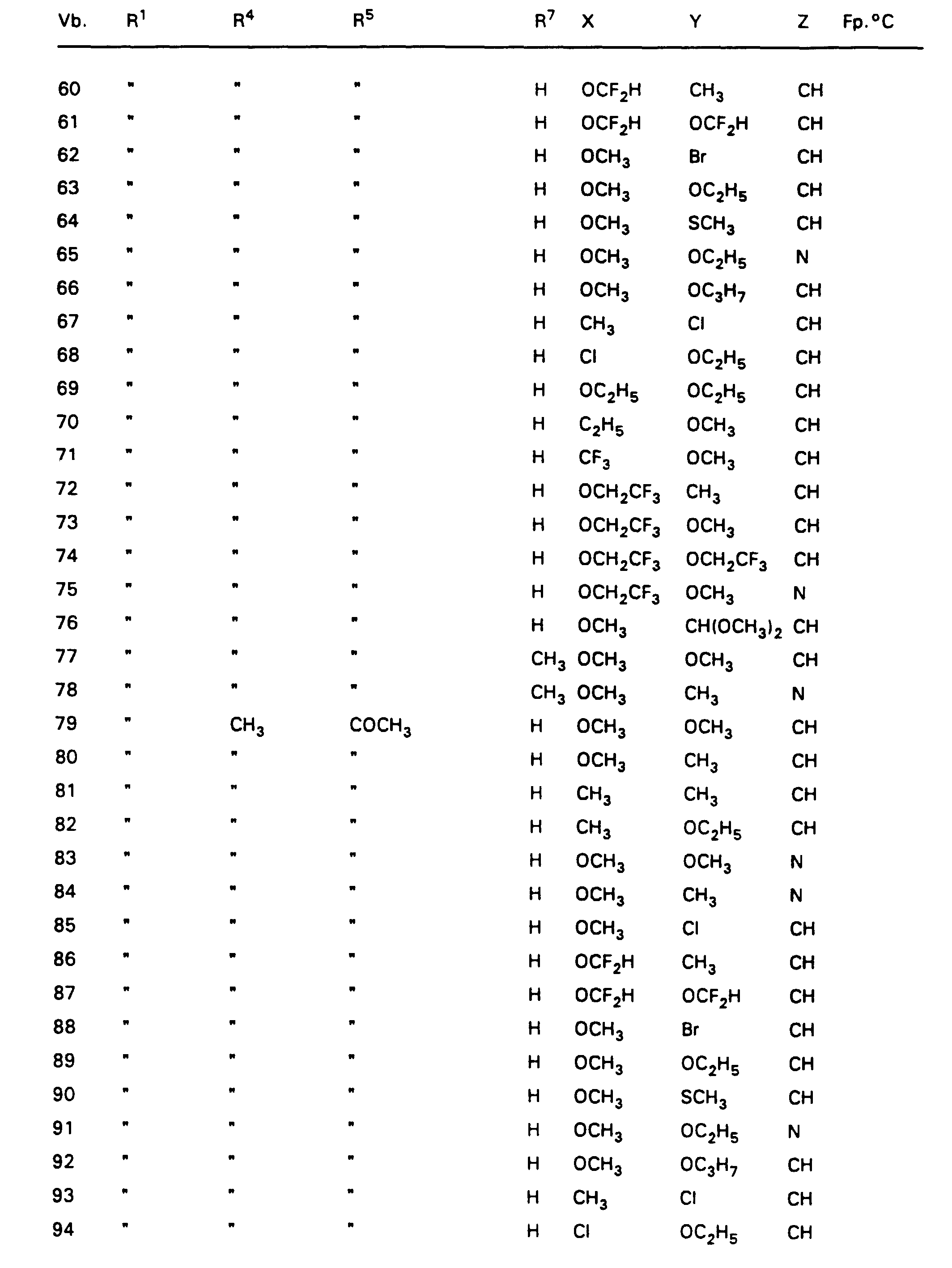

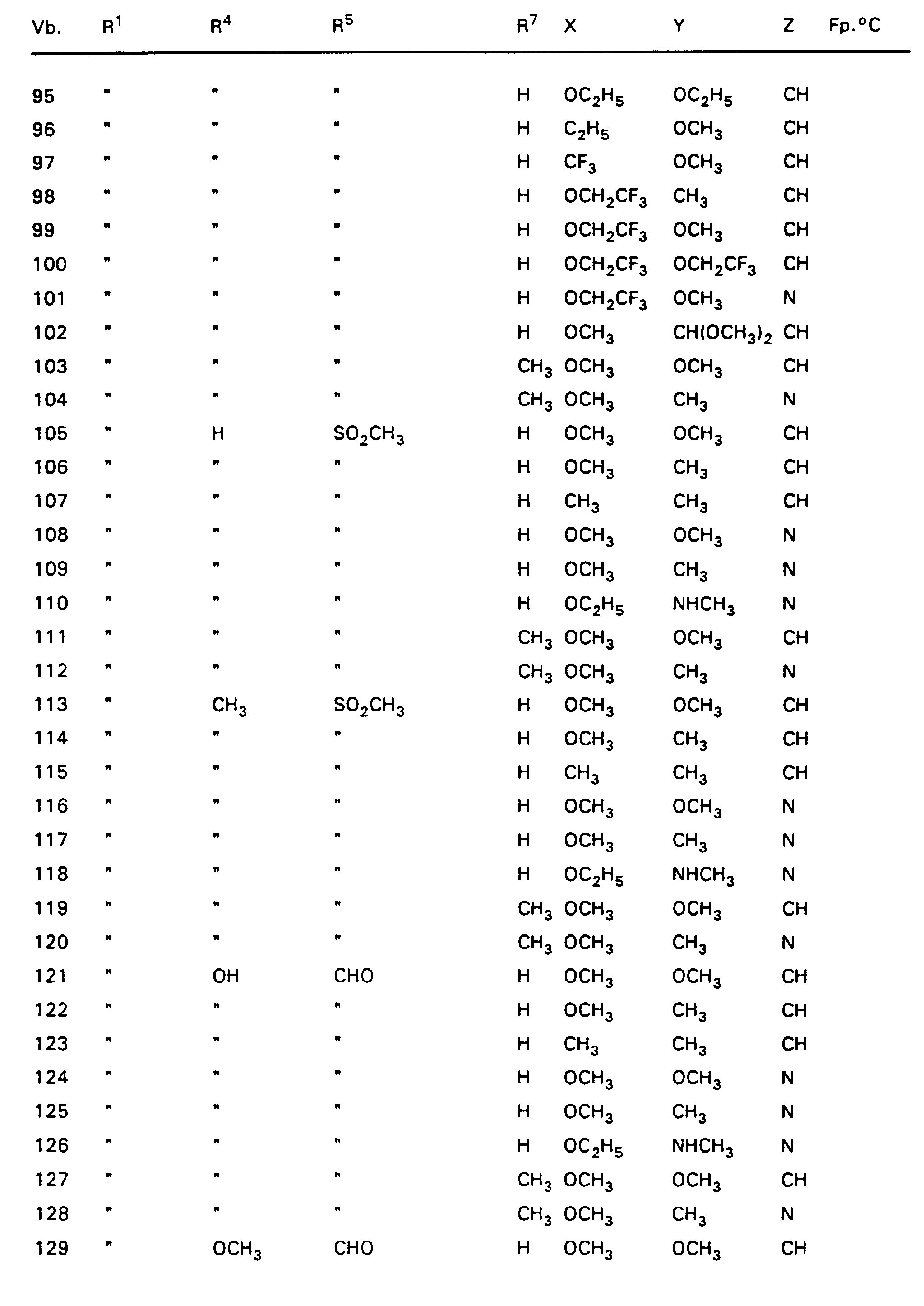

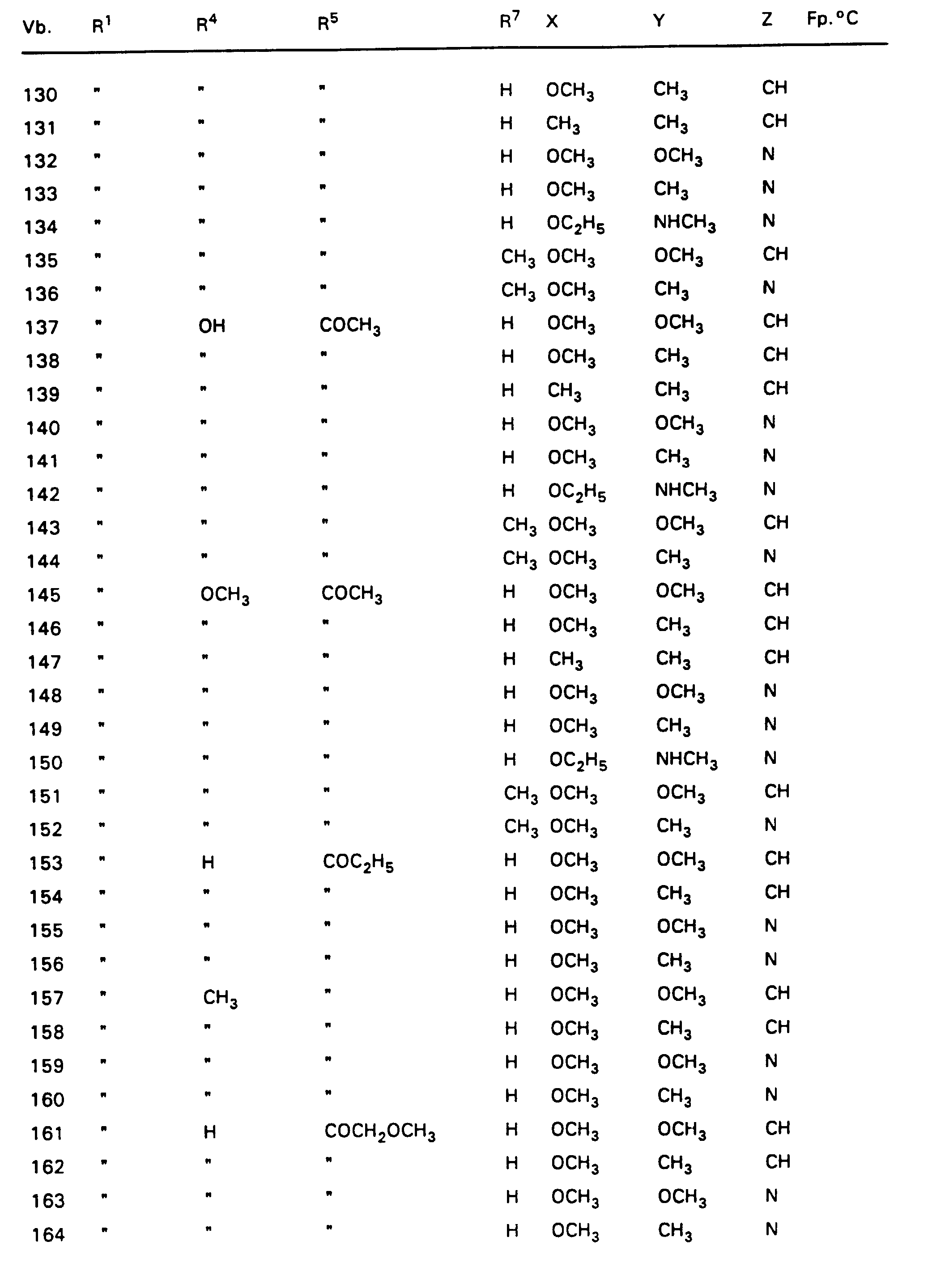

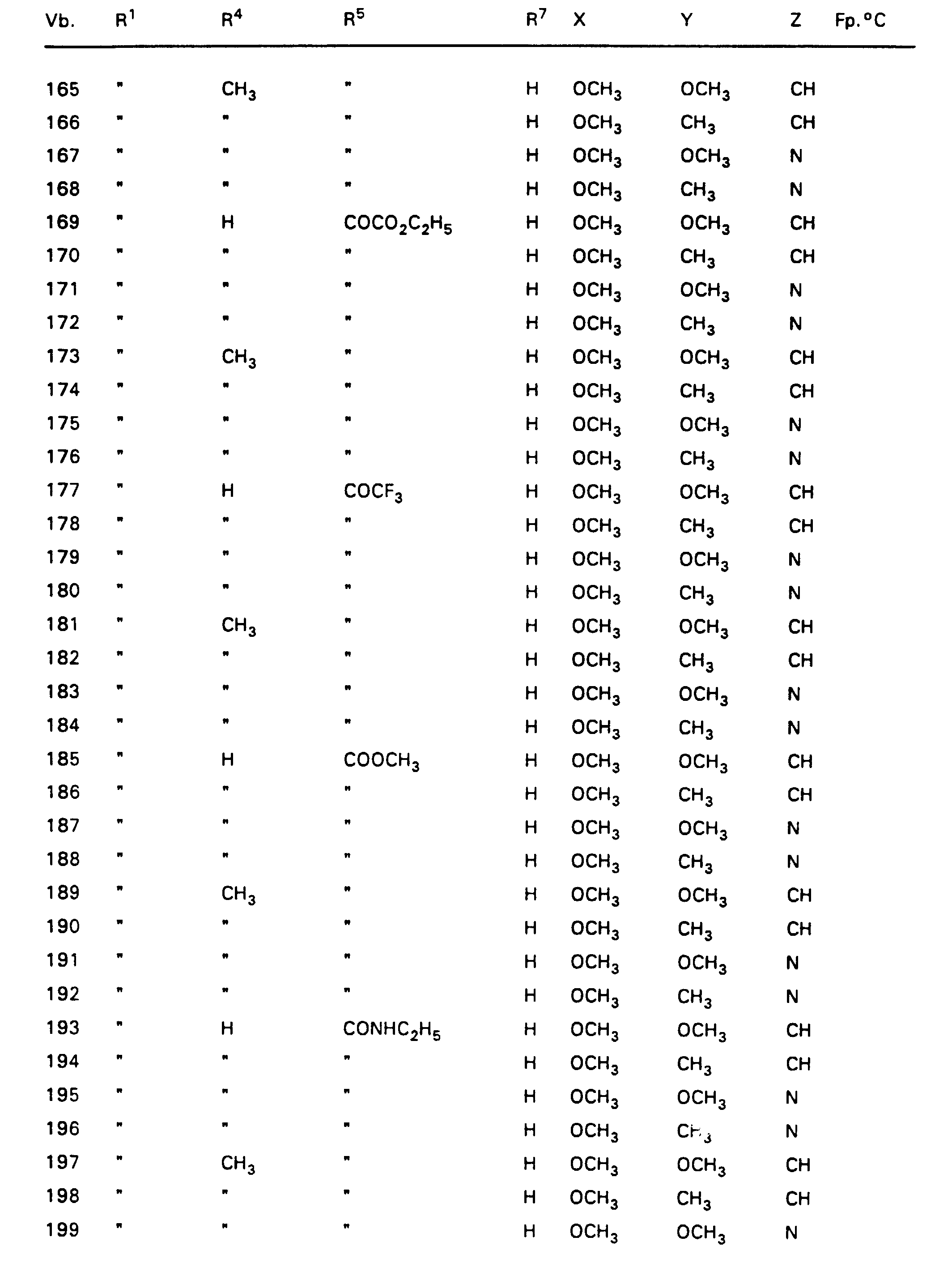

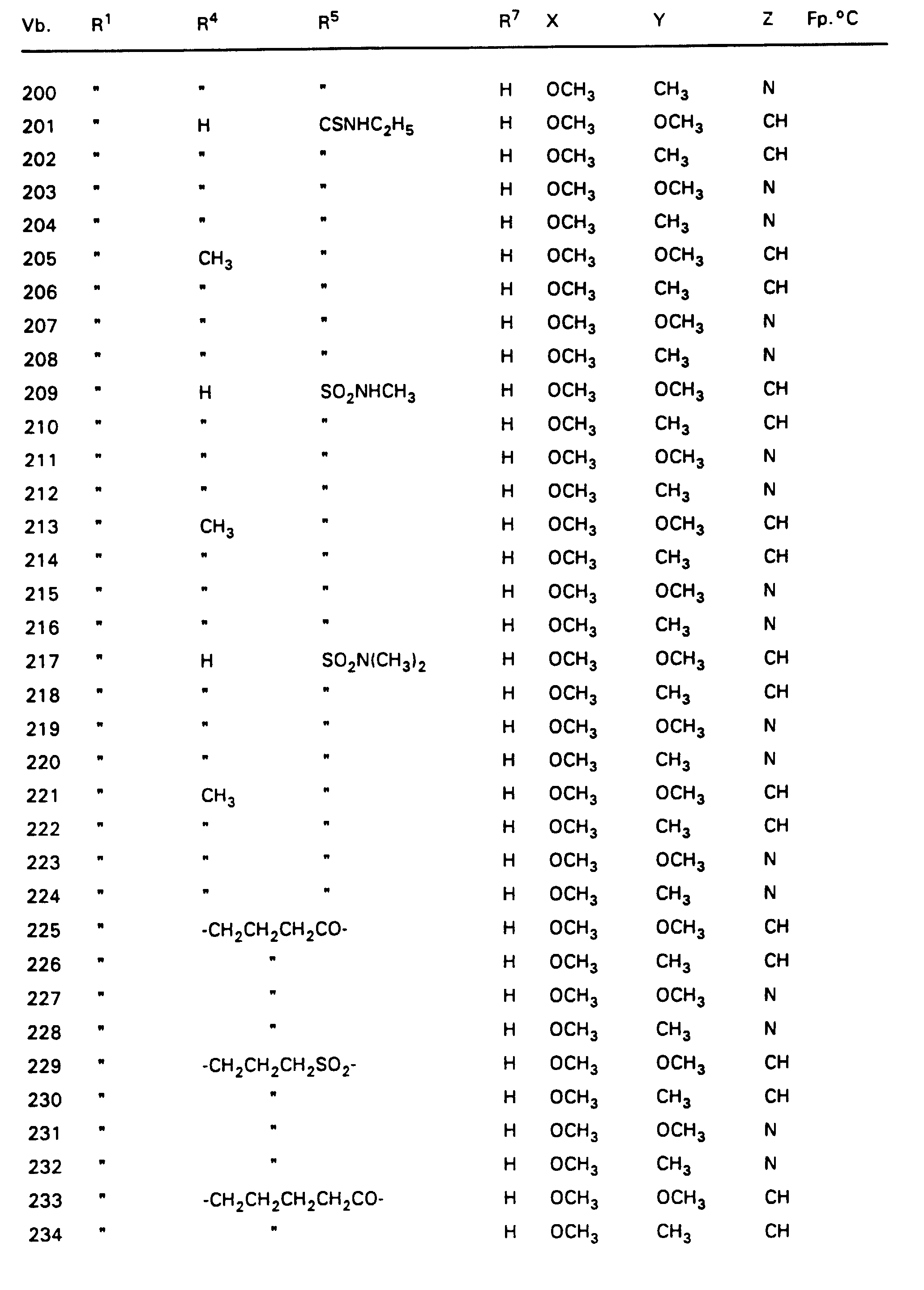

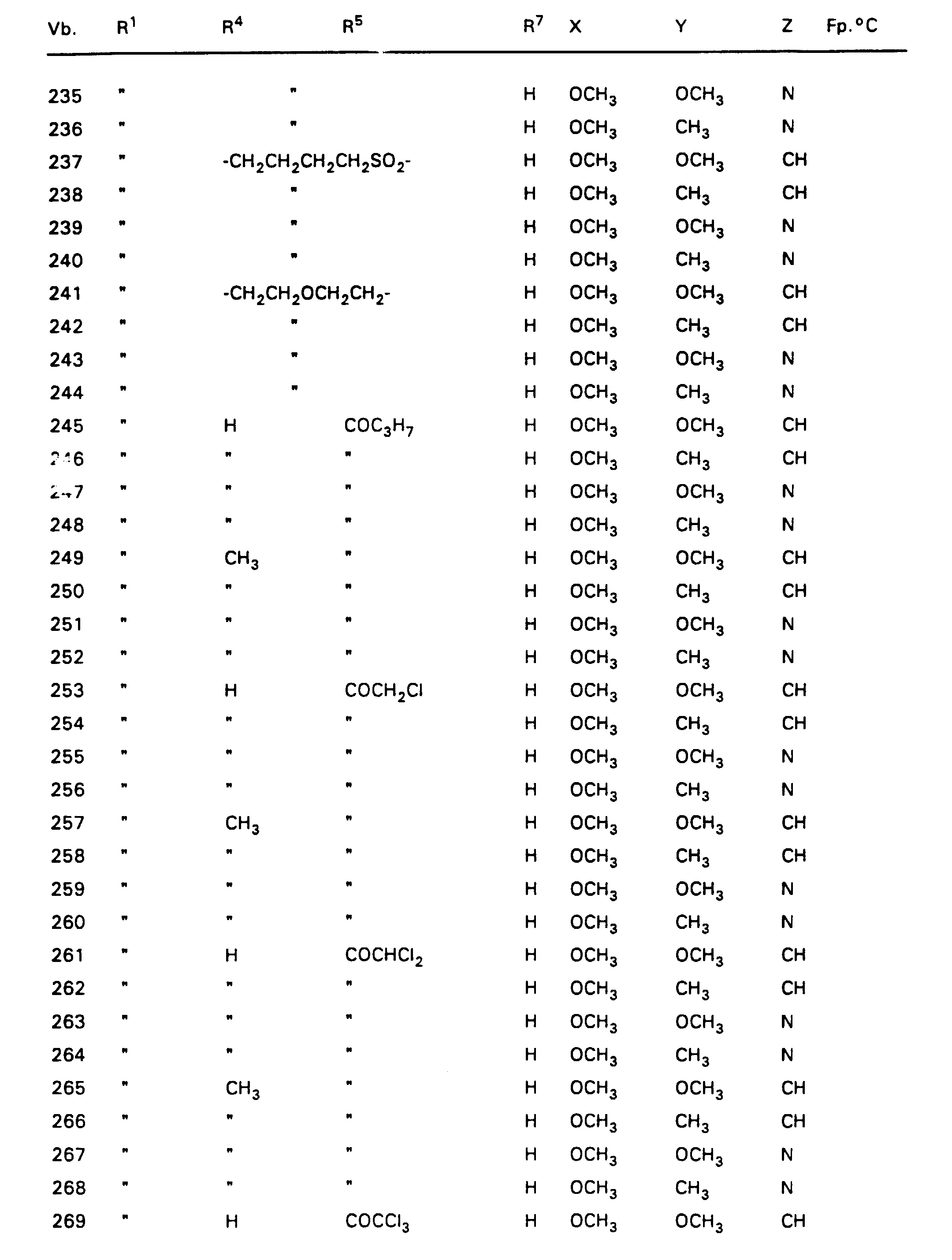

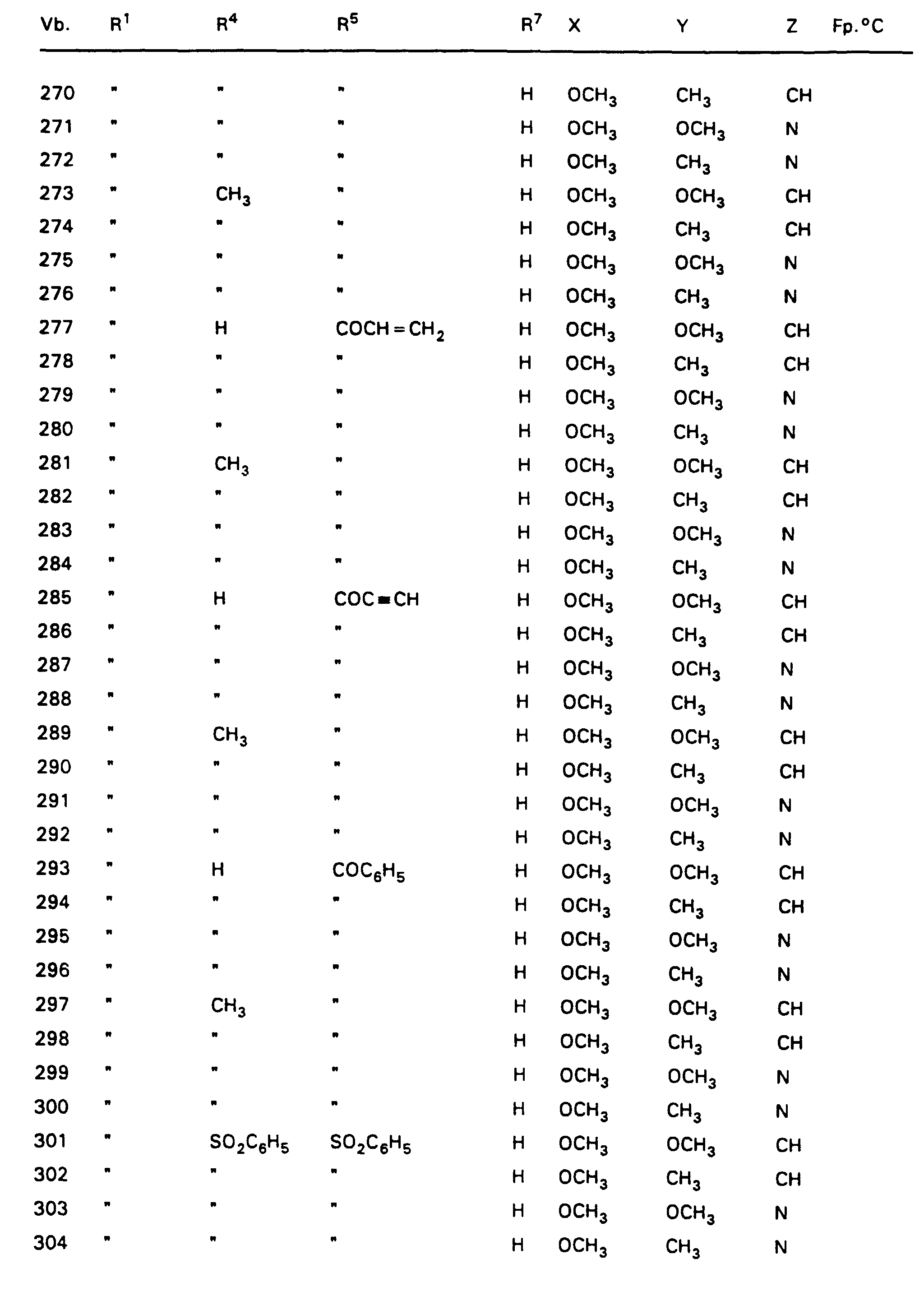

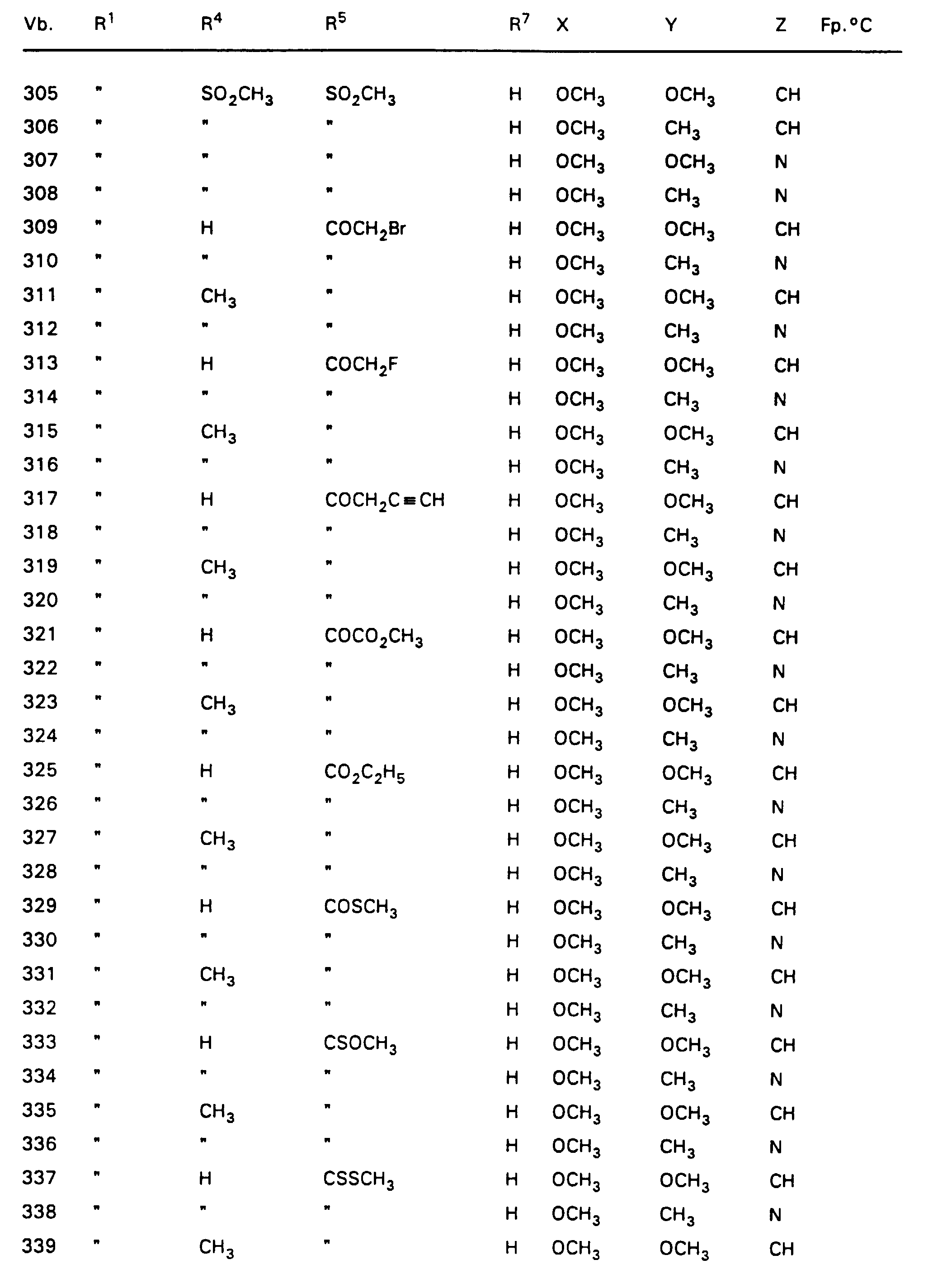

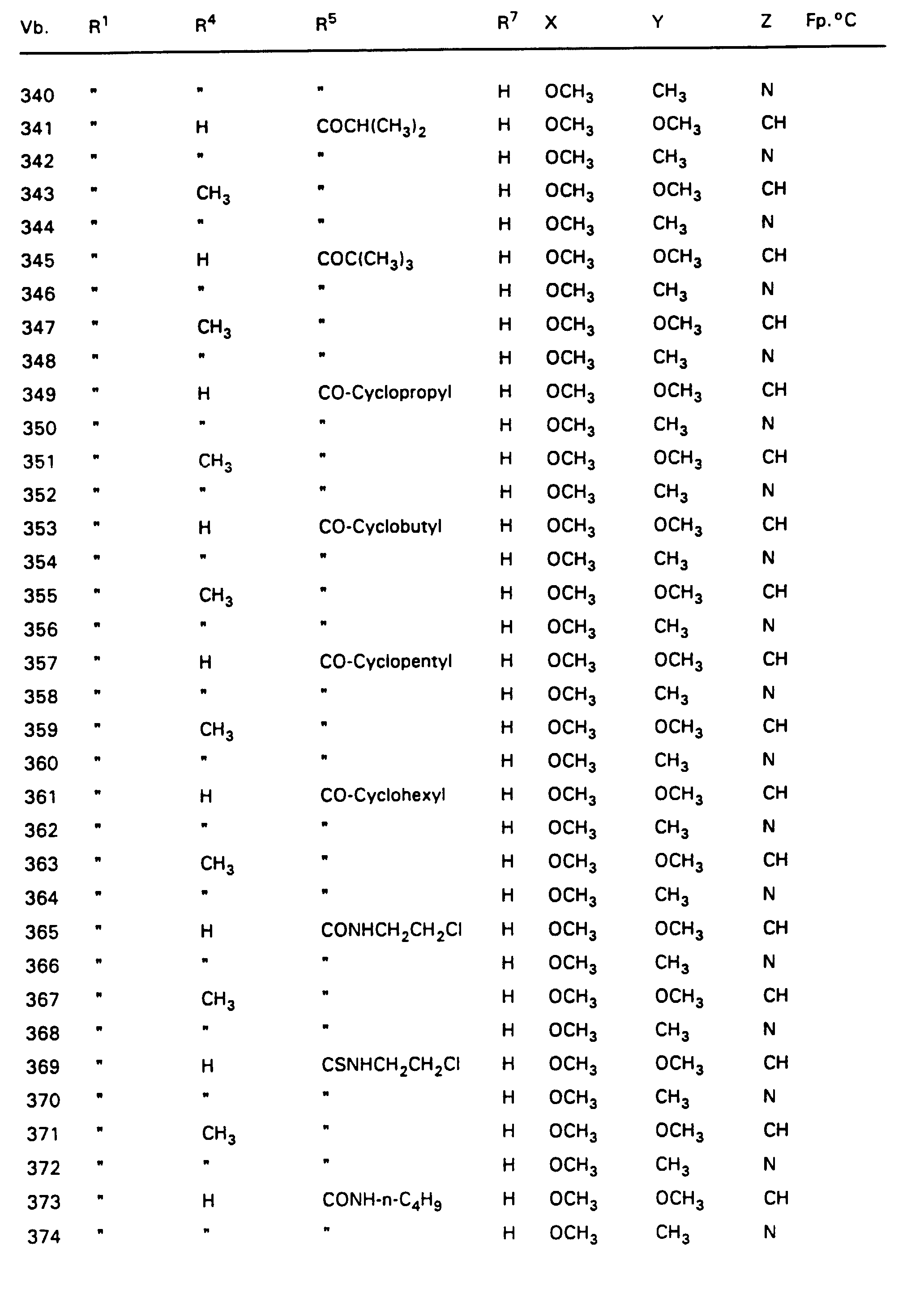

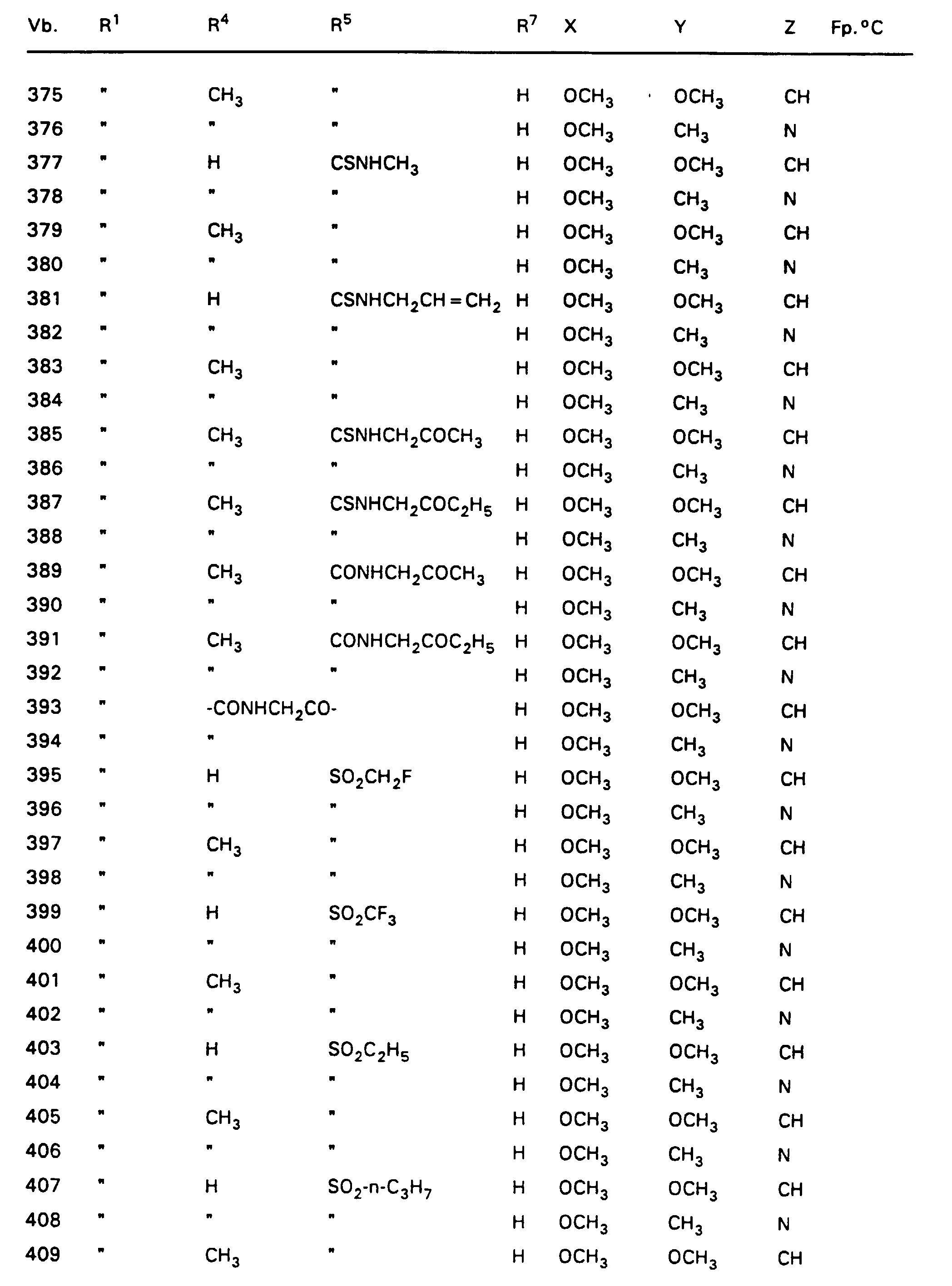

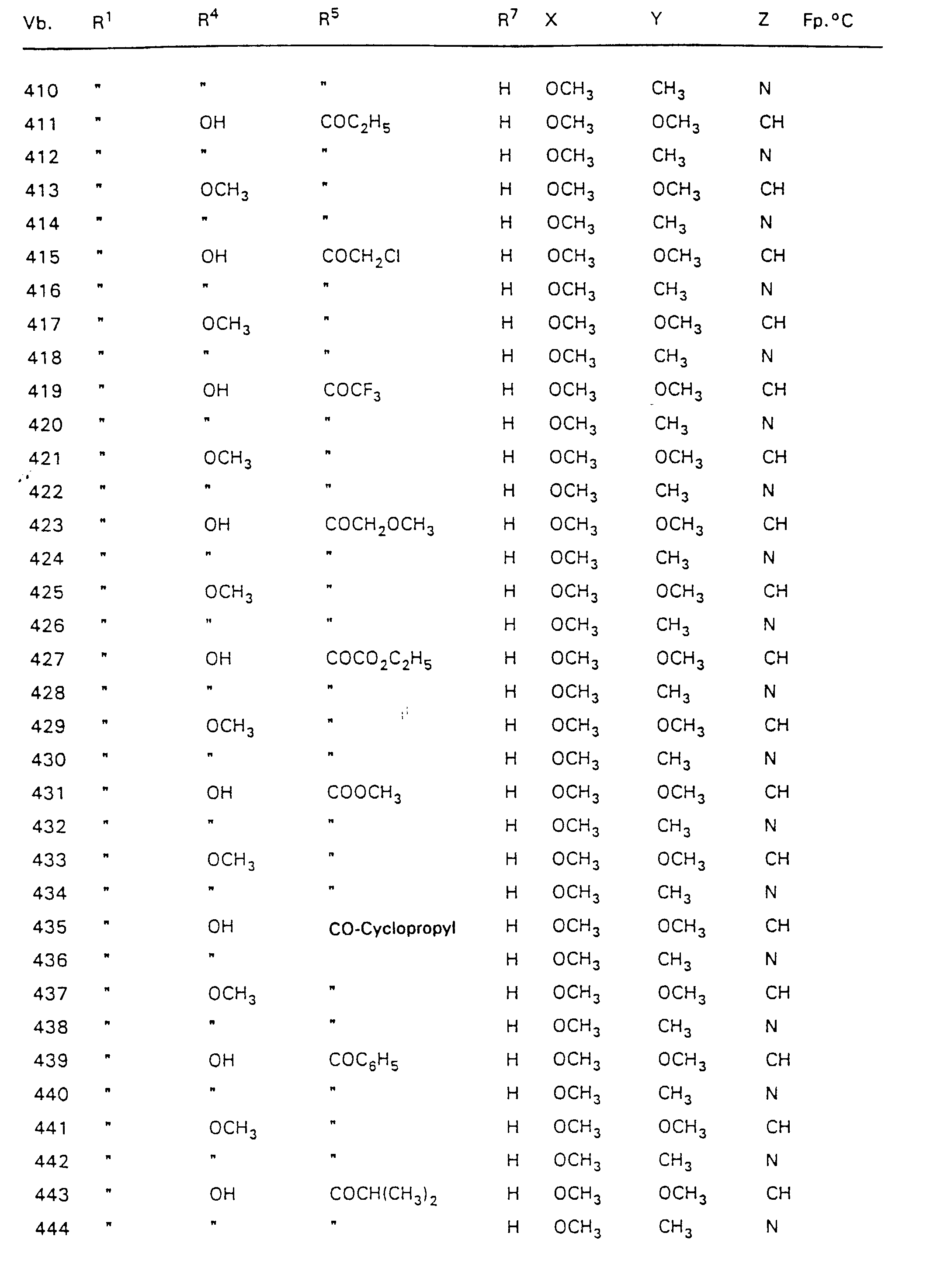

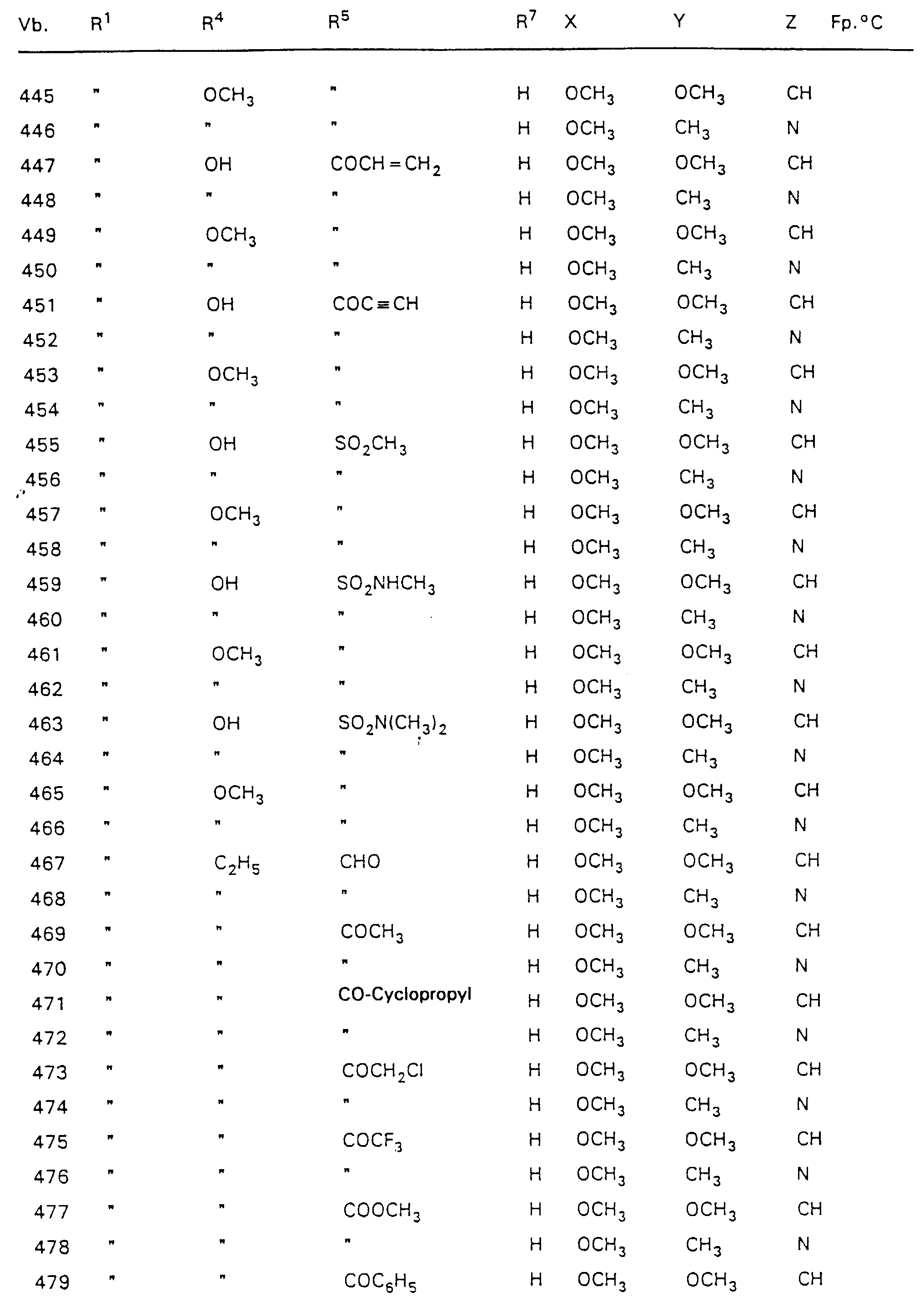

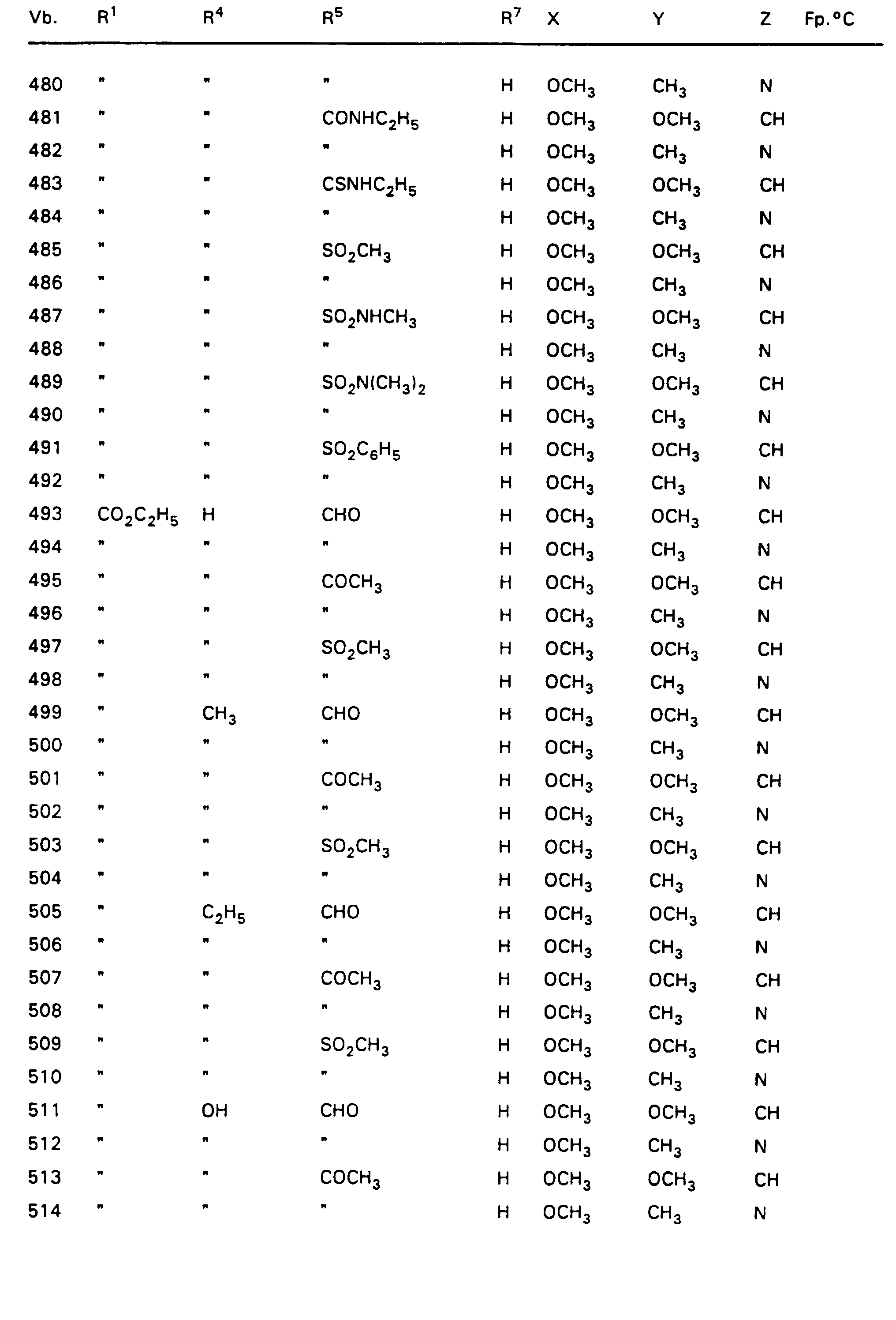

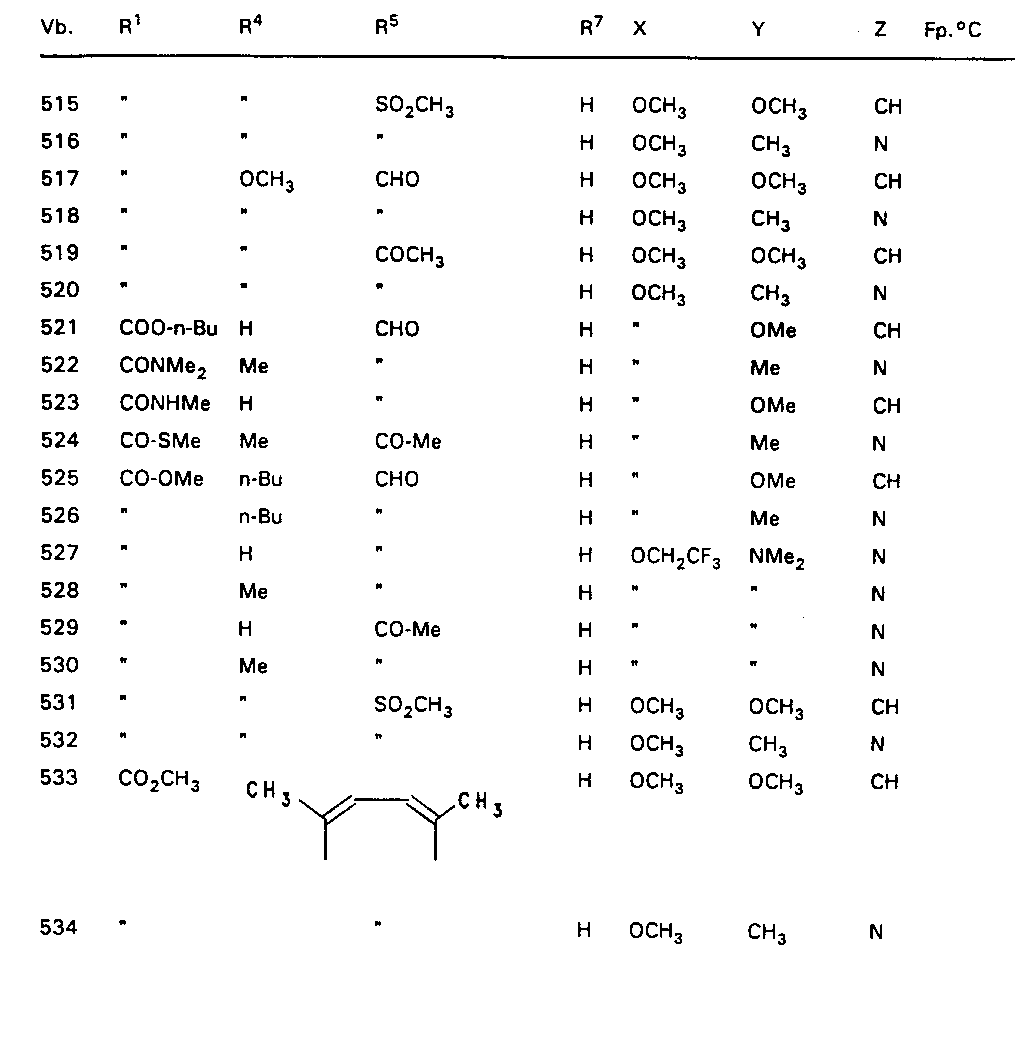

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (1), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (1), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | |
|---|---|
| 75 Gewichtsteile | einer Verbindung der Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | |
|---|---|
| 25 Gewichtsteile | einer Verbindung der Formel (I), |
| 5 " | 2,2'-dinaphthylmethan-6,6'-disulfonsaures Natrium |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen der Formel (I) oder deren Salze werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele 1, 27, 53 bis 55, 58, 59, 79, 105, 113, 129, 145, 153, 177, 181, 185, 189, 213, 217, 221, 253, 257, 421, 425, 429, 433, 437, 445, 457, 461, 465, 467, 485, 487, 535 bis 543, 547, 551 bis 559, 562, 563 und 565 bis 578 aus Tabelle 1 sowie die Beispiele 1, 27, 53, 79, 153, 161, 169, 177, 181, 185, 253, 261, 269, 341, 349, 365 und 373 aus Tabelle 2 sowie Beispiele 27 und 535 aus Tabelle 3 sowie Beispiel 53 aus Tabelle 4 sehr gute herbizide Wirkung gegen Schadpflanzen wie Sinapis alba, Stellaria media, Chrysanthemum segetum und Lolium multiflorum im Vorauflaufverfahren bei einer Aufwandmenge von 0,3 kg bis 0,005 kg Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen der Formel (I) oder deren Salze werden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen der Beispiele 1, 27, 53 bis 55, 58, 59, 79, 105, 113, 129, 145, 153, 177, 181, 185, 189, 213, 217, 221, 253, 257, 421, 425, 429, 433, 437, 445, 457, 461, 465, 467, 485, 487, 535 bis 543, 547, 551 bis 559, 562, 563 und 565 bis 578 aus Tabelle 1 sowie die Beispiele 1, 27, 53, 79, 153, 161, 169, 177, 181, 185, 253, 261, 269, 341, 349, 365 und 373 aus Tabelle 2 sowie Beispiele 27 und 535 aus Tabelle 3 sowie Beispiel 53 aus Tabelle 4 sehr gute herbizide Wirkung gegen Schadplanzen wie Sinapis alba, Stellaria media, Chrysanthemum segetum und Lolium multiflorum im Nachauflaufverfahren bei einer Aufwandmenge von 0,3 kg bis 0,005 kg Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen der Formel (I) oder deren Salze in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) oder deren Salze weisen somit eine hohe Selektivität bei Anwendung zur Bekämpfung von unerwünschtem Pflanzenwuchs in landwirtschaftlichen Kulturen auf.

## Patentansprüche

1. Verbindungen der Formel (I) und deren Salze, 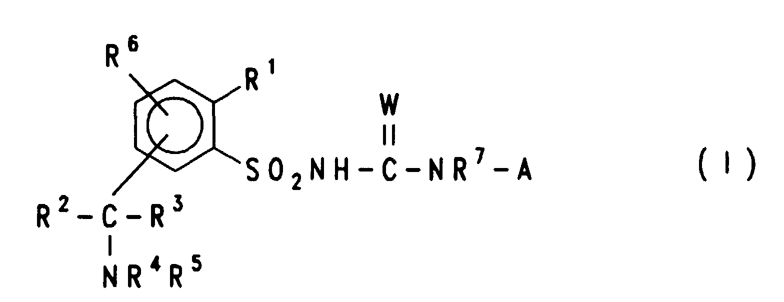 worin
R¹ CO-Q-R⁸_{,}
R², R³ unabhängig voneinander H oder (C₁-C₄)Alkyl,
R⁴ H, (C₁-C₈)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, [(C₁-C₄)Alkoxy]-carbonyl und CN substituiert ist, oder (C₃-C₆)Alkenyl, das unsubstituiert oder durch ein oder mehrere Halogenatome substituiert ist, oder (C₃-C₆)Alkinyl, das unsubstituiert oder durch ein oder mehrere Halogenatome substituiert ist, oder Hydroxy, (C₁-C₄)Alkoxy, [(C₁-C₄)Alkyl]-carbonyl oder (C₁-C₄)Alkylsulfonyl, wobei jeder der letztgenannten drei Reste unsubstituiert oder im Alkylteil durch ein oder mehrere Halogenatome oder durch (C₁-C₄)Alkoxy oder (C₁-C₄)Alkylthio substituiert ist, oder Phenylsulfonyl, wobei der Phenylrest unsubstituiert oder substituiert ist, und
R⁵ (C₁-C₄)Alkylsulfonyl oder (C₃-C₆)Alkenylsulfonyl, wobei jeder der beiden letztgenannten Reste unsubstituiert oder durch ein oder mehrere Halogenatome oder durch (C₁-C₄)Alkoxy oder (C₁-C₄)Alkylthio substituiert ist, oder Phenylsulfonyl oder Phenylcarbonyl, wobei der Phenylrest in jedem der beiden letztgenannten Reste unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, Hydroxy, Amino, Nitro, Cyano, [(C₁-C₄)Alkoxy]-carbonyl, [(C₁-C₄)Alkyl]-carbonyl, Formyl, Carbamoyl, Mono- und Di-[(C₁-C₄)alkyl]-aminocarbonyl, Mono- und Di-[(C₁-C₄)alkyl]-amino, (C₁-C₄)Alkylsulfinyl und (C₁-C₄)Alkylsulfonyl substituiert ist, oder Mono- oder Di-[(C₁-C₄)Alkyl]-aminosulfonyl oder [(C₁-C₆)Alkyl]-carbonyl , wobei jeder der drei letztgenannten Reste unsubstituiert oder durch einen oder mehrere Reste aus der Gruppe Halogen, (C₁-C₄)Alkoxy, (C₁-C₄)Alkylthio, (C₁-C₄)Alkylsulfinyl, (C₁-C₄)Alkylsulfonyl, [(C₁-C₄)Alkyl]-carbonyl, [(C₁-C4)Alkoxy]-carbonyl und CN substituiert ist, oder Formyl, eine Gruppe der Formel -CO-CO-R', worin R'=H, OH, (C₁-C₄)-Alkoxy oder (C₁-C₄)Alkyl ist, oder [(C₃-C₆)Cycloalkyl]-carbonyl, [(C₂-C₆)Alkenyl]-carbonyl oder [(C₂-C₆)Alkinyl]-carbonyl, wobei jeder der letztgenannten drei Reste unsubstituiert oder durch ein oder mehrere Halogenatome substituiert ist, oder eine Gruppe der Formel 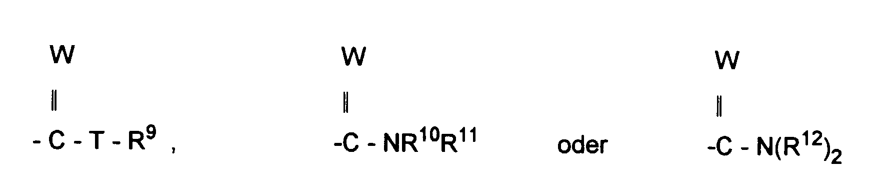 bedeuten,
wobei im Falle, daß der Rest -CR²R³-NR⁴R⁵ in Parastellung zur SO₂-Gruppe des Sulfonylharnstoffmoleküls steht, die Bedeutung R⁵ = unsubstituiertes [(C₁-C₆)Alkyl]-carbonyl ausgenommen ist, oder
R⁴ und R⁵ gemeinsam eine Kette der Formel (-CH₂)ₘ B- oder -B¹-(CH₂)ₘ₁-B-, wobei die Kette unsubstituiert oder durch einen oder mehrere (C₁-C₃)Alkylreste substituiert ist und m 3 oder 4 bzw. ml = 2 oder 3 sind, bedeuten sowie
W ein Sauerstoff- oder Schwefelatom,
B, B¹ unabhängig voneinander SO₂ oder CO,
Q O, S oder -NR¹³-,
T O oder S,
R⁶ H, (C₁-C₄)Alkyl, (C₁-C₄)Alkoxy, [(C₁-C₄)Alkyl]-carbonyl oder [(C₁-C₄)Alkoxy]-carbonyl, wobei jeder der letztgenannten 4 Reste unsubstituiert oder im Alkylteil durch ein oder mehrere Halogenatome substituiert ist, oder Halogen, NO₂ oder CN,
R⁷ H oder CH₃,
R⁸ H, (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, [(C₁-C₄)Alkyl]-carbonyl und [(C₁-C₄)Alkoxy]-carbonyl substituiert ist,
R⁹ (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, [(C₁-C₄)Alkyl]-carbonyl und [(C₁-C₄)Alkoxy]-carbonyl substituiert ist,
R¹⁰, R¹¹ unabhängig voneinander H, (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy, (C₁-C₄)Alkylthio, [(C₁-C₄)Alkyl]-carbonyl und [(C₁-C₄)Alkoxy]-carbonyl substituiert ist,
die Reste R¹² gemeinsam mit dem N-Atom einen heterocyclischen Ring mit 5 oder 6 Ringgliedern, der weitere Heteroatome aus der Gruppe N, O und S in den möglichen Oxidationsstufen enthalten kann und unsubstituiert oder durch (C₁-C₄)Alkyl oder die Oxogruppe substituiert ist oder benzokondensiert ist,
R¹³ H, (C₁-C₄)Alkyl, (C₃-C₄)Alkenyl oder (C₃-C₄)Alkinyl, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist,
A einen Rest der Formel 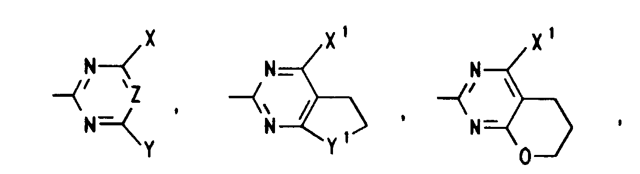 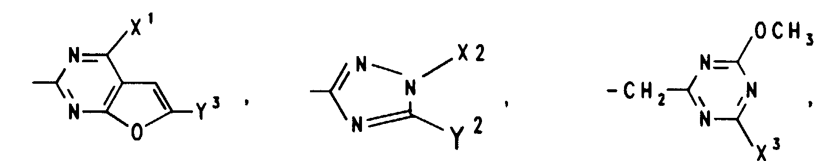 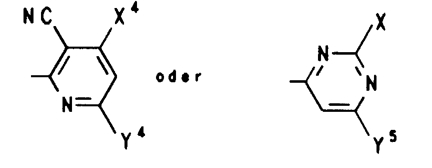
einer der Reste X und Y Wasserstoff, Halogen, (C₁-C₃)Alkyl oder (C₁-C₃)Alkoxy, wobei die beiden letztgenannten Reste unsubstituiert oder ein- oder mehrfach durch Halogen oder einfach durch (C₁-C₃)Alkoxy substituiert sind, und
der andere der Reste X und Y Wasserstoff, (C₁-C₃)Alkyl, (C₁-C₃)Alkoxy oder (C₁-C₃)Alkylthio, wobei die drei letztgenannten alkylhaltigen Reste unsubstituiert oder ein- oder mehrfach durch Halogen oder ein- oder zweifach durch (C₁-C₃)Alkoxy oder (C₁-C₃)Alkylthio substituiert sind, oder einen Rest der Formel NR¹⁴R¹⁵, (C₃-C₆)-Cycloalkyl, (C₂-C₄)Alkenyl, (C₂-C₄)Alkinyl, (C₃-C₄)-Alkenyloxy oder (C₃-C₄)Alkinyloxy,
Z CH oder N,
R¹⁴ und R¹⁵ unabhängig voneinander H, (C₁-C₃)Alkyl oder (C₃-C₄)Alkenyl,
X¹ CH₃, OCH₃, OC₂H₅ oder OCHF₂,
Y¹ -O- oder -CH₂- ,
X² CH₃, C₂H₅ oder CH₂CF₃ ,
Y² OCH₃, OC₂H₅, SCH₃, SCH₂CH₃, CH₃ oder C₂H₅,
X³ CH₃ oder OCH₃,
Y³ H oder CH₃,
X⁴ CH₃, OCH₃, OC₂H₅, CH₂OCH₃ oder Cl,
Y⁴_{'} CH₃, OCH₃, OC₂H₅ oder Cl und
Y⁵ CH₃, C₂H₅, OCH₃ oder Cl
bedeuten.

2. Verbindungen und deren Salze nach Anspruch 1, dadurch gekennzeichnet, daß
R⁴ H, (C₁-C₄)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]-carbonyl und CN substituiert ist, oder (C₃-C₄)Alkenyl, (C₃-C₄)Alkinyl, Hydroxy, (C₁-C₄)Alkoxy, [(C₁ -C₄)Alkyl]-carbonyl oder (C₁-C₄)Alkylsulfonyl, wobei jeder der letztgenannten fünf Reste unsubstituiert oder im Alkylteil durch ein oder mehrere Halogenatome substituiert ist, oder Phenylsulfonyl, wobei der Phenylrest unsubstituiert oder durch einen oder mehrere Reste aus der Gruppe Halogen, (C₁-C₄)Alkyl und (C₁-C₄)Alkoxy substituiert ist, und
R⁵ (C₁-C₄)Alkylsulfonyl, (C₁-C₄)Haloalkylsulfonyl, Phenylsulfonyl oder Phenylcarbonyl, wobei der Phenylrest in den beiden letztgenannten Resten unsubstituiert oder durch einen oder mehrere Reste aus der Gruppe Halogen, (C₁-C₄)Alkyl und (C₁-C₄)Alkoxy substituiert ist, oder Mono- oder Di-[(C₁-C₄)Alkyl]-aminosulfonyl, [(C₁-C₆)Alkyl]-carbonyl, das unsubstituiert oder durch ein oder mehrere Halogenatome oder durch (C₁-C₄)Alkoxy oder (C₁-C₄)Alkylthio substituiert ist, oder Formyl, OHC-CO-, 2-Oxo-(C₃-C₅)alkanoyl, [(C₁-C₄)Alkoxy]-oxalyl, [(C₃-C₆)Cycloalkyl]-carbonyl, [(C₂-C₄)Alkenyll-carbonyl oder [(C₂-C₄)Alkinyl]-carbonyl oder eine Gruppe der Formel 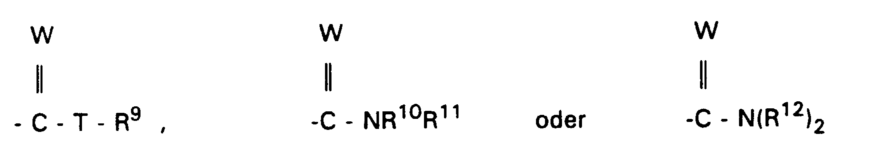
W O oder S,
R⁶ H, (C₁-C₄)Alkyl, (C₁-C₄)Haloalkyl, (C₁-C₄)Alkoxy, (C₁-C₄)-Haloalkoxy oder Halogen,
R⁸ unabhängig voneinander (C₁ -C₄)Alkyl, das unsubstituiert oder durch ein oder mehrere Halogenatome substituiert ist, oder (C₃-C₄)-Alkenyl oder (C₃-C₄)Alkinyl,
R⁹ H, (C₁-C₄)Alkyl, das unsubstituiert oder durch ein oder mehrere Halogenatome oder durch (C₁-C₄)Alkoxy, (C₁-C₄)Alkylthio, [(C₁-C₄)Alkoxy]-carbonyl und [(C₁-C R¹⁰, R¹¹ 4)Alkyl]-carbonyl substituiert ist,
R¹⁰, R¹¹ unabhängig voneinander H, (C₁-C₄)Alkyl, das unsubstituiert oder durch ein oder mehrere Halogenatome substituiert ist, oder (C₃-C₄)-Alkenyl oder (C₃-C₄)Alkinyl, wobei mindestens einer der Reste R¹⁰ und R¹¹ von Wasserstoff verschieden ist,
die Reste R¹² gemeinsam mit dem N-Atom einen heterocyclischen Ring mit 5 oder 6 Ringgliedern, der ein weiteres Heteroatom aus der Gruppe N, O und S in den verschiedenen Oxidationsstufen enthalten kann und unsubstituiert oder durch (C₁-C₄)Alkyl oder die Oxogruppe substituiert ist, und
R¹³ unabhängig voneinander H, (C₁-C₄)Alkyl, das unsubstituiert oder durch ein oder mehrere Halogenatome substituiert ist, oder (C₃-C₄)-Alkenyl oder (C₃-C₄)Alkinyl
bedeuten.

3. Verbindungen und deren Salze nach Anspruch 1 oder 2, dadurch gekennzeichnet, daß
R⁴ H, (C₁-C₄)Alkyl, Hydroxy oder (C₁-C₄)Alkoxy,
R⁵ (C₁-C₄)Alkylsulfonyl, CHO, [(C₁-C₄)Alkyl]-carbonyl, das unsubstituiert oder durch ein oder mehrere Halogenatome substituiert ist, [(C₁-C₄)Alkoxy]-oxalyl, [(C₃-C₆)cycloalkyl]-carbonyl oder eine Gruppe der Formel 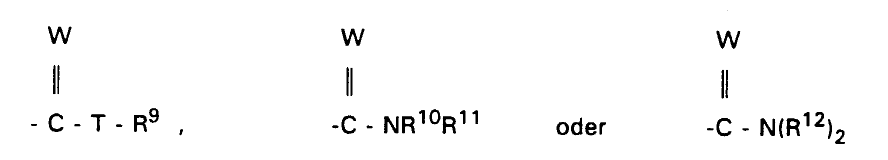 wobei
W, T, R⁹ bis R¹² wie oben in Formel (I) definiert sind,
R⁶ H, (C₁-C₃)Alkyl, (C₁-C₃)Alkoxy, Halogen,
A einen Rest der Formel 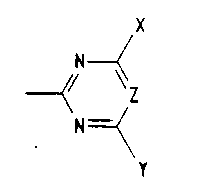
Z CH oder N und
einer der Reste X und Y Halogen, (C₁-C₂)Alkyl, (C₁-C₂)Alkoxy, OCF₂H, CF₃ oder OCH₂CF₃ und der andere der Reste X und Y (C₁-C₂)Alkyl, (C₁-C₂)Alkoxy oder (C₁-C₂)Haloalkoxy
bedeuten.

4. Verfahren zur Herstellung von Verbindungen der Formel (I) oder deren Salze gemäß Anspruch 1, dadurch gekennzeichnet, daß man
a) eine Verbindung der Formel (II) 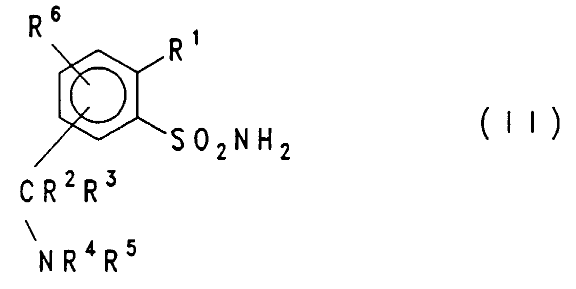 mit einem heterocyclischen Carbamat der Formel (III),
R* - O - CO - NR⁷ - A (III)
worin R^{*} gegebenenfalls substituiertes Phenyl oder (C₁-C₄)Alkyl bedeutet, umsetzt oder
b) ein Phenylsulfonylcarbamat der Formel (IV) 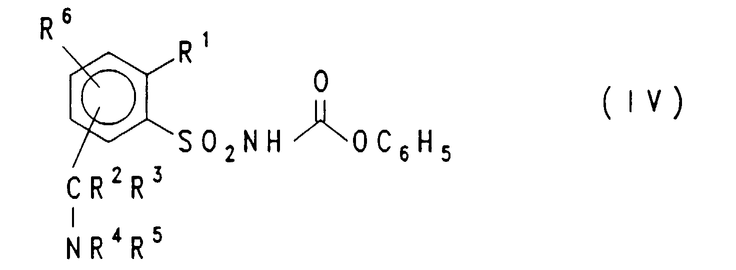 mit einem Aminoheterocyclus der Formel (V)
H - NR⁷ - A (V)
umsetzt oder
c) ein Sulfonylisocyanat der Formel (Vl) 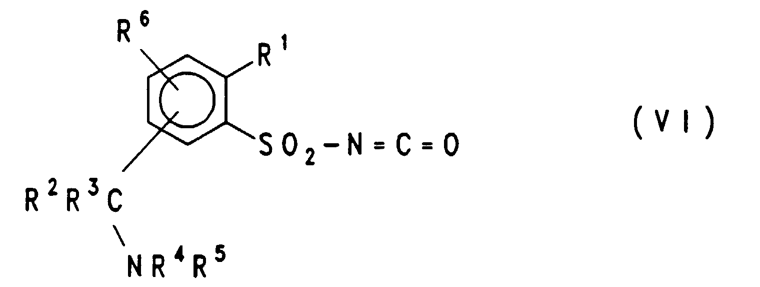 mit einem Aminoheterocyclus der Formel H-NR⁷-A (V) umsetzt oder
d) in einer Eintopfreaktion zunächst einen Aminoheterocyclus der Formel H-NR⁷-A (V) in Gegenwart einer Base mit Phosgen umsetzt und das gebildete Intermediat mit einem Phenylsulfonamid der Formel (II) umsetzt oder
e) ein Sulfonylchlorid der Formel (VII) 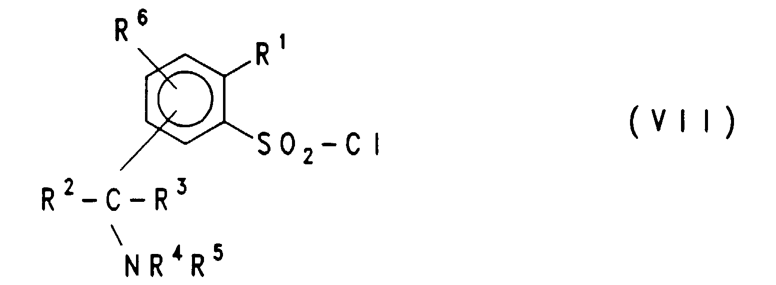 mit einem Cyanat M-OCN, worin M = NH₄, Na oder K bedeutet, und mit einem Aminoheterocyclus der Formel H-NR⁷-A (V) in Gegenwart einer Base umsetzt,
f) ein Sulfonamid der genannten Formel (II) mit einem (Thio-)lsocyanat der Formel (V')
W = C = N - A (V')
in Gegenwart einer Base umsetzt,
wobei in den Formeln (II)-(VII) und (V') die Reste bzw. Gruppen R¹-R⁷, A oder W wie in Formel (I) definiert sind und wobei in den Varianten a)-e) zunächst Verbindungen der Formel (I) mit W = O erhalten werden.

5. Herbizides oder pflanzenwachstumsregulierende Mittel, dadurch gekennzeichnet, daß es mindestens eine Verbindung der Formel (I) oder deren Salz nach einem der Ansprüche 1 bis 3 und im Pflanzenschutz übliche Formulierungshilfsmittel enthält.

6. Verfahren zur Bekämpfung von Schadpflanzen oder zur Wachstumsregulierung von Pflanzen, dadurch gekennzeichnet, daß man eine wirksame Menge von mindestens einer Verbindung der Formel (I) oder eines deren Salze nach einem der Ansprüche 1 bis 3 auf die Schadpflanzen bzw. Pflanzen, deren Pflanzensamen oder die Fläche, auf der sie wachsen, appliziert.

7. Verwendung der Verbindungen der Formel (I) oder deren Salze nach einem der Ansprüche 1 bis 3 als Herbizide oder Pflanzenwachstumsregulatoren.

8. Verbindungen der Formel (II), 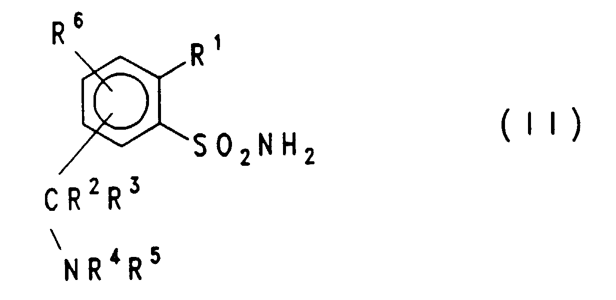 worin R¹ bis R⁶ wie in Formel (I) nach Anspruch 1 definiert sind.

9. Verfahren zur Herstellung von Verbindungen der Formel (II) nach Anspruch 8, dadurch gekennzeichnet, daß man eine Verbindung der Formel (XV) 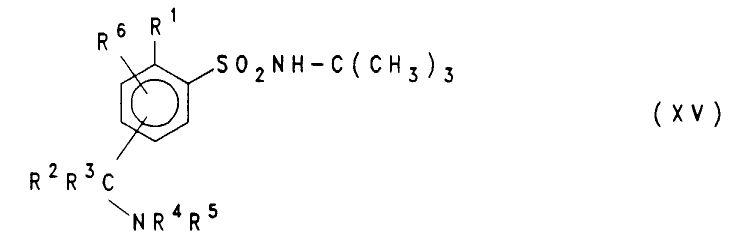 analog bekannter Methode zur Abspaltung der tert.-Butylschutzgruppe umsetzt, wobei in Formel (XV) die Reste R¹ bis R⁶ wie in Formel (II) definiert sind.

## Claims

1. A compound of the formula (I) or salt thereof, 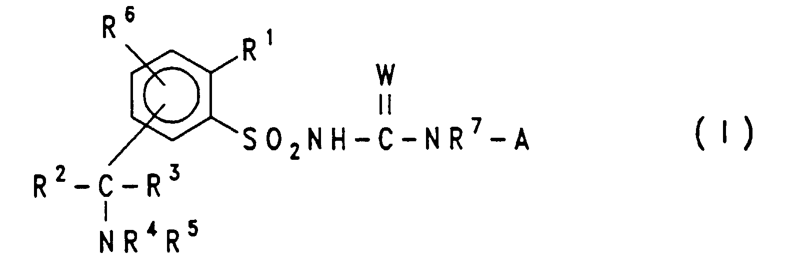 in which
R¹ is CO-Q-R⁸,
R² and R³ independently of one another are H or (C₁-C₄)alkyl,
R⁴ is H, (C₁-C₈)alkyl which is unsubstituted or is substituted by one or more radicals from the group consisting of halogen, (C₁-C₄)alkoxy, (C₁-C₄)alkylthio, (C₁-C₄)alkylsulfinyl, (C₁-C₄)alkylsulfonyl, [(C₁-C₄)alkoxy] carbonyl and CN, or is (C₃-C₆)alkenyl which is unsubstituted or is substituted by one or more halogen atoms, or is (C₃-C₆)alkynyl which is unsubstituted or is substituted by one or more halogen atoms, or is hydroxyl, (C₁-C₄)alkoxy, [(C₁-C₄)alkyl] carbonyl or (C₁-C₄)alkylsulfonyl, each of the latter three radicals being unsubstituted or substituted in the alkyl moiety by one or more halogen atoms or by (C₁-C₄)alkoxy or (C₁-C₄)alkylthio, or is phenylsulfonyl in which the phenyl radical is unsubstituted or substituted, and
R⁵ is (C₁-C₄)alkylsulfonyl or (C₃-C₆)alkenyl-sulfonyl, each of the two latter radicals being unsubstituted or substituted by one or more halogen atoms or by (C₁-C₄)alkoxy or (C₁-C₄)alkylthio, or is phenylsulfonyl or phenylcarbonyl, the phenyl radical in each of the two latter radicals being unsubstituted or substituted by one or more radicals from the group consisting of halogen, (C₁-C₄)alkyl, (C₁-C₄)haloalkyl,(C₁-C₄)alkoxy, (C₁-C₄)haloalkoxy, hydroxyl, amino, nitro, cyano, [(C₁-C₄)alkoxy] carbonyl, [(C₁-C₄)alkyl]-carbonyl, formyl, carbamoyl, mono- and di-[(C₁-C₄)alkyl] aminocarbonyl, mono- and di-[(C₁-C₄)alkyl] amino, (C₁-C₄)alkylsulfinyl and (C₁-C₄)alkylsulfonyl, or is mono- or di-[(C₁-C₄)alkyl]aminosulfonyl or [(C₁-C₆)alkyl] carbonyl, each of the three latter radicals being unsubstituted or substituted by one or more radicals from the group consisting of halogen, (C₁-C₄)alkoxy, (C₁-C₄)alkylthio, (C₁-C₄)alkylsulfinyl, (C₁-C₄)-alkylsulfonyl, [(C₁-C₄)alkyl] carbonyl, [(C₁-C₄)alkoxy] carbonyl and CN, or is formyl, a group of the formula -CO-CO-R' in which R' = H, OH, (C₁-C₄)alkoxy or (C₁-C₄)alkyl, or is [(C₃-C₆) cycloalkyl] carbonyl, [(C₂-C₆)alkenyl]-carbonyl or [(C₂-C₆)alkynyl] carbonyl, each of the latter three radicals being unsubstituted or substituted by one or more halogen atoms, or is a group of the formula 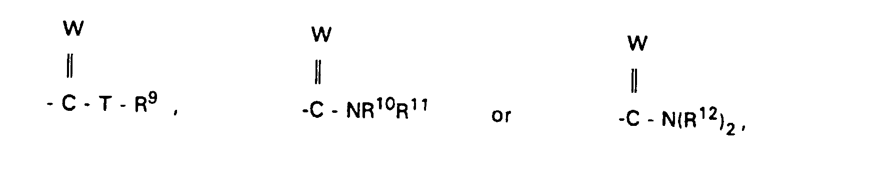 where, in the case that the radical -CR²R³-NR⁴R⁵ is in the para position to the SO₂ group of the sulfonylurea molecule, the meaning R⁵ = unsubstituted [(C₁-C₆)alkyl] carbonyl is excluded, or
R⁴ and R⁵ together are a chain of the formula (-CH₂)ₘ B- or -B¹-(CH₂)ₘ₁-B-, the chain being unsubstituted or substituted by one or more (C₁-C₃)alkyl radicals and m being 3 or 4 or ml being 2 or 3, and
W is an oxygen or sulfur atom,
B and B¹ independently of one another are SO₂ or CO,
Q is O, S or -NR¹³-,
T is O or S,
R⁶ is H, (C₁-C₄)alkyl, (C₁-C₄)alkoxy, [(C₁-C₄)alkyl] carbonyl or [(C₁-C₄)alkoxyl carbonyl, each of the latter 4 radicals being unsubstituted or substituted in the alkyl moiety by one or more halogen atoms, or is halogen, NO₂ or CN,
R⁷ is H or CH₃,
R⁸ is H, (C₁-C₄)alkyl, (C₃-C₄)alkenyl or (C₃-C₄)alkynyl, each of the three latter radicals being unsubstituted or substituted by one or more radicals from the group consisting of halogen, (C₁-C₄)alkoxy, (C₁-C₄)alkylthio, [(C₁-C₄)alkyl] carbonyl and [(C₁-C₄)alkoxyl-carbonyl,
R⁹ is (C₁-C₄)alkyl, (C₃-C₄)alkenyl or (C₃-C₄)alkynyl, each of the three latter radicals being unsubstituted or substituted by one or more radicals from the group consisting of halogen, (C₁-C₄)alkoxy, (C₁-C₄)alkylthio, [(C₁-C₄)alkyl] carbonyl and [(C₁-C₄)alkoxy]-carbonyl,
R¹⁰ and R¹¹ independently of one another are H, (C₁-C₄)alkyl, (C₃-C₄)alkenyl or (C₃-C₄)alkynyl, each of the three latter radicals being unsubstituted or substituted by one or more radicals from the group consisting of halogen, (C₁-C₄)alkoxy, (C₁-C₄)alkylthio, [(C₁-C₄)alkyl] carbonyl and [(C₁-C₄)alkoxy] carbonyl,
the radicals R¹² together with the nitrogen atom are a heterocyclic ring having 5 or 6 ring members, which may contain further heteroatoms from the group consisting of N, O and S in the possible oxidation states and is unsubstituted or is substituted by (C₁-C₄)alkyl or the oxo group, or is benzo-fused,
R¹³ is H, (C₁-C₄)alkyl, (C₃-C₄)alkenyl or (C₃-C₄)alkynyl, each of the three latter radicals being unsubstituted or substituted by one or more radicals from the group consisting of halogen, (C₁-C₄)alkoxy and (C₁-C₄)alkylthio,
A is a radical of the formula 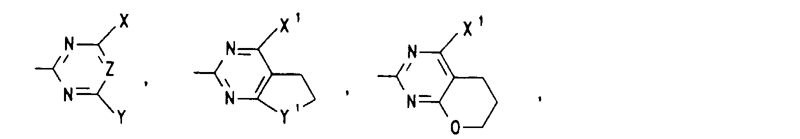 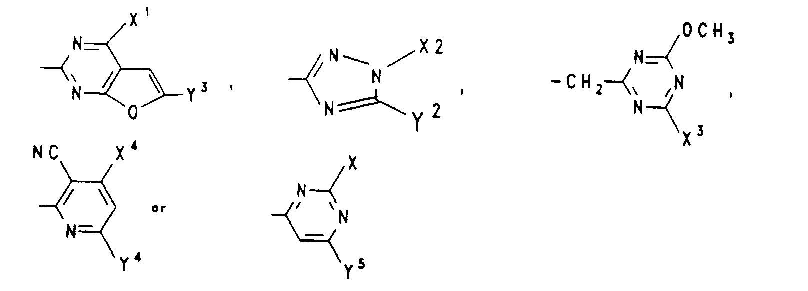
one of the radicals X and Y is hydrogen, halogen, (C₁-C₃)alkyl or (C₁-C₃)alkoxy, the two latter radicals being unsubstituted or mono- or polysubstituted by halogen or monosubstituted by (C₁-C₃)alkoxy, and
the other of the radicals X and Y is hydrogen, (C₁-C₃)alkyl, (C₁-C₃)alkoxy or (C₁-C₃)alkylthio, the three latter alkyl-containing radicals being unsubstituted or mono- or polysubstituted by halogen or mono- or disubstituted by (C₁-C₃)alkoxy or (C₁-C₃)alkylthio, or is a radical of the formula NR¹⁴R¹⁵, (C₃-C₆)cycloalkyl, (C₂-C₄)alkenyl, (C₂-C₄)alkynyl, (C₃-C₄)alkenyloxy or (C₃-C₄)alkynyloxy,
Z is CH or N,
R¹⁴ and R¹⁵ independently of one another are H, (C₁-C₃)alkyl or (C₃-C₄)alkenyl,
X¹ is CH₃, OCH₃, OC₂H₅ or OCHF₂,
Y¹ is -O- or -CH₂-,
X² is CH₃, C₂H₅ or CH₂CF₃,
Y² is OCH₃, OC₂H₅, SCH₃, SCH₂CH₃, CH₃ or C₂H₅,
X³ is CH₃ or OCH₃,
Y³ is H or CH₃,
X⁴ is CH₃, OCH₃, OC₂H₅, CH₂OCH₃ or Cl,
Y⁴ is CH₃, OCH₃, OC₂H₅ or Cl, and
Y⁵ is CH₃, C₂H₅, OCH₃ or Cl.

2. A compound or salt thereof as claimed in claim 1, wherein
R⁴ is H, (C₁-C₄)alkyl which is unsubstituted or is substituted by one or more radicals from the group consisting of halogen, (C₁-C₄)alkoxy, (C₁- C₄)alkylthio, (C₁-C₄)alkylsulfonyl, [(C₁-C₄)-alkoxy] carbonyl and CN, or is (C₃-C₄)alkenyl, (C₃-C₄)alkynyl, hydroxyl, (C₁-C₄)alkoxy, [(C₁-C₄)alkyl] carbonyl or (C₁-C₄)alkylsulfonyl, each of the latter five radicals being unsubstituted or substituted in the alkyl moiety by one or more halogen atoms, or is phenylsulfonyl in which the phenyl radical is unsubstituted or is substituted by one or more radicals from the group consisting of halogen, (C₁-C₄)alkyl and (C₁-C₄)alkoxy, and
R⁵ is (C₁-C₄)alkylsulfonyl, (C₁-C₄)haloalkyl-sulfonyl, phenylsulfonyl or phenylcarbonyl, the phenyl radical in the two latter radicals being unsubstituted or substituted by one or more radicals from the group consisting of halogen, (C₁-C₄)alkyl and (C₁-C₄)alkoxy, or is mono- or di- [(C₁-C₄)alkyl] aminosulfonyl, [(C₁-C₆)alkyl]-carbonyl which is unsubstituted or is substituted by one or more halogen atoms or by (C₁-C₄)alkoxy or (C₁-C₄)alkylthio, or is formyl, OHC-CO-, 2-oxo-(C₃-C₅)alkanoyl, [(C₁-C₄)alkoxyl oxalyl, [(C₃-C₆)cycloalkyl]-carbonyl, [(C₂-C₄)alkenyl] carbonyl or [(C₂-C₄)alkynyl]carbonyl or is a group of the formula 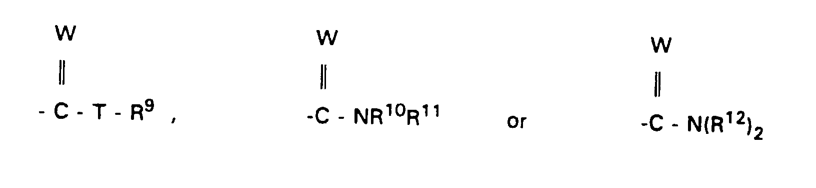
W is O or S,
R⁶ is H, (C₁-C₄)alkyl, (C₁-C₄)haloalkyl, (C₁-C₄)alkoxy, (C₁-C₄)haloalkoxy or halogen,
R⁸ is at each occurrence (C₁-C₄)alkyl which is unsubstituted or is substituted by one or more halogen atoms, or is (C₃-C₄)alkenyl or (C₃-C₄)alkynyl,
R⁹ is H, (C₁-C₄)alkyl which is unsubstituted or is substituted by one or more halogen atoms or by (C₁-C₄)alkoxy, (C₁-C₄)alkylthio, [(C₁-C₄)-alkoxy] carbonyl and [(C₁-C₄)alkyl] carbonyl,
R¹⁰ and R¹¹ independently of one another are H, (C₁-C₄)alkyl which is unsubstituted or is substituted by one or more halogen atoms, or are (C₃-C₄)alkenyl or (C₃-C₄)alkynyl, at least one of the radicals R¹⁰ and R¹¹ being different from hydrogen,
the radicals R¹² together with the nitrogen atom are a heterocyclic ring having 5 or 6 ring members, which may contain a further heteroatom from the group consisting of N, O and S in the various oxidation states and is unsubstituted or is substituted by (C₁-C₄)alkyl or the oxo group, and
R¹³ is at each occurrence H, (C₁-C₄)alkyl which is unsubstituted or is substituted by one or more halogen atoms, or is (C₃-C₄)alkenyl or (C₃-C₄)alkynyl.

3. A compound or salt thereof as claimed in claim 1 or 2, wherein
R⁴ is H, (C₁-C₄)alkyl, hydroxyl or (C₁-C₄)alkoxy,
R⁵ is (C₁-C₄)alkylsulfonyl, CHO, [(C₁-C₄)alkyl]-carbonyl which is unsubstituted or is substituted by one or more halogen atoms, or is [(C₁-C₄)alkoxy] oxalyl, [(C₃-C₆)cycloalkyl]-carbonyl or a group of the formula 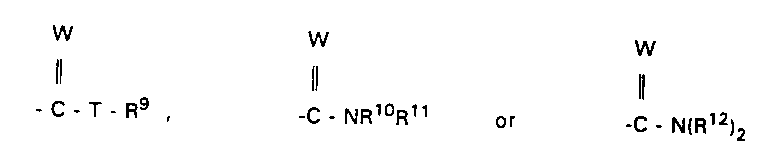 in which
W, T and R⁹ to R¹² are as defined above for formula (I),
R⁶ is H, (C₁-C₃)alkyl, (C₁-C₃)alkoxy or halogen,
A is a radical of the formula 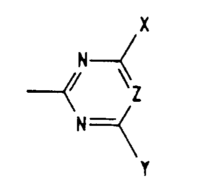
Z is CH or N, and
one of the radicals X and Y is halogen, (C₁-C₂)alkyl, (C₁-C₂)alkoxy, OCF₂H, CF₃ or OCH₂CF₃ and the other of the radicals X and Y is (C₁-C₂)alkyl, (C₁-C₂)alkoxy or (C₁-C₂)haloalkoxy.

4. A process for the preparation of a compound of the formula (I) or salt thereof as claimed in claim 1, which comprises
a) reacting a compound of the formula (II) 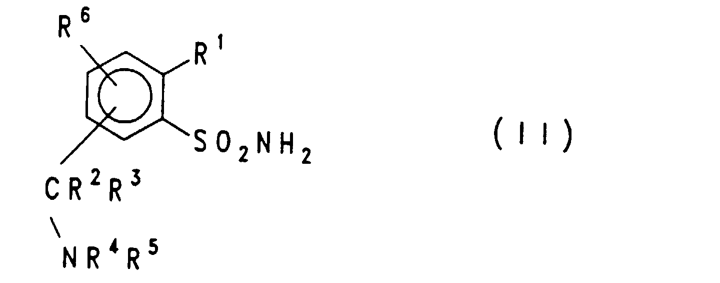 with a heterocyclic carbamate of the formula (III)
R* - O - CO - NR⁷ - A (III)
in which R* is unsubstituted or substituted phenyl or (C₁-C₄)alkyl, or
b) reacting a phenyl sulfonylcarbamate of the formula (IV) 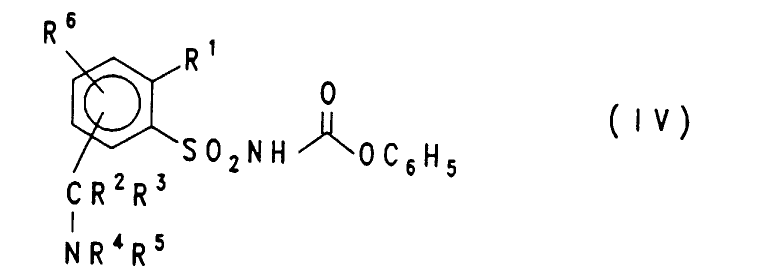 with an amino heterocycle of the formula (V)
H - NR⁷ - A (V)
or
c) reacting a sulfonyl isocyanate of the formula (VI) 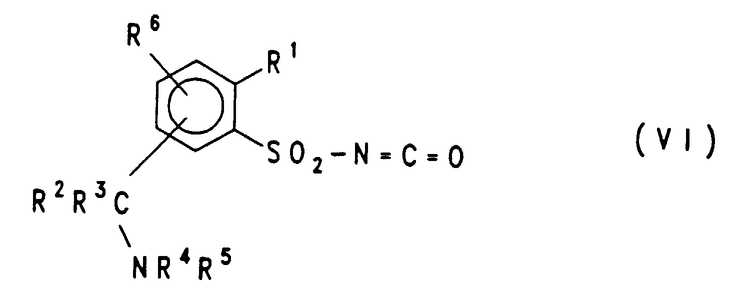 with an amino heterocycle of the formula H-NR⁷-A (V), or
d) in a one-pot reaction, first reacting an amino heterocycle of the formula H-NR⁷-A (V) with phosgene in the presence of a base and reacting the intermediate formed with a phenyl sulfonamide of the formula (II), or
e) reacting a sulfonyl chloride of the formula (VII) 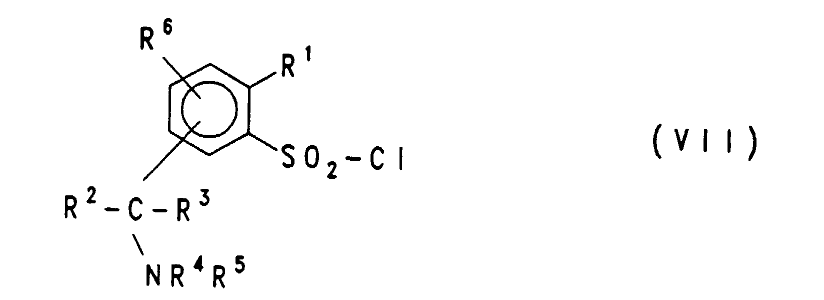 with a cyanate M-OCN in which M = NH₄, Na or K, and with an amino heterocycle of the formula H-NR⁷-A (V) in the presence of a base, or
f) reacting a sulfonamide of the abovementioned formula (II) with a (thio)isocyanate of the formula (V')
W = C = N - A (V')
in the presence of a base,
the radicals and groups R¹ to R⁷, A and W in the formulae (II)-(VII) and (V') being as defined for formula (I) and, in variants a)-e), compounds of the formula (I) where W = O being obtained initially.

5. A herbicidal or plant growth-regulating composition, which comprises at least one compound of the formula (I) or salt thereof as claimed in any one of claims 1 to 3 and formulation auxiliaries which are conventional in crop protection.

6. A method for controlling nuisance plants or for regulating the growth of plants, which comprises applying an effective quantity of at least one compound of the formula (I) or of a salt thereof as claimed in any one of claims 1 to 3 to the nuisance plants or plants, their plant seeds or the area on which they are growing.

7. The use of a compound of the formula (I) or salt thereof as claimed in any one of claims 1 to 3 as a herbicide or plant growth regulator.

8. A compound of the formula (II), 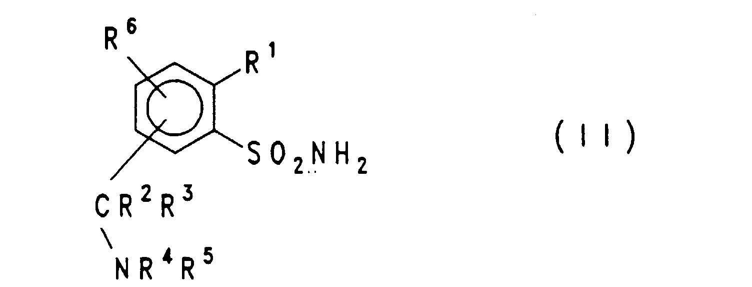 in which R¹ to R⁶ are as defined for formula (I) as claimed in claim 1.

9. A process for the preparation of a compound of the formula (II) as claimed in claim 8, which comprises reacting a compound of the formula (XV) 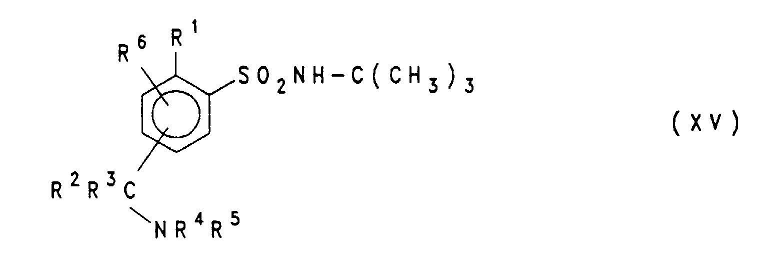 by analogy with a known method in order to eliminate the tert-butyl protecting group, the radicals R¹ to R⁶ in formula (XV) being as defined for formula (II).

## Revendications

1. Composés de formule (I) et leurs sels, 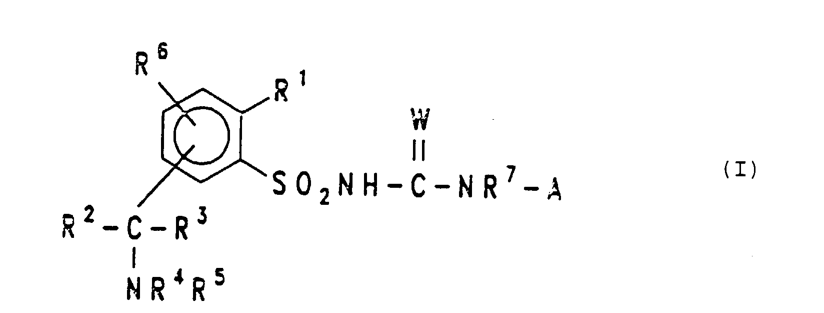 dans laquelle
R¹ CO-Q-R⁸,
R², R³, indépendamment l'un de l'autre, représentent un atome d'hydrogène ou un groupe alkyle en C₁-C₄,
R⁴ représente un atome d'hydrogène, un groupe alkyle en C₁-C₈, qui est non substitué ou substitué par un ou plusieurs restes pris dans le groupe comportant des atomes d'halogènes, des groupes alcoxy en C₁-C₄, (alkyl en C₁-C₄)thio, (alkyl en C₁-C₄)sulfinyle, (alkyl en C₁-C₄)-sulfonyle, [(alcoxy en C₁-C₄)]-carbonyle et CN, ou représente alcényle en C₃-C₆, qui est non substitué ou substitué par un ou plusieurs atomes d'halogènes ou représente alcynyle en C₃-C₆, qui est non substitué ou substitué par un ou plusieurs atomes d'halogène, ou représente des groupes hydroxy, alcoxy en C₁-C₄, (alkyl en C₁-C₄)-carbonyle ou (alkyl en C₁-C₄)sulfonyle, les trois derniers restes cités pouvant être non substitués ou substitués dans la partie alkyle par un ou plusieurs atomes d'halogènes ou par des groupes alcoxy en C₁-C₄ ou (alkyle en C₁- C₄)-thio, ou représente phénylsulfonyle, le reste phényle étant non substitué ou non substitué, et
R⁵ représente (alkyl en C₁-C₄)sulfonyle ou (alcényle en C₃-C₆)sulfonyle, chacun des deux derniers restes cités pouvant être non substitué ou substitué par un ou plusieurs atomes d'halogènes ou par des groupes alcoxy en C₁-C₄ ou (alkyl en C₁-C₄)thio, ou représente phénylsulfonyle ou phénylcarbonyle, le reste phényle dans chacun des deux derniers restes cités pouvant être non substitué ou substitué par un ou plusieurs restes pris dans le groupe comportant des atomes d'halogènes, des groupes alkyle en C₁-C₄, haloalkyle en C₁-C₄, alcoxy en C₁-C₄, haloalcoxy en C₁-C₄, hydroxy, amino, nitro, cyano, (alcoxy en C₁-C₄)-carbonyle, (alkyl en C₁-C₄)-carbonyle, formyle, carbamoyle, mono- et di-(alkyl en C₁-C₄)-aminocarbonyle, mono- et di(alkyl en C₁-C₄)amino, (alkyl en C₁-C₄)sulfynyle et (alkyl en C₁-C₄)--sulfonyle, ou représente des groupes mono- ou di(alkyl en C₁-C₄)aminosulfonyle ou (alkyl en C₁-C₆)-carbonyle, chacun des trois derniers restes cités pouvant être non substitué ou substitué par un ou plusieurs restes pris dans le groupe comportant des atomes d'halogènes, des groupes alcoxy en C₁-C₄, (alkyl en C₁-C₄)thio, (alkyl en C₁-C₄)-sulfinyle, (alkyl en C₁-C₄)sulfonyle, (alkyl en C₁-C₄)carbonyle, (alcoxy en C₁-C₄)carbonyle et CN, ou représente un groupe formyle, un groupe de formule -CO-CO-R', dans laquelle R' = H, OH, un groupe alcoxy en C₁-C₄ ou alkyle en C₁-C₄ ou représente (cycloalkyl en C₃-C₆)-carbonyle, (alcényl en C₂-C₆)carbonyle ou (alcynyl en C₂-C₆)carbonyle, chacun des trois derniers restes cités pouvant être non substitué ou substitué par un ou plusieurs atomes d'halogènes, ou représente un groupe de formule 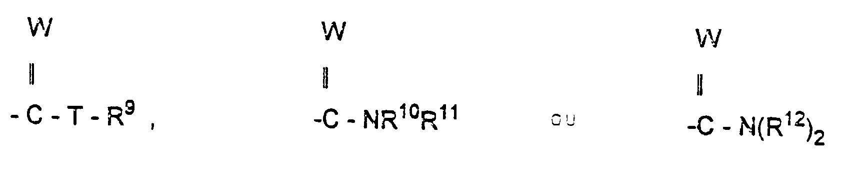 dans le cas où le reste -CR²R³-NR⁴R⁵ se trouve en position para par rapport au groupe SO₂ dans la molécule sulfonylurée, la signification de R⁵ = (alkyl en C₁-C₆)carbonyle non substitué étant exclue, ou
R⁴ et R⁵ ensemble représentent une chaîne de formule (-CH₂)ₘB- ou -B¹-(CH₂)ₘ₁-B-, la chaîne pouvant être non substituée ou substituée par un ou plusieurs restes alkyle en C₁-C₃ et m = 3 ou 4 ou bien m1 = 2 ou 3, ainsi que
W représente un atome d'oxygène ou de soufre,
B, B¹, indépendamment l'un de l'autre, représentent SO₂ ou CO,
Q représente O, S ou -NR¹³-,
T représente O ou S,
R⁶ représente un atome d'hydrogène, des groupes alkyle en C₁-C₄, alcoxy en C₁-C₄, (alkyl en C₁-C₄)-carbonyle ou (alcoxy en C₁-C₄)carbonyle, chacun des quatre derniers restes cités pouvant être non substitué ou substitué dans la partie alkyle par un ou plusieurs atomes d'halogènes, ou représente un atome halogène, des groupes NO₂ ou CN,
R⁷ représente H ou CH₃,
R⁸ représente un atome d'hydrogène, des groupes alkyle en C₁-C₄, alcényle en C₃-C₄ ou alcynyle en C₃-C₄, chacun des trois derniers restes cités pouvant être non substitué ou substitué par un ou plusieurs restes pris dans le groupe comportant des atomes d'halogènes, des groupes alcoxy en C₁-C₄, (alkyl en C₁-C₄)thio, (alkyl en C₁-C₄)carbonyle et (alcoxy en C₁-C₄)-carbonyle,
R⁹ représente des groupes alkyle en C₁-C₄, alcényle en C₃-C₄ ou alcynyle en C₃-C₄, les trois derniers restes cités pouvant être non substitués ou substitués par un ou plusieurs restes pris dans le groupe comportant des atomes d'halogènes, des groupes alcoxy en C₁-C₄, (alkyl en C₁-C₄)thio, (alkyl en C₁-C₄)carbonyle et (alcoxy en C₁-C₄)carbonyle,
R¹⁰, R¹¹, indépendamment l'un de l'autre, représentent un atome d'hydrogène, des groupes alkyle en C₁-C₄, alcényle en C₃-C₄ ou alcynyle en C₃-C₄, chacun des trois derniers restes cités pouvant être non substitué ou substitué par un ou plusieurs restes pris dans le groupe comportant des atomes d'halogène, des groupes alcoxy en C₁-C₄, (alkyl en C₁-C₄)thio, (alkyl en C₁-C₄)carbonyle et (alcoxy en C₁-C₄)carbonyle,
les restes R¹² ensemble avec l'atome d'azote forment un cycle hétérocyclique avec 5 à 6 chaînons, qui peut contenir d'autres hétéroatomes pris dans le groupe comportant des atomes d'azote, d'oxygène et de soufre dans les degrés d'oxydation possibles et pouvant être non substitué ou substitué par un groupe alkyle en C₁-C₄ ou le groupe oxo ou condensé sur un cycle benzène,
R¹³ représente un atome d'hydrogène, des groupes alkyle en C₁-C₄, alcényle en C₃-C₄ ou alcynyle en C₃-C₄, chacun des trois derniers restes cités pouvant être non substitué ou substitué par un ou plusieurs restes pris dans le groupe comportant des atomes d'halogène, des groupes alcoxy en C₁-C₄ ou alkylthio en C₁-C₄,
A représente un reste de formule 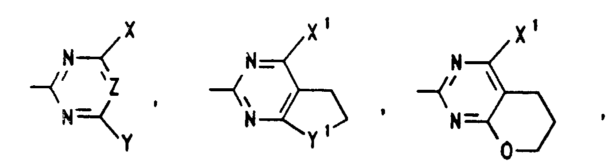 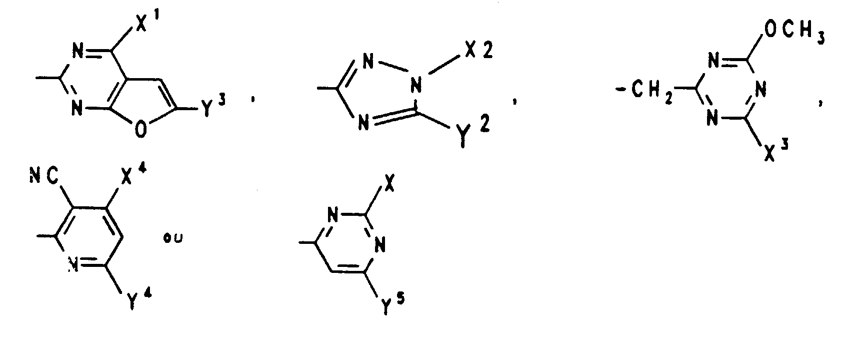
un des restes X et Y représente un atome d'hydrogène, un atome d'halogène, un groupe alkyle en C₁-C₃ ou alcoxy en C₁-C₃, les deux derniers restes cités pouvant être non substitués ou substitués une ou plusieurs fois par des atomes d'halogènes ou une fois par un groupe alcoxy en C₁-C₃,
et
l'autre des restes X et Y représente un atome d'hydrogène, des groupes alkyle en C₁-C₃, alcoxy en C₁-C₃ ou (alkyl en C₁-C₃)thio les trois derniers restes cités comportant des restes alkyle pouvant être non substitués ou substitués une ou plusieurs fois par des atomes d'halogènes ou une ou deux fois par des groupes alcoxy en C₁-C₃ ou (alkyl en C₁-C₃)thio ou représentent un reste de formule NR¹⁴R¹⁵, des groupes cycloalkyle en C₃-C₆, alcényle en C₂-C₄, alcynyle en C₂-C₄, alcényloxy en C₃-C₄ ou alcynyloxy en C₃-C₄,
Z représente CH ou N,
R¹⁴ et R¹⁵, indépendamment l'un de l'autre, représentent un atome d'hydrogène, un groupe alkyle en C₁-C₃ ou un groupe alcényle en C₃-C₄,
X¹ CH₃, OCH₃, OC₂H₅ ou OCHF₂,
Y¹ -O- ou -CH₂-,
X² CH₃, C₂H₅ ou CH₂CF₃,
Y² OCH₃, OC₂H₅, SCH₃, SCH₂CH₃, CH₃ ou C₂H₅,
X³ CH₃ ou OCH₃,
Y³ H ou CH₃,
X⁴ CH₃, OCH₃, OC₂H₅, CH₂OCH₃ ou Cl,
Y⁴ CH₃, OCH₃, OC₂H₅ ou Cl et
Y⁵ CH₃, C₂H₅, OCH₃ ou Cl.

2. Composés et leurs sels selon la revendication 1, caractérisés en ce que
R⁴ représente un atome d'hydrogène, un groupe alkyle en C₁-C₄, lequel est non substitué ou substitué par un ou plusieurs restes pris dans le groupe comportant des halogènes, des groupes alcoxy en C₁-C₄, (alkyl en C₁-C₄,)thio, (alkyl en C₁-C₄)sulfonyle, (alcoxy en C₁-C₄)-carbonyle et CN, ou représente alcényle en C₃-C₄, alcynyle en C₃-C₄, hydroxy, alcoxy en C₁-C₄, (alkyle en C₁-C₄)-carbonyle ou (alkyl en C₁-C₄)-sulfonyle, chacun des cinq derniers restes cités pouvant être non substitué ou substitué dans la partie alkyle par un ou plusieurs atomes d'halogènes, ou représente phénylsulfonyle, le reste phényle pouvant être non substitué ou substitué par un plusieurs restes pris dans le groupe comportant des atomes d'halogènes, des groupes alkyle en C₁-C₄ et alcoxy en C₁-C₄ et
R⁵ représente des groupes (alkyl en C₁-C₄)sulfonyle, halo(alkyl en C₁-C₄)sulfonyle, phénylsulfonyle ou phénylcarbonyle, le reste phényle dans les deux derniers restes cités pouvant être non substitué ou substitué par un ou plusieurs restes pris dans le groupe comportant des atomes halogènes, des groupes alkyle en C₁-C₄ et alcoxy en C₁-C₄, ou représente des groupes mono- ou di-(alkyl en C₁-C₄)aminosulfonyle, (alkyl en C₁-C₆)carbonyle, qui est non substitué ou substitué par un ou plusieurs atomes d'halogènes ou des groupes alcoxy en C₁-C₄ ou (alkyl en C₁-C₄)thio, ou représente des groupes formyle, OHC-CO, 2-oxo-(alcanoyl en C₃-C₅), (alcoxy en C₁-C₄) -oxalyle, (cycloalkyl en C₃-C₆)carbonyle, (alcényl en C₂-C₄)-carbonyle ou (alcynyl en C₂-C₄)carbonyle ou un groupe de formule 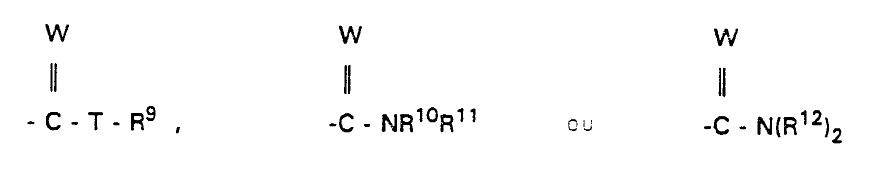
W représente O ou S,
R⁶ représente un atome d'hydrogène, un groupe alkyle en C₁-C₄, haloalkyle en C₁-C₄, alcoxy en C₁-C₄, haloalcoxy en C₁-C₄ ou des atomes d'halogène,
R⁸ représentent, indépendamment l'un de l'autre, des groupes alkyle en C₁-C₄, non substitués ou substitués par un ou plusieurs atomes d'halogène ou des groupes alcényle en C₃-C₄ ou alcynyle en C₃-C₄,
R⁹ représente un atome d'hydrogène, un groupe alkyle en C₁-C₄ qui est non substitué ou substitué par un ou plusieurs atomes d'halogènes ou par des groupes alcoxy en C₁-C₄, (alkyl en C₁-C₄)thio, (alcoxy en C₁-C₄)-carbonyle et (alkyl en C₁-C₄)carbonyle,
R¹⁰, R¹¹, indépendamment l'un de l'autre, représentent un atome d'hydrogène, un groupe alkyle en C₁-C₄ qui est non substitué ou substitué par un ou plusieurs atomes d'halogènes, ou représente des groupes alcényle en C₃-C₄ ou alcynyle en C₃-C₄, au moins un des restes R¹⁰ et R¹¹ étant différent de l'hydrogène,
les restes R¹² ensemble avec l'atome d'azote forment un cycle hétérocyclique à 5 ou 6 chaînons, qui peut porter un autre hétéroatome pris dans le groupe comportant N, O et S dans les différents degrés d'oxydation et qui peut être non substitué ou substitué par un groupe alkyle en C₁-C₄ ou le groupe oxo, et
R¹³, indépendamment l'un de l'autre, représentent un atome d'hydrogène, un groupe alkyle en C₁-C₄ qui est non substitué ou substitué par un ou plusieurs atomes d'halogènes, ou représente groupe alcényle en C₃-C₄ ou alcynyle en C₁-C₄.

3. Composés et leurs sels selon la revendication 1 ou 2, caractérisés en ce que
R⁴ représente un atome d'hydrogène, des groupes alkyle en C₁-C₄, hydroxy ou alcoxy en C₁-C₄,
R⁵ représente des groupes (alkyl en C₁-C₄)sulfonyle, CHO, (alkyl en C₁-C₄)carbonyle, qui est non substitué ou substitué par un ou plusieurs atomes d'halogène, ou représente des groupes (alcoxy en C₁-C₄)oxalyle, (cyclalkyl en C₃-C₆)carbonyle ou un groupe de formule 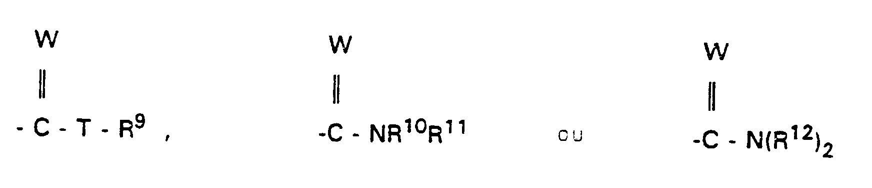 dans laquelle
W, T, R⁹ à R¹² ayant les définitions données dans la formule (I) ci-dessus,
R⁶ représente un atome d'hydrogène, un groupe alkyle en C₁-C₃, un groupe alcoxy en C₁-C₃, un atome d'halogène,
A représente le reste de formule 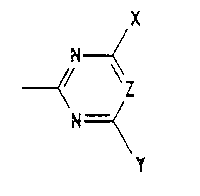
Z représente CH ou N, de préférence CH et
un des restes X et Y représente des atomes d'halogènes, des groupes alkyle en C₁-C₂, alcoxy en C₁-C₂, OCF₂H, CF₃ ou OCH₂CF₃ et l'autre des restes X et Y représente des groupes alkyle en C₁-C₂, alcoxy en C₁-C₂ ou haloalcoxy en C₁-C₂.

4. Procédé pour la préparation de composés de formule (I) ou de leurs sels selon la revendication 1, caractérisé
a) un composé de formule (II) 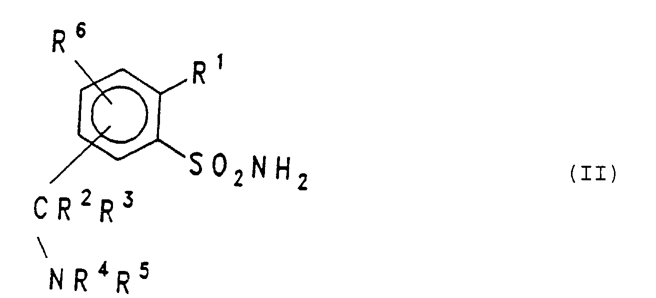 avec un carbamate hétérocyclique de formule (III)
R* - O - CO - NR⁷ - A (III)
dans laquelle R* représente un phényle éventuellement substitué ou alkyle en C₁-C₄, ou
b) un phénylsulfonylcarbamate de formule (IV) 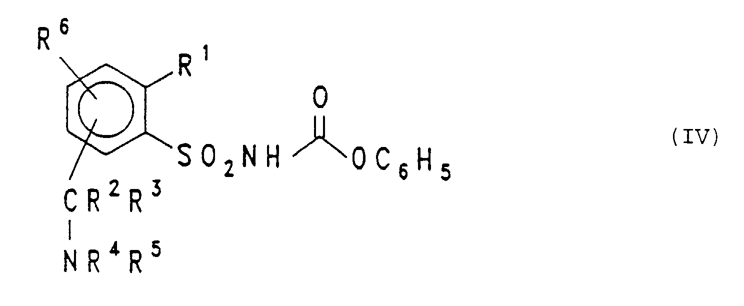 avec un hétérocycle aminé de formule (V)
H - NR⁷ - A (V)
ou
c) un sulfonylisocyanate de formule (VI) 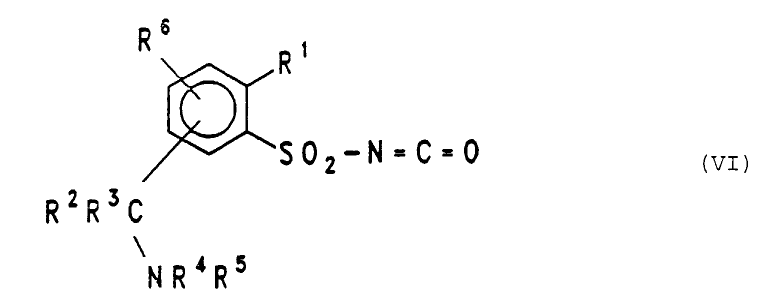 avec un hétérocycle aminé de formule H-NR⁷-A (V) ou
d) dans une réaction à un réacteur, on fait réagir d'abord un hétérocycle aminé de formule H-NR⁷-A (V) en présence d'une base avec le phosgène et on fait réagir le produit intermédiaire formé avec un phénylsulfonyl-amide de formule (II), ou
e) on fait réagir un chlorure de sulfonyle de formule (VII) 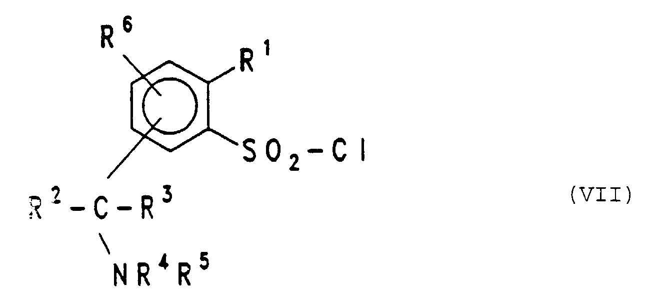 avec un cyanate M-OCN, où M = NH₄, Na ou K et avec un hétérocycle aminé de formule H-NR⁷-A (V) en présence d'une base,
f) on fait réagir un sulfonamide de formule (II) précité avec un thioisocyanate de formule (V')
W = C = N - A (V')
en présence d'une base,
dans les formules (II) à (VII) et (V'), les restes, respectivement les groupes R¹-R⁷, A ou W sont définis comme dans la formule (I) et dans les variantes a) à e) on obtient d'abord des composés de formule (I) avec W = O.

5. Agent herbicide ou régulateur de croissance, caractérisé en ce qu'il contient au moins un composé de formule (I) ou son sel selon l'une des revendications 1 à 3 et des adjuvants de formulation usuels en phytoprotection.

6. Procédé pour la lutte contre les plantes nuisibles ou pour la régulation de la croissance des plantes, caractérisé en ce que l'on applique une quantité efficace d'au moins un composé de formule (I) ou d'un de ses sels selon l'une des revendications 1 à 3 sur les plantes nuisibles, respectivement sur les plantes,leurs grains ou le sol dans lequel elles poussent.

7. Utilisation des composés de formule (I) ou de leurs sels selon l'une des revendications 1 à 3, en tant qu'herbicides ou régulateurs de croissance.

8. Composé de formule (II) 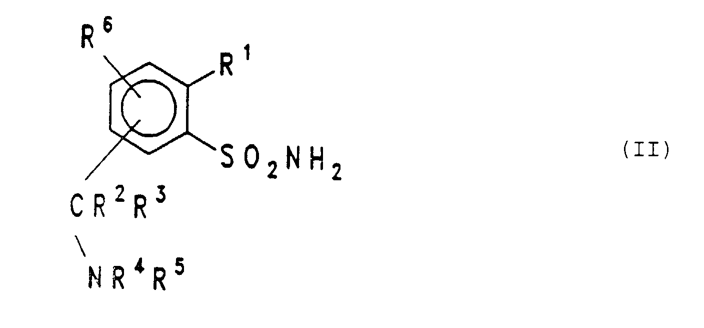 dans laquelle R¹ à R⁶ sont définis comme dans la formule (I) selon la revendication 1.

9. Procédé pour la préparation de composés de formule (II) selon la revendication 8, caractérisé en ce que l'on fait réagir un composé de formule (XV) 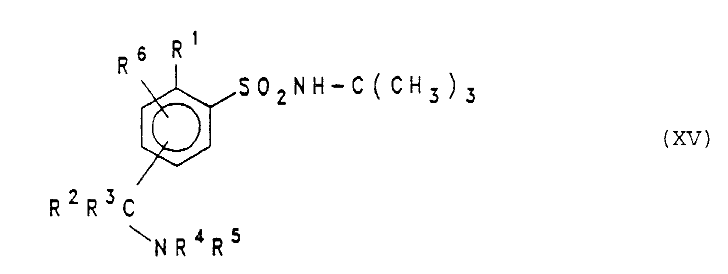 de façon analogue aux méthodes connues pour la dissociation du groupe protecteur tert-butyle, les restes R¹ à R⁶ dans la formule (XV) étant définis comme dans la formule (II).
